(19) Europäisches Patentamt
European Patent Office
Office européen des brevets

(11) Publication number: **0 234 113 A1**

(12) # EUROPEAN PATENT APPLICATION

(21) Application number: 86309767.1

(22) Date of filing: 15.12.86

(51) Int.Cl.[3]: C 07 C 87/458
C 07 C 91/28, C 07 C 97/10
C 07 C 93/14, C 07 D 295/06
C 07 C 93/26, C 07 D 205/04
C 07 D 319/06, A 61 K 31/135
A 61 K 31/215, A 61 K 31/395

(30) Priority: 31.12.85 US 815367

(43) Date of publication of application: 02.09.87 Bulletin 87/36

(84) Designated Contracting States: AT ES GR

(71) Applicant: THE UPJOHN COMPANY
301 Henrietta Street
Kalamazoo, Michigan 49001(US)

(72) Inventor: Szmuszkovicz, Jacob
The Upjohn Co. 301 Henrietta Street
Kalamazoo Michigan 49001(US)

(72) Inventor: Darlington, William H.
The Upjohn Co. 301 Henrietta Street
Kalamazoo Michigan 49001(US)

(72) Inventor: VonVoigtlander, Philip F.
The Upjohn Co. 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) **2,3-Dihydro-1H-phenalene-2-amino compounds as anti-psychotic drugs.**

(57) 2-3-Dihydro-1H-phenalene-2-amino compounds of the formula

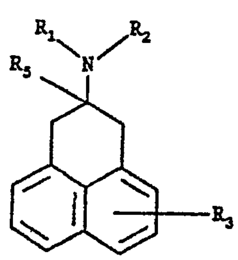

(I)

some of which are new, e.g., 2-3-dihydro-2-(N,N-di-n-propylamino)-1H-phenalen-5-ol, and pharmaceutically acceptable salts of such compounds have been found to be useful anti-psychotic drug compounds.

EP 0 234 113 A1

# TITLE

## 2,3-DIHYDRO-1H-PHENALENE-2-AMINO COMPOUNDS AS ANTI-PSYCHOTIC DRUGS

## INTRODUCTION

This invention relates to 2-amino-2,3-dihydro-1H-phenalene ring compounds, some of which are new compounds, which have been found to have anti-psychotic drug activity. More particularly, this invention provides a group of 2-amino-2,3-dihydro-1H-phenalene compounds, either unsubstituted in the phenalene ring system, or substituted on the 4-, 5- or 6-position with a substituent defined hereinbelow, which have been found to have useful ranges of anti-psychotic activity.

## BACKGROUND OF THE INVENTION

Known commercially available, organic compounds (non-lithium-containing drugs), e.g., prochlorperazine, thoridazine, thiothixene, fluphenazine, piperacetazine, trifluoroperazine, are neuroleptic drugs. Neuroleptic drugs are known to exhibit, to a lesser or higher degree, extrapyramidal system side effects, such as catalepsy, which side effects Central Nervous System (CNS) drug researchers would prefer to avoid.

The 2-amino-2,3-dihydro-1H-phenalene compounds, the antipsychotic use of which is claimed herein, are believed to have excellent antipsychotic activity as predicted by some standard laboratory animal tests, and it is hoped that the compounds hereof will have no, or at least reduced, CNS extrapyramidal side effects in their use as antipsychotic drugs.

Some 2-amino-2,3-dihydro-1H-phenalene derivative compounds, per se, have been described in publications such as

(1) Chem. Scand., 19, 755 (1965), which shows an N-(methoxycarbonyl)amino-2,3-dihydro-1H-phenalene, but it does not disclose the compounds claimed here or the anti-psychotic drug use of this invention.

(2) Chim. Therap., 4, 95 (1969), discloses the
2-amino-1-oxo-2,3-dihydro-1H-phenalene hydrochloride,
2-(N-acetylamino)-1-hydroxy-2,3-dihydro-1H-phenalene,
2-amino-1-hydroxy-2,3-dihydro-1H-phenalene hydrochloride, and
2-(N-ethylamino)-1-hydroxy-2,3-dihydro-1H-phenalene, but such

publication does not disclose the compounds claimed herein or the antipsychotic drug use for the compounds disclosed therein.

(3) The Chimie Therapeutique, 6, 196 Mai-Juin, 1971, No. 3, discloses 2-amino-2,3-dihydro-1H-phenalene, the 2-(N,N-dimethylamino--2,3-dihydro-1H-phenalene, the 2-(N,N-dimethylamino-2,3-dihydro-1H-phenalene, and the 2-(N-methylamino)-2,3-dihydro-1H-phenalene, but such publication does not disclose the antipsychotic use which has been found for such compounds according to this invention.

(4) In the J. Chem. Soc., (London), Section C, Organic, (1971), pp 1607-1609, C. Evans et al. discloses N-ethyl-2,3-dihydro-4-methyl-phenalen-2-amine, and how to make it but it does not disclose any specific pharmaceutical use activity for the compound.

(5) Derwent Abstract 85-165888/28 of W. German Patent Offen. 3,346,573A discloses some 4-amino-tetrahydro-benz-indoles as CNS medicaments, but it does not mention the compounds disclosed here or any use as an antiphychotic drug.

OBJECTS OF THE INVENTION

It is an object of this invention to provide a process or method for treating human and valuable warm-blooded animal patients suffering from psychotic symptoms with an amount of one of the herein defined 2-amino-2,3-dihydro-1H-phenalene compounds effective to relieve the psychotic symptoms in said patient.

It is another object of this invention to provide a group of defined, new 2-amino-2,3-dihydro-1H-phenalenes, as new compounds per se, which are useful in appropriate dosage unit forms as antipsychotic drug agents to remove or reduce the psychotic drug behavior in human and valuable animal patients who exhibit s-psychotic behavior.

SUMMARY OF THE INVENTION

Briefly, this invention provides a method or process for treating psychotic symptoms in human and valuable warm-blooded animal patients which comprises administering to said patient a 2-amino-2,3-dihydro-1H-phenalene compound of formula I herebelow, in an amount sufficient to relieve the symptoms of psychotic behavior in said patient.

This invention also provides a defined group of new compounds per se, of formula I herebelow, which are useful in appropriate pharmaceutical dosage unit forms, as drugs for treating patients suffering from psychotic symptoms, to relieve those symptoms of psychosis in said patient.

In contrast to known neuroleptic type antipsychic compounds, the compounds of this invention hopefully will show the advantage over neuroleptic type drugs of having little or no extrapyramidal CNS side effects.

To date, the lead compound of this group of 2-amino-2,3-dihydro-1H-phenalenes being tested as an antipsychotic drug is 2-(N,N-di-n-propylamino)-5-hydroxy-2,3-dihydro-1H-phenalene, in its racemic (±) form or as its separated stereo dextro (+) and levo (-) isomeric forms. Advanced testing is being suggested with the dextro (+) isomer of this compound.

## DETAILED DESCRIPTION OF THE INVENTION

More specifically, this invention provides a new method or process and some new compounds for treating psychotic patients to eliminate or reduce the symptoms of psychosis, which comprises administering to such a psychotic patient, in appropriate dosage unit form, a 2-amino-2,3-dihydro-1H-phenalene compound of formula I (See the structural Formula sheets) where $R_1$ and $R_2$ are independently selected from the group consisting of hydrogen, $C_1$ to $C_4$-alkyl, $C_2$ to $C_4$-alkenyl, $C_3$ to $C_6$-cycloalkyl, or

$R_1$ and $R_2$ are taken together with the nitrogen to which they are bonded to complete a nitrogen containing ring selected from the group consisting of 1-azetidinyl, 1-pyrrolidinyl, 1-piperidinyl and 4-morpholinyl;

$R_3$ is selected from the group consisting of

hydrogen,

$C_1$ to $C_4$-alkyl,

$C_1$ to $C_4$-alkyloxy,

-trifluoromethyl,

-fluorine, chlorine or bromine,

-hydroxy,

-thio-$C_1$ to $C_4$-alkyl, thio(O)-$C_1$ to $C_4$-alkyl, or thio$(O)^2$-$C_1$ to $C_4$-alkyl,

-$C_1$ to 9-alkanoyloxy,

-$C_1$ to $C_4$-alkyloxycarbonyl (alkyl-O-C(O)-),

-2-($C_1$ to $C_4$-alkyl-1,3-dioxan-2-yl),

-1-hydroxy-$C_1$ to $C_4$-alkyl,

-$C_1$ to $C_5$-alkanoyl

-$R_4C(O)O$-,

-$R_4CH_2C(O)O$-,

-$R_4CH_2O$-,

-$R_4C(O)$-;

$R_4$ is phenyl, or phenyl substituted with 1 to 3 substituents selected from the group consisting of

$C_1$ to $C_4$-alkyl,

$C_1$ to $C_4$-alkyloxy,

$C_1$ to $C_4$-alkyl-S-,

fluorine, chlorine and bromine,

$R_5$ is hydrogen or methyl;

n is 0 to 2;

m is 0 to 2 in its racemic or optically active form, or a pharmaceutically acceptable acid addition salt thereof.

In the above formula I compounds, the term "$C_1$ to $C_2$-alkyl" means the methyl and ethyl groups. The term "$C_1$ to $C_3$-alkyl" further includes n-propyl and isopropyl groups. The term $C_1$ to $C_4$-alkyl further includes the butyl group in its various isomeric forms. The term "$C_1$ to $C_2$-alkyloxy" means methyloxy and ethyloxy. The term "$C_1$ to $C_2$-alkyloxycarbonyl" means methoxycarbonyl ($CH_3OC(O)$-) or ethyloxycarbonyl $C_2H_5$-$OC(O)$-. The term "$C_1$ to $C_5$-alkanoyloxy" means acetyloxy, propionyloxy, butanoyloxy or pentanoyloxy, e.g., $CH_3COO$- is acetyloxy.

Examples of acid addition salts of these compounds include the hydrohalide salts such as the hydrochloride, hydrobromide, hydrofluoride and hydroiodide, the sulfate and bisulfate, various phosphorus acid salts, the methanesulfonate, the p-toluenesulfonate, the benzoate, the acetate, and other alkanoic acid salts, as well as the salts of various dicarboxylic and tricarboxylic acids such as maleic, succinic, fumaric, malic, oxalic, itaconic acids, and the like. Some of these acids, e.g., oxalic acid, may be preferred for extracting the active amino or azetidine derivative from its reaction mixture, while other acids, e.g., succinic, maleic or p-toluenesulfonic may be preferred when the resulting amine or azetidine salt is to be formulated into pharmaceutically useful form. Also, the formula I compound and

its acid addition salt in their crystalline state may sometimes be isolated as solvates, i.e., with a discrete quantity of water or other solvent such as ethyl acetate, ethanol, and the like, associated physically and thus removable without effective alteration of the active chemical drug entity per se.

If desired the formula I compounds of this invention can be resolved into their respective d- and l-optical isomers by methods known in the art. In this case, the optical resolution can be done by at least two different routes. The resolving agents by either route are any of the known resolving agents such as optically active dibenzoyltartaric acid, camphorsulfonic acid, bis-o-toluoyltartaric acid, tartaric acid, and diacetyl tartaric acid which are commercially available and which are commonly used for resolution of amines (bases), as for example in Organic Synthesis, Coll. Vol. V., p. 932 (1973), resolution of R-(+) and S-(-)-α-phenylethylamine with (-)-tartaric acid.

By one preferred method for resolving the compounds of this invention, for example, one of the formula I, 2-(N,N-di-n-propyl)-5-methyl-2,3-dihydro-1H-phenalen-2-ylamine, or other amine compounds can be converted into its optically active diastereomeric salts by reaction with an optically active acid - examples mentioned above- in a manner standard in the isomer resolution art. These diastereomeric salts can then be separated by conventional means such as differential crystallization. Diastereomeric salts have different crystallization properties, which are taken advantage of in this separation. On neutralization of each diastereomeric salt with aqueous base the corresponding optically active enantiomers of the formula I amine or other amine compound can be obtained, each of which can subsequently and separately be converted as hereinafter described in the examples to the desired acid addition salt, if desired.

Alternatively an amine-containing precursor to a formula I compound can first be resolved as above and then converted to an optically active form of a formula I compound.

The new compounds of this invention are compounds of formula I, where

$R_1$ and $R_2$ are independently selected from the group consisting

of hydrogen, $C_1$ to $C_4$-alkyl, $C_2$ to $C_4$-alkenyl, $C_3$ to $C_6$-cycloalkyl, or

$R_1$ and $R_2$ are taken together with the nitrogen to which they are bonded to complete a nitrogen containing ring selected from the group consisting of 1-azetidinyl, 1-pyrrolidinyl, 1-piperidinyl and 4-morpholinyl;

$R_3$ is selected from the group consisting of

hydrogen,

$C_1$ to $C_4$-alkyl,

$C_1$ to $C_4$-alkyloxy,

-trifluoromethyl,

-fluorine, chlorine or bromine,

-hydroxy,

-thio-$C_1$ to $C_4$-alkyl, thio(O)-$C_1$ to $C_4$-alkyl, or thio$(O)_2$-$C_1$ to $C_4$-alkyl,

-$C_1$ to 9-alkanoyloxy,

-$C_1$ to $C_4$-alkyloxycarbonyl (alkyl-O-C(O)-),

-2-($C_1$ to $C_4$-alkyl-1,3-dioxan-2-yl),

-1-hydroxy-$C_1$ to $C_4$-alkyl,

-$C_1$ to $C_5$-alkanoyl

-$R_4C(O)O$-,

-$R_4CH_2C(O)O$-,

-$R_4CH_2O$-,

-$R_4C(O)$-,

$R_4$ is phenyl, or phenyl substituted with 1 to 3 substituents selected from the group consisting of

$C_1$ to $C_4$-alkyl,

$C_1$ to $C_4$-alkyloxy,

$C_1$ to $C_4$-alkyl-S-,

fluorine, chlorine and bromine,

$R_5$ is hydrogen or methyl;

n is 0 to 2;

m is 0 to 2; or an acid addition salt thereof, except

2,3-dihydro-N-methyl-1H-phenalen-2-amine,

2,3-dihydro-N-(dimethyl)-1H-phenalen-2-amine,

1-[2,3-dihydro-2-(dimethylamino)-1H-phenalen-6-yl]ethanone,

or an acid addition salt thereof, except

2,3-dihydro-N-methyl-1H-phenalen-2-amine,

2,3-dihydro-N,N-dimethyl-1H-phenalen-2-amine,

1-[2,3-dihydro-2-(dimethylamino)-1H-phenalen-6-yl]ethanone, and

2,3-dihydro-4-methyl-N-monoethyl-1H-phenalen-2-amine, or an acid addition salt thereof.

A preferred group of these 2-amino-2,3-dihydro-1H-phenalene compounds for this antipsychotic drug use are compounds of formula III (See Structural Formula Sheet) where $R_3$ is hydrogen or is a substituent in the 5- position of the ring system thereof, $R_1$ and $R_2$ are each hydrogen or $C_1$ to $C_4$-alkyl, and $R_3$ is $C_1$ to $C_4$-alkyloxy or hydroxy, or a pharmaceutically acceptable salt thereof. Examples of such compounds include:

2-(N,N-dimethylamino)-5-methoxy-2,3-dihydro-1H-phenalene,

2-(N,N-diethylamino)-5-methoxy-2,3-dihydro-1H-phenalene,

2-(N-methylamino)-2,3-dihydro-1H-phenalene,

2-(N,N-di-n-butylamino)-5-methoxy-2,3-dihydro-1H-phenalene,

2-(N,N-di-isopropylamino)-5-methoxy-2,3-dihydro-1H-phenalene,

2-(N,N-di-tert-leutylamino)-5-methoxy-2,3-dihydro-1H-phenalene

and the corresponding 5-hydroxy-phenalen-5-ol compounds such as:

2-(N,N-dimethylamino)-5-hydroxy-2,3-dihydro-1H-phenalene,

2-(N,N-diethylamino)-5-hydroxy-2,3-dihydro-1H-phenalene,

2-(N,N-n-propylamino)-5-hydroxy-2,3-dihydro-1H-phenalene,

2-(N,N-di-isopropylamino)-5-hydroxy-2,3-dihydro-1H-phenalene,

2-(N,N-di-n-butylamino)-5-hydroxy-2,3-dihydro-1H-phenalene,

and the pharmaceutically acceptable acid addition salts thereof. Similarly, the alkyloxy group $R_3$ in the 5-position can also be ethyloxy, n-propyloxy, isopropyloxy, n-butyloxy, isobutyloxy or tert-butoxy. A few examples of such compounds include:

2-(N,N-di-n-propylamino)-5-ethoxy-2,3-dihydro-1H-phenalene,

2-(N,N-diethylamino)-5-n-propyloxy-2,3-dihydro-1H-phenalene,

2-(N,N-dimethylamino)-5-n-butoxy-2,3-dihydro-1H-phenalene,

and the like, and pharmaceutically acceptable salts thereof.

Other useful preferred groups of 2-amino-2,3-dihydro-1H-phenalene compounds are those wherein the $R_3$-substituent is in the 4- or 6-position, as shown by the sub-generic Structural Formulas II and IV, hereinbelow. Examples of such compounds are like those listed

above, except that the $R_3$ substituent is in the 4- or 6-positions, e.g.,

2-(N,N-dimethylamino)-4-methoxy-2,3-dihydro-1H-phenalene,

2-(N,N-diethylamino)-6-methoxy-2,3-dihydro-1H-phenalene,

2-(N,N-di-n-propylamino)-4-methoxy-2,3-dihydro-1H-phenalene,

2-(N,N-di-n-butylamino)-6-methoxy-2,3-dihydro-1H-phenalene,

2-(N,N-di-n-propylamino)-4-hydroxy-2,3-dihydro-1H-phenalene,

2-(N,N-di-n-propylamino)-6-hydroxy-2,3-dihydro-1H-phenalene,

and the like, and the pharmaceutically acceptable salts thereof.

Lead compounds of these types being considered for further advanced studies are the (±)2-(N,N-di-n-propylamino)-2,3-dihydro-1H-phenalen-5-ol, mixed stereo isomer compound, and pharmaceutically acceptable salts thereof, and this compound in its separated stereoisomer form, namely, (+)-2-(N,N-di-n-propylamino)-2,3-dihydro-1H-phenalen-5-ol, and its levo enantiomer, (-)-2-(N,N-di-n-propylamino)-2,3-dihydro-1H-phenalen-5-ol, and their pharmaceutically acceptable salts.

Other lead study compounds for antipsychotic use activity are 2,3-dihydro-N-mono-methyl-1H-phenalen-2-amine, and its pharmacologically acceptable salts, e.g., its hydrochloride salt, hereinafter referred to as U-64,273A, and 2,3-dihydro-N,N-dimethyl-1H-phenalen-2-amine, and salts thereof.

By the intravenous (i.v.) route of administration to anesthetized rats, U-64,273A is potent in reversing the effect of d-amphetamine on nigral DA neuronal firing. Standard antipsychotic agents such as haloperidol and clozapine also reversed amphetamine's depressions of DA neuronal firing, as has been reported by other laboratories (1). The ability of neuroleptics to actually increase firing rates above control values has been associates (1,2) with the tendency of that agent to induce extrapyramidal side effects (EPS). Thus, haloperidol, which induces severe EPS in patients, increased firing rates to a level 64% greater than controls whereas clozapine, which induces far less EPS, (3), completely reversed the amphetamine effect but did not increase rates above controls (Table 2).

Unlike haloperidol the amphetamine effect reversal by U-64,273A was not quite complete. This suggests that U-64,273A in addition to being a dopamine antagonist has a small amount (partial) of dopamine

agonist activity. A partial agonist property is further suggested by the fact that U-64,273 is able to weakly inhibit the DA neuronal firing when given alone. This pattern of effect is similar to that other partial agonists, trans-dihydrolisuride (TDHL), and (-)PPP (4,-5). However, the dopamine antagonist properties of TDHL and (-)PPP are much less prominant than with U-64,273A. Thus, U-64,273A is 70% antagonist and 30% agonist while the antagonist/agonist ratios for TDHL and (-)PPP are approximately 50:50 and 30:70 respectively (Table 2). In addition the antagonist potency of U-64,273A is considerably greater than that of clozapine. The dopamine antagonist properties of U-64,273A should be sufficient to antagonize overactive DA systems such as those though to underly schizophrenia. By avoiding a complete blockage, there may be a reduced severity of side-effects such as pseudo-parkinsonism and tardive dyskinesia in using this compound as an antipsychotic drug.

Consistant with its having a minor amount of agonist activity, the overall behavioral effects of U-64,273A conform to that of a weak DA antagonist (Table 3). It antagonized apomorphine in the following tests: mouse climbing screen, locomotor stimulation in reserpinized mice, emesis in dogs and discriminative effects in monkeys. U-64,273A also antagonized d-amphetamine in the turning behavior of striatal-lesioned rats. However, it did not protect mice from a lethal dose of d-amphetamine nor did it block the stereotypic behaviors produced by apomorphine. When given by itself, U-64,273A affected locomotor activities in rodents in a way resembling both DA auto-receptor and postsynaptic receptor agonists. It suppressed conditioned avoidance behavior of rats with a limited efficacy. It produced no locomotor stimulation in reserpinized mice.

The effects of U-64,273A on the metabolites of DA and 5-HT in rat brains suggest an antagonist action of DA and an agonist action of 5-HT (Table 4). The elevation of HVA was only moderate in comparison to that produced by haloperidol. It did not increase the plasma level of prolactin as did haloperidol. Both the HVA and prolactin effects are consistent with the expected low EPS. U-64,273A is relatively weak in the in vitro receptor binding screen but did displace spiperone in vivo. (The numbers in parenthesis are to References listed below Table 5 hereinbelow).

The dimethylated derivative, U-65,556, is also of interest because it has antipsychotic activity with little or no propensity to proauce EPS since it, 2,3-dihydro-N,N-dimethyl-1H-phenalen-2-amine, like U-64,273A, also reversed amphetamine's depressions of DA neurons without causing firing rates to increase over control.

The 2-amino-phenalene compounds of this invention and those within the scope of the anti-psychotic method of use claims of this invention can be prepared by either a Diels-Alder type reaction, or by starting the reactions with an unsubstituted or appropriately substituted naphthalic anhydride, by procedures described herein, or by procedures known to those in the art. The Diels-Alder process variation starts from the selected commercially available non-$R_3$-substituted or -$R_3$-substituted 2-hydroxybenzaldehyde and 2-formyltetrahydrofuran, as outlined in Process Chart A, hereinbelow. A second usable process starts from an unsubstituted or $R_3$-substituted naphthalene or naphthalenic anhydride and oxanilide and phosphorus pentachloride, or their equivalents. The $R_3$-substitution, if present, is present in a position such that in the desired 2-amino-2,3-dihydro-1H-phenalene end product compound an $R_3$-substituent will be in the 4-, 5- or 6-position. The selection of the process can sometime depend upon the $R_3$-substituent which is to be present in the end product compound. Also, some of the above listed $R_3$ groups are obtained after progression of the process to a point where it is desirable to remove a protecting group, e.g., by the use of a starting material containing an $R_3$-methoxy group, and later removal of the methyl group to form the end product 2-amino-2,3-dihydro-1H-phenalen-4-, 5- or 6-ol compound, which can then be further treated, if desired, to form esters, ethers and/or acid addition salts thereof.

Thus, referring to Chart A, the Diels-Alder process, the selected 2-formyl-($R_{13}$)-phenol (A-1) where $R_{13}$ is, for example, a $C_1$ to $C_4$-alkyloxy, hydroxy or $R_4$-$CH_2$-O group where $R_4$ is phenyl or substututed phenyl as defined hereinabove, can be halogenated, for example, in step (A) with chlorine, bromine or iodine, preferably bromine, in the presence of a basic salt, such as an alkali metal acetate, and a mild acid such as acetic acid, followed by treatment with a halogenation promoter such as a stannous halide, e.g., stannous chloride, to form the 3-halo-2-hydroxy-$R_3$-benazldehyde (A-2). The halogenated benzaldehyde (A-2) can then be treated in step B, with a hydroxy group protecting agent, e.g., with p-toluenesulfonyl chloride or bromide, to form the 3-bromo-2-(p-toluenesulfonyloxy)-$R_3$-benzaldehyde (A-3).

In a separate operation, to prepare another reactant, furfuraldehyde (A-4) can be refluxed in step C, with $\beta$-alanine in a $C_1$ to $C_3$-alkanol containing mixture, e.g., ethanol, for a sufficient time to form the 2-(2-nitroethenyl)furan compound (A-5). The 2-(nitroethenyl)furan compound (A-5) can be reduced in step D by known methods, e.g., with an alkali metal borohydride, to form the 2-(2-nitroethyl)furan (A-6).

Then, the hydroxy group protected-bromo-$R_3$-benzaldehyde compound (A-3) and the 2-(2-nitroethyl)furan (A-6) can be reacted in step E, e.g., in a solvent mixture containing piperidine, acetic acid and a hydrocarbon solvent such as hexane, e.g., a commercially available hexane mixture such as SKELLYSOLVE B, to form the adduct (A-7). The adduct A-7 can be reduced in step F by known means, e.g., with lithium aluminum hydride in an acid medium and an ether solvent such as tetrahydrofuran (THF), to form the corresponding amine compound (A-8), which can then be converted to a salt with an achiral acid, e.g., furmaric acid, or with a chiral acid, e.g., di-p-toluoyltartaric acid. This amine compound A-8 can, if desired, be resolved in step G at this point by known procedures, to form and separate the corresponding dextro (+) amine compound (A-9) and the levo (-) amine compound A-9.

Then, in step H, the racemic (±) mixture of the amine (A-9), or its separated (+) amine A-9 or (-) amine A-9 can be treated to protect the amine group, e.g., with acetonylacetone to form the amine-nitrogen protecting 2,5-dimethyl pyrrolyl ring compound (A-10).

The amino-protected compound A-10 can then be treated in step I with a deprotecting chemical, e.g., with phenyllithium, to remove the hydroxyl protecting group, here, e.g., the p-toluenesulfonyl group, to allow adduct formation or ring closure to occur to form the oxo-bridged, unsaturated ring structure compound (A-11). The oxa-bridged, ring unsaturated compound A-11 can then be reduced, in step J, e.g., by hydrogenation in the presence of a hydrogenation catalyst such as palladium on carbon to form the oxa-bridged, saturated ring compound (A-12). The oxa-bridged compound A-12 can be treated in

step K with an aromatizing agent, e.g., with boron trifluoride etherate, to remove the oxa-bridge oxygen atom and to form the amino-nitrogen protected-2,3-dihydro-1H-$R_3$-phenalene ring compound (A-13). The amino nitrogen protected compound A-13 can be treated in step L with a nitrogen-deprotecting agent, e.g., with hydroxyamine, to form the 2-amino-2,3-dihydro-1H-$R_3$-phenalene compound A-14.

From here on, various process options are open depending upon which end product compound is desired. These 2-amino-2,3-dihydro-1H-$R_3$-phenalene compounds have anti-psychotic activity in their own right. For use as drugs these 2-aminophenalene compounds A-14 can be further processed to remove protecting groups, if desired, e.g., to remove alkyl groups from $R_3$-$C_1$ to $C_4$-alkyl groups to form the corresponding phenalen-4-, 5- or 6-ol compounds, and then purified as the free amine base compound, or converted to its appropriate acid addition salt one or more times for purification and isolation from its reaction mixture, and then to the selected pharmaceutically acceptable acid addition salt for further purification and crystallization for use in making the pharmaceutical compositions thereof, for use as an antipsychotic drug product.

Alternatively, and preferably the primary 2-amino nitrogen of compound A-14 can be alkylated in step M, e.g., with appropriate halogenated hydrocarbons such as a $C_1$ to $C_4$-alkyl halide such as a methyl, ethyl, n-propyl, isopropyl, n-butyl, isobutyl or tert-butyl bromide in amounts and for a time sufficient to form the desired corresponding 2-(mono- or (di-alkylamino)-2,3-dihydro-1H-phenalene compound, or with a selected X-$C_3$ to $C_5$-X dihaloalkane where X is chloro, bromo, or iodo, e.g., with 1,3-dibromopropane, 1,4-dibromobutane, 1,5-dibromopentane to form the corresponding 1-azetidine, 1-pyrrolidine or 1-piperidine compound or with 2-(2-bromoethoxy)ethyl bromide to form the 4-morpholinyl compound. Such ring compounds can also be ring substituted on ring carbons thereof with 1 to 2-methyl groups, as ethyl group, a methoxy group or a hydroxy group by the corresponding selection of the dibromo-substituted alkane reactant. One can also make the corresponding $R_1$-$R_2$-$C_1$ to $C_4$-alkenyl amine compounds by alkylating the 2-amino compound XVIII with a C2 to C4-alkenyl bromide to form the corresponding N-$C_2$ to $C_4$-alkenylamino- and N,N-bis ($C_2$ to $C_4$-alkenyl)amino compounds, e.g.,

2-(N,N-di-allylamino)-2,3-dihydro-1H-phenalen-5-ol, and

2-(N,N-(bis-2-butenylamino)-2,3-dihydro-1H-phenolen-5-ol, and the like. Similarly, one can alkylate the 2-amino nitrogen of compound XVIII in step M with a $C_3$ to $C_6$-cycloalkyl bromide to form N-mono- or N,N-di-cycloalkylamino-2,3-dihydro-1H-phenalene compounds, such as

2-(N-cyclohexylamino)-5-methoxy-2,3-dihydro-1H-phenalene, and

2-(N,N-di-cyclobutyl-2,3-dihydro-1H-phenalen-5-ol,

and then optionally if a more desirable physical form, e.g., a crystal form, of the 2-amino-2,3-dihydro-1H-phenalene compound A-15 is desired for purification or for pharmaceutical formulation handling advantages, in step N, one can form any of a variety of acid addition salt forms of said compounds A-16 by known dissolution, purification and crystallization procedures.

Alternatively, and for a variety of reasons such as availability of starting materials, cost, and ease of processing, it may be preferred to prepare the selected formula I compound, starting from a naphthalene source. For example, and referring to Process Chart B, the unsubstituted or $R_3$-substituted naphthalene (B-1), e.g., an $R_{13}$-substituted naphthalene where $R_{13}$ is a $C_1$ to $C_4$-alkyloxy, hydroxy or an $R_4$-$CH_2$-O-group where $R_4$ is phenyl or substituted phenyl as defined hereinabove, can be reacted with the bis[(N-phenyl)(chloro)-methylimine] (B-2) and aluminum chloride (B-3), or their equivalents, to form the $R_{13}$-naphthalene anhydride compound (B-4). The anhydride (B-4) can then be ring opened with base and esterified to form the 1,8-diester compound (B-5). The diester (B-5) can then be reduced, e.g., with lithium aluminum hydride, to form the $R_3$-naphth-1,8-yldimethanol compound (B-6). The di-ol compound B-6 can then be halogenated, e.g., with phosphorus tribromide, to form the $R_{13}$-naphth-1,8-ylbis(methyl bromide) compound (B-7). The di-bromomethyl compound (B-7) can then be ring-closed and esterified in the 2-position of the 2,3-dihydro-1H-$R_3$-phenalene compound, e.g., by treatment with a malonate ester in the presence of an alkali metal hydride to form one or both of $R_{13}$-phenalene ring esters (B-8) and (B-9). The ester intermediate (B-8 and/or B-9) can then be de-esterified to form the 2-dicarboxy-$R_{13}$ -phenalene compound (B-10). The di-carboxy compound B-10 can be heated to a temperature sufficient to remove one carboxyl

group and to form the 2-mono-carboxy $R_3$-phenalene compound (B-11). The 2-carboxy compound (B-11) can be treated with diphenyl phosphorus azide in the presence of a $C_1$ to $C_8$-alkanol, preferably tert-butanol, to form the corresponding 2-alkyloxycarbonylamino)phenalene (2-carbamate) compound (B-12). The 2-carbamoyl compound (B-12) can be treated with acid, e.g., trifluoroacetic acid, to form the 2-amino-$R_3$-phenalene compound (B-13). As above, the chemical operator at this point has the options of treating the primary 2-amino compound (B-13) to remove protecting groups, or to alkylate the primary 2-amino nitrogen of compound B-13 to form the desired 2-(N-mono- or N,N-di-substituted amino)-2,3-dihydro-1H-$R_3$-phenalene compounds (B-14). If desired, the 2-amine compound B-13 can be treated with formaldehyde and alkali metal borohydride, by known methods, to form the 2-(N-methyl- or N,N-dimethylamino)-2,3-dihydro-1H-$R_3$-phenalene compound (B-14). Alternatively, as above, one can alkylate the 2-amino nitrogen with one or two $C_1$ to $C_4$-alkyl groups, or with di-bromoalkane compounds to form the corresponding 1-azetidine, 1-pyrrolidine or 1-piperidine or 4-morpholine derivative compounds B-14. Further, the selected 2-amino-2,3-dihydro-1H-phenalene compound (B-14) can be converted to an acid addition salt forms thereof successively, for isolation, purification, and pharmaceutical composition preparation purposes.

Processes for preparing compounds of the invention are further exemplified by detailed Examples 38 to 43 and Charts C through G which illustrate in chemical structure flow sheet formats how compounds of this invention were and can be made. Examples 38 to 40 together with Charts C, D and E illustrate and exemplify how 6-position substituted-2,3-dihydro-1H-phenalen-2-amine compounds, useful as antipsychotic drugs can be prepared. Examples 41 and 42 together with Charts F and G exemplify and illustrate how some of the preferred 5-position substituted 2,3-dihydro-1H-phenalen-2-amine compounds can be prepared.

This invention also relates to compositions containing a new formula I compound as an active ingredient in a pharmaceutical carrier. The compositions are useful in pharmaceutical dosage unit forms of the formula I compounds for local (topical) and systemic administration (oral, rectal and parenteral administration form) in therapy for treating and alleviating symptoms of psychoses in humans

and valuable animals, including dogs, cats and other commercially valuable and domestic animals.

The term "dosage unit form" as used in this specification and in the claims refers to physically discrete units suitable as unitary dosages for mammalian subjects, each unit containing a predetermined quantity of the essential active ingredient compound of this invention calculated to produce the desired effect, in combination with the required pharmaceutical means which adapt the said ingredient for systemic administration. The specification for the novel dosage unit forms of this invention are dictated by and directly dependent on the physical characteristics of the essential active ingredient and the particular effect to be achieved in view of the limitations inherent in the art of compounding such an essential active material for beneficial effects in humans and animals as disclosed in detail in this specification under exemplified embodiments, these being features of the present invention. Examples of suitable dosage unit forms in accordance with this invention are tablets, capsules, orally administered liquid preparations in suitable liquid vehicles, sterile preparations in suitable liquid vehicles for intramuscular and intravenous administration, suppositories, and sterile dry preparations for the extemporaneous preparation of sterile injectable preparations in a suitable liquid vehicle. Suitable solid diluents or carriers for the solid oral pharmaceutical dosage unit forms are selected from the group consisting of lipids, carbohydrates, proteins and mineral solids, for example, starch, sucrose, lactose, kaolin, dicalcium phosphate, gelatin, acacia, corn syrup, corn starch, talc and the like. Capsules, both hard and soft, are filled with compositions of the selected formula I compound or salt thereof ingredients in combination with suitable diluents and excipients, for example, edible oils, talc, calcium carbonate and the like and also calcium stearate. Liquid preparations for oral administration are prepared in water or aqueous vehicles which advantageously contain suspending agents, for example, methylcellulose, acacia, polyvinylpyrrolidone, polyvinyl alcohol and the like. In the case of injectable forms, the injectable formulation must be sterile and must be fluid to the extent that easy syringeability exists. Such preparations must be stable under the conditions of manufacture and storage, and ordinarily contain in addition to the basic solvent or suspending liquid, preservatives in the nature of bacteriostatic and fungistatic agents, for example, parabens, chlorobutanol, benzyl alcohol, phenol, thimerosal, and the like. In many cases, it is preferable to include osmotically active agents, for example, sugars or sodium chloride in isotonic concentrations. Carriers and vehicles include vegetable oils, ethanol, polyols, for example, glycerol, propylene glycol, liquid polyethylene glycol, and the like. Any solid preparations for subsequent extemporaneous preparation of sterile injectable preparations are sterilized, preferably by exposure to a sterilizing gas, for example, ethylene oxide. The aforesaid carriers, vehicles, diluents, excipients, preservatives, isotonic agents and the like constitute the pharmaceutical means which adapt the preparations for systemic administration.

For psychotic, including schizophrenic, disease, a daily dose of 1 to 700 mg is indicated, preferentially 10 to 200 mg; in units of two or three or four subdivided doses, and the exact amount is adjusted based on the weight, age and condition of the patient.

The pharmaceutical dosage unit forms are prepared in accordance with the preceding general description to provide from about 0.5 mg to about 100 mg of the essential active ingredient per dosage unit form. The amount of the essential active ingredient provided in the pharmaceutical dosage unit forms is based on the finding that the effective amount of 2-N,N-(di-n-propylamino)-2,3-dihydro-1H-phenalen-5-ol, a representative example of the compounds of the invention and acid addition salts thereof, for obtaining an antipsychotic effect in humans is expected to be within a range from about 0.01 mg/kg to about 10 mg/kg, preferably 0.06 to 1.0 mg/kg.

The active ingredients of this invention can also be compounded in combination with other ingredients. The amount of such other active ingredients is to be determined with reference to the usual dosage of each such ingredient. Thus, these active compounds can be combined with hypotensive agents such as α-methyldopa (100-250 mg); with diuretics such as hydrochlorothiazide (10-50 mg); tranquilizers such as meprobamate (200-400 mg), diazepam (2-10 mg), muscle relaxants, such as carisoprodol (200-400 mg).

The compounds listed below were tested and found to have possible useful anti-psychotic activity properties as indicated by their having CNS test result, $ED_{50}$ numbers of less than 50 mg/kg values in the known Hypothermia and/or the Apomorphine gnawing test. The lower $ED_{50}$ data numbers in one test or the other is believed to be an indication of whether the compound acts by pre-synoptic agonist mechanism or by a dopamine receptor antagonist mechanism in accomplishing its antipsychotic drug effect. Most of these compounds also show some analgesic potency in standard analgesic laboratory animal tests.

The Effect on Body Temperature (Hypothermia) Test and the Antagonism of Apomorphone-Induced Case Climbing (ACC), (Apomorphine Gnawing Test) are described on page 1398 of the publication, Journal of Medicinal Chemistry, Vol. 22, No. 11, pp. 1390-1398, in an article entitled "6-Aryl-4H-s-triazolo[4,3-a][1,4]benzodiazepines. ...Action" by J. B. Hester, Jr., et al.

For the tests here, the Hypothermia Test procedure was run as follows:

A group of four CF-1 male mice (18-22 g each) was injected intraperitoneally with the test compound prepared in 0.25 percent w/v methylcellulose in water solution. After 45 min, abdominal temperature of each mouse was measured using a thermister probe. A control group of four mice was treated with vehicle only and the temperature of the control group was taken in a similar manner. A compound was considered to have a significant effect on body temperature if the mean temperature in the test compound treated group deviated more than 3.5°C from the mean temperature of the parallel control group. Stimulants tend to elevate temperatures; depressants tend to lower body temperature.

As an example, when tested in this hypothermia test, the compound (±)2-(N,N-di-n-propylamino)-2,3-dihydro-1-phenalen-5-ol of this invention caused hypothermia in the test mice, with a calculated $ED_{50}$ of 1 mg/kg of body weight. The test data appear to suggest that the hypothermic effect of (±)2-(N,N-di-n-propylamino)-2,3-dihydro-1H-phenalen-5-ol is the result of the activation of dopamine receptors, since the known dopamine blocker, haloperidol, also significantly blocks the hypothermia effect induced by 2-(N,N-di-n-propylamino)-2,3-dihydro-1H-phenalen-5-ol. Moreover, the known alpha-2 adrenergic

blocker, yohimbine, did not alter the 2-(N,N-di-n-propylamino)-2,3-dihydro-1H-phenalen-5-ol induced hypothermia, but yohimbine did block the hypothermia usually induced by the known antihypertensive clonidine. The dopamine agonist, apomorphine, was similar to 2-(N,N-di-n-propylamino)-2,3-dihydro-1H-phenalen-5-ol in regard to antagonism by these agents. These results suggest usefulness of the 2-amino-2,3-dihydro-1H-phenalene compounds, claimed herein, as anti-psychotic drug compounds within useful dosage ranges.

The following compounds were tested and considered to be active in either the hypothermic or the apomorphine antogonism test:

(1) N-methyl-2,3-dihydro-1H-phenalen-2-amine hydrochloride,

(2) 6-Bromo-N,N-dimethyl-2,3-dihydro-1H-phenalen-2-amine hydrochloride,

(3) 2-(N,N-dimethylamino-2,3-dihydro-1H-phenalen-6-ol hydrochloride,

(4) 6-Bromo-N,N-di-n-propyl)-2,3-dihydro-1H-phenalen-2-amine,

(5) N-methyl-6-(2-methyl-1,3-dioxan-2-yl)-2,3-dihydro-1H-phenalen-2-amine,

(6) N-methyl-6-(2-methyl-1,3-dioxan-2-yl)-2,3-dihydro-1H-phenalen-2-amine maleate salt,

(7) N,N-dimethyl-2,3-dihydro-1H-phenalen-2-amine hydrochloride,

(8) N,N-dimethyl-6-(2-methyl-1,3-dioxan-2-yl)-2,3-dihydro-1H-phenalen-2-amine,

(9) 2-(N,N-di-n-propylamino)-2,3-dihydro-1H-phenalen-6-ol hydrochloride,

(10) 1-[2-(N,N-dimethylamino)-2,3-dihydro-1H-phenalen-y-yl]-ethan-1-ol hydrochloride,

(11) 1-[2-(N,N-dimethylamino)-2,3-dihydro-1H-phenalen-2-yl]azetidine,

(12) 1-[6-bromo-2,3-dihydro-1H-phenalen-2-yl]azetidine,

(13) 5-[2-(N,N-dimethylamino)-2,3-dihydro-1H-phenalen-2-yl]azetidine hydrochloride,

(14) N-methyl-6-acetyl-2,3-dihydro-1H-phenalen-2-amine hydrochloride,

(15) (±)2-(N,N-di-n-propylamino)-2,3-dihydro-1H-phenalen-5-ol,

(16) (±)2-(N,N-di-n-propylamino)-2,3-dihydro-1H-phenalen-5-ol hydrochloride,

(17) (±)2-(N,N-di-n-propylamino)-2,3-dihydro-1H-phenalen-5-ol hydrobromide salt,

(18) [2-(N,N-di-n-propylamino)-2,3-dihydro-1H-phenalen-5-yl]-α-benzyl ether,

(19) [2-(N,N-dimethylamino)-2,3-dihydro-1H-phenalen-6-yl]phenone maleate salt,

(20) [2-(N-methylamino)-2,3-dihydro-1H-phenalen-6-yl]phenone methanesulfonate salt,

(21) [2-(N-methylamino)-2,3-dihydro-1H-phenalen-6-yl]benzyl ether, p-toluene sulfonate salt,

(22) [2-(N,N-dimethylamino)-2,3-dihydro-1H-phenalen-6-yl]benzyl ether hydrochloride,

(23) 2-(N,N-dimethylamino)-2,3-dihydro-1H-phenalen-5-ol,

(24) 4-methoxy-2,3-dihydro-1H-phenalen-2-amine,

(25) N,N-di-n-propyl-4-methoxy-2,3-dihydro-1H-phenalen-2-amine hydrochloride,

(26) 1-[6-bromo-2,3-dihydro-1H-phenalen-2-yl]pyrrolidine,

(27) 6-bromo-2,3-dihydro-1H-phenalen-2-ylamine hydrochloride,

(28) 2-(N,N-di-n-propylamino)-2,3-dihydro-1H-phenalen-4-ol hydrobromide,

(29) 1-[6-bromo-2,3-dihydro-1H-phenalen-2-yl]piperadine,

(30) N,N-dimethyl-4-methoxy-1H-phenalen-2-ylamine hydrochloride, 0.25 $H_2O$ hydrate,

(31) N,N-dimethyl-4-methoxy-2,3-dihydro-1H-phenalen-2-ylamine maleate salt,

(32) N,N-di-n-propyl-4-methoxy-2,3-dihydro-1H-phenalen-2-ylamine hydrochloride,

(33) (+)-2-(N,N-di-n-propylamino)-2,3-dihydro-1H-phenalen-5-ol hydrobromide,

(34) (-)-2-(N,N-di-n-propylamino)-2,3-dihydro-1H-phenalen-5-ol hydrobromide,

(35) N,N-dimethyl-4-methoxy-2,3-dihydro-1H-phenalen-2-ylamine hydrochloride,

(36) 2-(N,N-dimethylamino)-2,3-dihydro-1H-phenalen-4-ol hydrobromide,

(37) [2-(N,N-di-n-propylamino)-2,3-dihydro-1H-phenalen-5-yl]acetate ester,

(38) [2-amino-2,3-dihydro-1H-phenalen-5-yl]-α-benzyl ether hydrochloride,

(39) [2-(N,N-dimethylamino-2,3-dihydro-1H-phenalen-6-yl]phenone maleate salt, hydrochloride,

(40) [2-(N-methylamino)-2,3-dihydro-1H-phenalen-6-yl]phenone methanesulfonate salt,

(41) [2-(N-methylamino)-2,3-dihydro-1H-phenalen-6-yl]-α-benzylether p-toluenesulfonate salt monohydrate,

(42) [2-(N,N-dimethylamino)-2,3-dihydro-1H-phenalen-6-yl]-α-benzyl ether hydrochloride salt,

(43) 2-(N,N-dimethylamino)-2,3-dihydro-1H-phenalen-5-ol,

(44) 2-(N,N-di-n-propylamino)-2,3-dihydro-1H-phenalen-5-ol, 2,2-dimethylpropyl ester hydrochloride,

(45) [2-(N,N-di-n-propylamino)-2,3-dihydro-1H-phenalen-5-yl]benzoate ester hydrochloride,

(46) [2-(N,N-di-n-propylamino)-2,3-dihydro-1H-phenalen-5-yl]heptanoate ester hydrochloride,

(47) [2-(N,N-di-n-propylamino)-2,3-dihydro-1H-phenalen-5-yl]-ethyl carbonate (ester),

(48) [2-(N,N-di-n-propylamino)-2,3-dihydro-1H-phenalen-5-yl]-α-phenylacetate ester hydrochloride, 0.5 $H_2O$ hydrate,

(49) [2-amino-2,3-dihydro-1H-phenalen-6-yl]-α-benzyl ether maleate salt,

(50) 5-chloro-2,3-dihydro-1H-phenalen-2-ylamine hydrochloride,

(51) 6-chloro-N,N-di-n-propyl-2,3-dihydro-1H-phenalen-2-ylamine hydrochloride,

(52) 6-chloro-N,N-dimethyl-2,3-dihydro-1H-phenalen-2-amine hydrochloride,

(53) N,N-di-n-propyl-2,3-dihydro-1H-phenalen-2-amine hydrochloride,

(54) 1-[2,3-dihydro-1H-phenalen-2-yl]azetidine succinate salt,

(55) N,N-diethyl-2,3-dihydro-1H-phenalen-2-amine hydrochloride, 0.25 $H_2O$ hydrate,

(56) N-n-propyl-2,3-dihydro-1H-phenalen-2-amine hydrochloride, and

(57) N-ethyl-2,3-dihydro-1H-phenalen-2-amine hydrochloride.

Other tests for helping to assess possible antipsychotic drug use activity include tests which (1) determine receptor binding activity of the test compound, or (2) antagonize the action of D-amphetamine. Activity in any one of these four tests indicates possible antipsychotic activity interest, based upon possibly different pharmacological mechanisms of action.

The Receptor Binding Assay test for antipsychotic acitvity, was conducted as follows:

A rat brain homogenate was prepared (1) by homogenizing a whole rat brain minus the cerebellum in 10 ml of 0.05M THAM-HCl, pH 7.4, using a Polytron at a setting of 7 for 30 sec. and centrifuging at 15,000xg for 10 min. The supernatant was discarded, the pellet resuspended in 10 ml of buffer using the Polytron for 10 sec., and the centrifugation repeated. The pellet was resuspended and diluted to 100 times the original tissue weight with buffer. Then 100 ml of the homogenate was diluted to 400 ml with buffer.

The test drug was weighed out in a scintillation vial in a 1-5 mg ammount. They were dissolved by adding 0.5 ml of N-N-dimethylformamide of (DMF) and then 0.05 ml of 1M acetic acid, and vortexed to solubilize the drug. Then 10 ml of deionized and filtered water was added to the required volume. All compounds were tested at a single concentration of 1 uM.

Samples were prepared for incubation using an $^3$H-spiperone (antipsychotic).

Incubation was conducted at 30°C for 30 min. on a shaking water bath. Every 30 sec., 24 tubes were filled with 1 ml of homogenate and put into the bath (this is done every 3 min. until all tubes are in the bath). Upon completion of the incubation procedure, the samples are filtered and rinsed.

The punched filters are dropped into vials, which are in turn filled with ACS liquid scintillation fluid and capped and counted in a liquid scintillation counter. Counting data is recorded and analyzed.

Results are expressed as percent decrease from control. A decrease from control may indicate antipsychotic activity. Data for testing compounds of Examples 38 to 41 are set forth in Table 1.

The methods for preparing the compounds of this invention are further exemplified by the following detailed examples which are not intended as being limiting on the scope of the invention. All temperatures are in degrees Celcius unless otherwise indicated. Letter symbols are used in some places for brevity in references to common chemical reagents and analytical procedures. For example, IR means infrared, UV means ultraviolet, NMR means nuclear magnetic resonance spectral analyses. THF means tetrahydrofuran, ether, used alone, means diethyl ether, petroleum ether means a commercial solvent having the indicated boiling point range. MeOH means methanol, ETOAC means ethyl acetate, and the like.

Example 1 2,3-Dihydro-N,N-dimethyl-1H-phenalen-2-amine, and its monohydrochloride.

Sodium cyanoborohydride (0.42 g; 6.8 mmole) was added to a solution of 2,3-dihydro-N-monomethyl-1H-phenalen-2-amine (0.71 g; 3.4 mmole) and 1.7 ml of 37% aqueous formaldehyde solution in 17 ml of acetonitrile and 6.8 ml of methanol. The pH of the mixture was adjusted to 7 with acetic acid. After stirring the mixture for 25 min, the pH was adjusted to 7 and the mixture was stirred for three hr. An additional 0.42 g of sodium cyano borohydride and 0.17 ml of formaldehyde solution were added. The pH was adjusted and the mixture was stirred overnight. Then the mixture was cooled, acidified to pH 3, and evaporated at 35°C, and the residue was taken up in a diethyl ether/water mixture. The aqueous layer was cooled, basified with 15% w/v sodium hydroxide in water solution and extracted with chloroform. The chloroform layer extract was washed with saturated sodium chloride solution, separated therefrom, and the organic liquid layer was dried with magnesium sulfate, and evaporated to leave as residue 0.4 g of the titled amine as an oil. An NMR spectrum was consistent with the named amine.

The amine oil was dissolved in diethyl ether, the resulting solution was filtered and treated with a hydrogen chloride in diethyl ester solution to form the titled amine hydrochloride salt. The hydrochloride salt was crystallized from a methanol/diethyl ether mixture at -10°C, weight 0.28 g, m.p. 283-284°C (dec.).

UV sh 211 nm (ε 27,050), sh 222 (58,500), λ max 227 (81,400), sh 265 (3,950), 274 (6,350), 284 (7,750), sh 291 (5,550), 296 (5,250),

sh 305 (1,250), sh 309 (666), 316 (570), 321 (577). IR-CH 3055, 3038, 3013: $NH^+$ 2640, 2578, 2525, 2495, 2478; C=C 1620, 1601, 1511, 1494; aromatic 811, 764. Mass spec. $M^+$211.

Anal. Calcd. for $C_{15}H_{17}N{\cdot}HCl$: C, 72.71; H, 7.32; C., 14.31; N, 5.65.

Found: C, 72.56; H, 7.66; Cl, 14.43; N, 5.61.

## 4-SUBSTITUTED SERIES

<u>Example 2</u> 2-Amino-4-methoxy-2,3-dihydro-1H-phenalene, and hydrochloride thereof.

A. Preparation of bis[chloromethyl(phenyl)imine].

A mixture of oxanilide (200 g; 0.82 mole), phosphorus pentachloride (400 g; 1.93 mole) and 600 ml of toluene (dried over 4A molecular sieves) was refluxed for 3 hr to give a deep yellow solution. About 400 ml of solvent was distilled off. Upon cooling the residual reaction mixture in ice, a thick suspension resulted. It was diluted with petroleum ether (b.p. 30-60°C), and filtered. The crystalline intermediate product $(Cl\text{-}C{=}N\text{-}C_6H_5)_2$, named above, was washed well with petroleum ether and dried in vacuo at room temperature, and then weighed. The weight was 152.8 g (70% yield), m.p. 113-114°C.

This is a procedure reported by R. Bauer, in Ber. 40, 2650 (1907), who reported m.p. of 115°C after recrystallization from petroleum ether.

B. Preparation of the 2-methoxy-1,8-naphthenic anhydride.

A suspension of the bis (N-phenyl)(chloromethyl)imine from part A hereinabove, (152.8 g; 0.57 mole) and (180.3 g; 1.14 mole) of 2-methoxynaphthalene in 1200 ml of benzene was added during 75 min to a stirred suspension of aluminum trichloride (151.9 g; 1.14 mole) in 220 ml of benzene. A mild exothermic reaction occurred and the temperature reached 34°C. The resulting dark brown suspension was then stirred for 3.5 hr at room temperature, and then at 50-55°C for 50 min and allowed to stand overnight. The reaction mixture was decomposed with 1 kg of ice and 220 ml of concentrated hydrochloric acid. Benzene was steam-distilled off from the mixture. The resulting suspension was cooled, filtered and the filter solid was washed with water The solid was heated at 90°C and stirred in 1 liter of 40% w/v sodium sulfite solution for 30 min; cooled to room temperature

and the suspension was filtered. This procedure was repeated with another 1 liter of sodium sulfite solution as above. The combined filtrate was acidified with 2.2 liters of concentrated hydrochloric acid. The resulting suspension was allowed to stand for 1 hr, filtered and the filter solid was washed with water to obtain 86 g (71% yield) of the herein sub-titled anhydride compound, m.p. 219-220°C.

This compound was reported by Standinger et al, Helv. Chim. Acta, 4, 342 (1921) as having m.p. 215-216.

C. Preparation of 2-methoxynaphthalic anhydride.

The dione (21.2 g; 0.1 mole) from part B herein in 400 ml of acetic acid was refluxed and cooled to 90°. Sodium dichromatic dihydrate (59.2 g; 0.198 mole) was added portionwise during 5 min, and the mixture was refluxed for 45 min. A solution resulted after the initial 10 min of reflux. It was cooled to 50°, 400 ml of water was added and the resulting suspension was filtered and the solid washed with water to obtain 16.5 g (73% yield) of the sub-titled intermediate compound. m.p. 253-254°. UV λmax 218nm (21,050), 240 (41,950), sh 295 (3,400), 309 (6,500), 323 (9,950), 363 (9,200), 376 (9,200). IR C=O 1731, 1768. NMR ($d_6$DMSO) δ 4.15 (s,3,$OCH_3$), 7.60-8.55 (m,5,aromatic). Mass spec. $M^+$228.

This compound was prepared before by J. Cason et al., J. Org. Chem. 33, 3404 (1968) who used the hydrogen peroxide procedure and reported m.p. 261-262° (from 95% EtOH). In our hands the hydrogen peroxide procedure gave the desired material in poor yield.

D. Dimethyl 2-methoxy-1,8-naphthalate.

A solution of potassium hydroxide (7.9 g; 0.32 mole) in 250 ml of methanol was added during 30 min to a stirred suspension of 2-methoxy-1,8-naphthalic anhydride (11.4 g; 0.05 mole), 250 ml of methanol and dimethylsulfate (39.7 g; 0.31 mole). The temperature was kept at 29° with occasional cooling. A solution resulted after the initial 10 min which then formed a suspension. The mixture was stirred at room temperature for 1 hr, evaporated in vacuo at 30°, and the residue was taken up in diethyl ether and water. The ether extract was washed twice with sodium bicarbonate solution, water, saturated salt solution, dried in magnesium sulfate and concentrated until crystallization started to obtain the above-named di-methyl ester compound as colorless prisms, 11.14 g (87% yield), m.p. 103-104°. UV λmax 233

nm (54,700), 288 (5,450), 297 (5,300), 338 (4,650). IR-CH 3091, 3033, 3013; C=O 1716, 1694; C=C 1618, 1597, 1571, 1510; CH def. 1443; C-O/other 1272, 1239, 1205, 1164, 1149, 1095, 1039, 1003; aromatic 829, 797, 790, 759. NMR ($CDCl_3$ $\delta$ 3.91 (s,6,COOMe), 3.97 (s,3,OMe), 7.26-8.0 (m,5,aromatic). Mass spec. $M^+$274.

Anal. Calcd. for $C_{15}H_{14}O_5$: C, 65.69; H, 5.14.

Found: C, 65.60; H, 5.04.

E. 2-Methoxynaphthalene-1,8-bismethanol.

A solution of the dimethyl ester from part D hereinabove (11.14 g; 0.041 mole) in 180 ml of tetrahydrofuran was added during 30 min to a solution of lithium aluminum hydride (11.14 g) in 270 ml of tetrahydrofuran keeping the temperature at 20°. It was then stirred at room temperature for 2 hr and decomposed in succession with 11.1 ml of water, 11.1 ml of 15% u/v NaOH and 33.3 ml of water. The suspension was stirred for 1 hr, filtered and the cake washed well with tetrahydrofuran. The tetrahydrofuran solution was dried (magnesium sulfate) and evaporated to give 8.7 g of the sub-titled diol (98% yield) which was suitable for the next step. The analytical sample was obtained by crystallization from diethyl ether, m.p. 124-125°. UV sh 216 nm (23,150), λmax 233 (66,750), sh 275 (4,400), 284 (5,750), 296 (5,100), 325 (2,550), 337 (2,800). IR OH 3270, 2732, 2620, 2434; =CH 3057; C=C 1617, 1599, 1576, 1513, 1487; C-O/other 1264, 1252, 1137, 991, 983; aromatic 817, 766. NMR ($CDCl_3$) $\delta$ 3.05 (broad s,2,OH exch), 3.99 (s,3,$OCH_3$), 525, 528 (s,4,$CH_2$), 7.25-7.91 (m,5,aromatic). Mass spec. $M^+$218.

Anal. Calcd. for $C_{13}H_{14}O_3 \cdot 1/6\ H_2O$: C, 70.57; H, 6.52

Found: C, 70.54; H, 6.26.

F. 2-Methoxynaphthalene-1,8-bis (methyl bromide).

A solution of phosphorus tribromide (17 g; 0.063 mole) in 15 ml of benzene was added during 10 min to a suspension of the diol from part E, hereinabove (6.6 g; 0.03 mole) in 150 ml of benzene keeping the temperature at 5°. A dark complex separated on the walls of the flask. The mixture was stirred 30 min at 5°, decomposed with 100 ml of water and stirred for 30 min. The organic layer was separated, the aqueous layer was extracted twice with diethyl ether, and the combined organic layer was washed twice with water, saturated salt solution, dried (magnesium sulfate) and evaporated to give 10.31 g of

amber sub-titled intermediate which was suitable for the next step. It also contained by NMR some of the oxide (2-methoxynaphthalen-1,8-yl(1-ox-3,4,5-ine).

When this reaction was carried out by adding a solution of phosphorus tribromide (4.65 g; 17.2 mmole) in 5 ml of benzene during 10 min to a suspension of the diol (1.8 g; 8.2 mmole) in benzene containing 2 drops of pyridine at 60° and stirring at this temperature for 2.5 hr and worked up as above, the crude product amount to 1.8 g. Crystallization from ether gave 0.6 g of the dibromide (+ filtrate A), m.p. 131-132° unchanged on recrystallization. UV sh 214 nm (70,700), λ max 233 (67,950), sh 275 (4,500), 284 (6,000), 296 (5,350), 324 (2,800), 337 (3,050). IR=CH 3073, 3053; C=C 1615, 1597, 1568, 1513; C-O/other 1267, 1252, 1198, 1152, 1045, 1037; aromatic 830, 814, 796, 763. NMR ($CDCl_3$) δ 4.05 (s,3,$OCH_3$), 5.30 (s broad,2,-$CH_2$), 5.45 (s sharp,2,$CH_2$), 7.2-7.9 (m,5,aromatic). Mass spec. $M^+$344.

Anal. Calcd. for $C_{13}H_{12}Br_2O$: C, 45.38; H, 3.52; Br, 46.45.

Found: C, 45.09; H, 3.45; Br, 46.28.

Filtrate A was evaporated and the residue (1.2 g) was subject to LPLC using 110 ml, Michel-Miller column packed with 230-400 mesh silica gel and 3% ethyl acetate-cyclohexane (10 ml fractions were collected). Fractions 1-10 gave 50 mg (discarded). Fractions 11-30 (6% ethyl acetate-cyclohexane) gave 0.85 g of the colorless crystallization solid above-named oxine by-product. Crystallization from petroleum ether (30-60°) and a few drops of ether gave 0.65 g, m.p. 66-67. UV λ max 232 (63,550), sh 262 (2,450), sh 272 (4,100), 282 (5,850), 294 (5,400), sh 309 (1,350), 322 (2,250), 336 (2,760). IR=CH 3047, 3012; C=C 1622, 1599 1584, 1510; C-O/other 1260, 1243, 1217, 1094, 1074, 1052, 1037; aromatic 823, 764. NMR ($CDCl_3$) δ 3.93 (s,3,$OCH_3$), 4.98 (s,2,$CH_2$), 5.09 (s,2,$CH_2$), 7.0-7.75 (m,5,aromatic). Mass spec. $M^+$200.

Anal. Calcd. for $C_{13}H_{12}O_2$: C, 77.98; H, 6.04.

Found: C, 77.65; H, 6.00.

G. Preparation of 4-methoxy-2,2-bis(ethoxycarbonyl)-2,3-dihydro-1H-phenalene.

A solution of diethylmalonate (4.96 g; 0.031 mole) in 30 ml of tetrahydrofuran was added during 5 min to a suspension of sodium

hydride (1.48 g; 0.031 mole of 50% suspension in mineral oil, washed with petroleum ether (30-60°) in 30 ml of tetrahydrofuran. The mixture was stirred for 30 min. It was cooled to -15°, a solution of the dibromo compound from part F hereinabove (10.3 g; 0.03 mole) in 30 ml of tetrahydrofuran was added during 10 min at -15°, stirred for 2 hr at room temperature. Another 0.031 mole of sodium hydride was added at room temperature. The mixture was stirred 4 hr and allowed to stand over the weekend. It was diluted with diethyl ether, washed twice with water, saturated sodium chloride solution, dried (magnesium sulfate) and evaporated to give 10.3 g of a yellow oil. Chromatography was carried out with 1 kg of silica gel (70-230 mesh) using ethyl acetate cyclohexane (30 ml fractions were collected). Fractions 1-59 gave no material. Fractions 60-82 gave 0.5 g of crystalline compound, m.p. 65-66°, which was identical by NMR to the methoxy-naphthenyl oxine by-product referred to in part F hereinabove. Fractions 83-94 gave no material. Fractions 95-115 (40 ml each) gave 4.38 g (42% yield) of the above sub-titled diester as a colorless oil. UV λ max 232 nm (61,600), sh 275 (4,600), 284 (6,700), 296 (6,600), 324 (2,800), 339 (3,300). IR-CH 3050; CH 2980, 2939, 2906, 2840; C=O 1733; C=C 1623, 1598, 1511; C=C/CH def. 1465, 1445, 1436, 1366; C-O/other 1307, 1260, 1235, 1183, 1142, 1096, 1071, 1055, 1042; aromatic 862, 819, 758. NMR ($CDCl_3$) δ 1.11 (t,6,C-$CH_3$, J=7.1Hz), 3.56 (s,4,C-$CH_2$-C), 3.95 (s,3,$OCH_3$), 4.10 (quad,4,$OCH_2$,J=7.1Hz), 7.17-7.7 (m,5,aromatic).

HR mass spec. Calcd. for $C_{20}H_{22}O_5$: 342.1467.
Found: 342.1445.

H. Preparation of 4-methoxy-2,2-dicarboxy-2,3-dihydro-1H-phenalene.

A solution of potassium hydroxide (1.7 g; 0.031 mole) in 4.3 ml of water was added to a solution of the diester from part G hereinabove (0.54 g; 1.6 mmole) in 5 ml of methanol and refluxed 18 hr. The mixture was cooled to room temperature, filtered to remove a trace of solid, and the clear yellow solution was evaporated in vacuo. The resulting yellow solid was dissolved in 15 ml of water. The solution was extracted twice with diethyl ether (discard ether), and acidified with conc. hydrochloride. The resulting yellow solid was filtered, washed with water and dried at 50° in vacuo to obtain the above sub-titled 2,2-dicarboxylic acid compound; 0.41 g (90%

yield), m.p. 208° efferv. UV λ max 232 nm (61,850), sh 264 (2,550), sh 275 (4,550), 284 (6,300), 297 (6,100), 324 (2,400), 338 (2,800). IR=CH 3015; acid OH 3,000, 2699, 2624, 2541; C=O 1710; C=C 1626, 1602, 1587, 1512; C-O 1281, 1262, 1248; acid OH 940, 928; aromatic 812, 776, 748. NMR ($d^6$DMSO) δ 3,38 (s,broad,$CH_2$+exch.), 3.91 (s,3,-$OCH_3$), 7.20-7.85 (m,5,aromatic). Mass spec. $M^+$286.

Anal. Calcd. for $C_{16}H_{14}O_5$: C, 67.13; H, 4.93.

Found: C, 67.47; H, 4.92.

I. Preparation of 4-methoxy-2-carboxy-2,3-dihydro-1H-phenalene.

The 4-methoxy-2,2-di-carboxy-2,3-dihydro-1H-phenalene (diacid) from part H hereinabove (21.2 g; 0.074 mole), was heated in an oil bath kept at 210-215° for 1 hr to remove one carboxyl group. The resulting brown oil solidified on standing to form the sub-titled compound, 17.9 g (100% yield), m.p. 180-182°. Crystallization from diethyl ether gave colorless small prisms, m.p. 180-181°. UV sh 213 nm (21,600), λ max 232 (64,150), sh 264 (2,600), sh 275 (4,500), 284 (6,500), 297 (6,300), sh 311 (1,600), 324 (2,650), 338 (3,100). IR acid OH 2714, 2660, 2617, 2558; C=O 1730; C=C 1622, 1597, 1587, 1511; C-O 1302, 1257, 1212; acid OH 947; aromatic 812, 785, 760. NMR ($d_6$DMSO) δ 2.35-3.8 (m,6,$CH_2$,CH,exch), 3.91 (s,3,$OCH_3$), 7.20-7.85 (m,5,aromatic).

HR mass spec. Calcd: 242.0943.

Found: 242.0937.

Anal. Calcd. for $C_{15}H_{14}O_3$: C, 74.36; H, 5.82.

Found: C, 74.84; H, 5.79.

J. Preparation of 4-methoxy-2-(tert-butoxycarbonylamino)-2,3-dihydro-1H-phenalene.

Diphenyl phosphoryl azide (2.75 g; 0.01 mole) and triethylamine (1.11 g; 0.011 mole) were added to a suspension of the 4-methoxy-2-carboxy-2,3-dihydro-1H-phenalene acid, from part I hereinabove (2.4 g, 0.01 mole) in 50 ml of tert-butyl alcohol. The mixture was stirred at room temperature for 15 min and then refluxed for 22 hr. The resulting suspension was evaporated to remove solvent, and the residue was taken up in a methylene chloride/water mixture, and filtered to remove a small amount of solid. The organic liquid phase was separated and washed with water, with saturated sodium chloride solution, dried with magnesium sulfate, and evaporated to remove solvent. The residue was extracted with 4 x 100 ml portions of boiling SKELLYSOLVE B brand of hexanes to separate the product form an insoluble yellow oil. The extracts were combined and concentrated to about 50 ml, and the residue was allowed to sit to allow crystallization to occur. There was obtained 2.8 g of the crude hereinabove sub-titled compound, m.p. 146-148°C. After filtration, a second crop weighing 0.12 g, same melting point of the same compound was obtained from the filtrate. This material was pure enough for use in the next step of the process.

To obtain a more pure sample of this compound for analysis, an 0.5 g portion of this material was subjected to low performance liquid chromatography using a 110 ml Michel-Miller column packed with 230-400 mesh size silica gel particles, collecting the recovered liquid therefrom in 10 ml fractions. Franctions 1 to 21, obtained using 5% v/v ethyl acetate in cyclohexane as eluting liquid gave a trace of product. Fractions 33 to 43 using 10% v/v ethyl acetate in cyclohexane as eluting liquid gave 0.43 g of the sub-titled compound as crystals, m.p. 155-156°C, which melting point was unchanged upon recrystallization from SKELLYSOLVE B brand of hexanes.

UV sh 215 nm (23,300), λ max 232 (66,100), sh 275 (4,600), 284 (6,700), 296 (6,500), 324 (2,600), sh 334, 337 (3,050). IR NH 3360; =CH/NH 3055, 3048, 3011; C=O 1678; C=C 1622, 1599, 1586; Amide II 1518; C-O/C-N 1304, 1257, 1234, 1169, 1049; aromatic 812, 750. NMR ($CDCl_3$) δ 1.92 (s,9,t-butyl), 275-3.45 (m,4,$CH_2$), 3.93 (s,3,$OCH_3$), 4.1-4.65 (m broad, 1, CH-N), 7.18-7.75 (m,5,aromatic). Mass spec. $M^+$313.

Anal. Calcd. for $C_{19}H_{23}NO_3$: C, 72.82; H, 7.40; N, 4.47.
Found: C, 72.73; H, 7.55; N, 4.43.

K. 2-Amino-4-methoxy-2,3-dihydro-1H-phenalene, and its hydrochloride.

Trifluoroacetic acid, 50 ml, was added to a 17.7 g (0.056 mole), non-chromatographed portion of the 4-methoxy-2-(tert-butoxycarbonylamino)-2,3-dihydro-1H-phenalene, obtained as described in part J hereinabove. The resulting solution was stirred for 0.5 hr. The solution was quenched with ice, followed by the addition of 20% w/v sodium hydroxide solution, and then the mixture was extracted with chloroform. The chloroform extract phase was washed with water, with

saturated sodium bicarbonate solution, with saturated sodium chloride salt solution, dried with magnesium sulfate and evaporated. The resulting above-named amine compound, as an oil residue, 9.42 g, was again dissolved in 20 ml of chloroform to convert the amine to its hydrochloride salt by shaking the amine in chloroform solution with 50 ml of 10% v/v hydrochloric acid solution. The solid salt was filtered, washed with 5 ml of an ice/cold water mixture and then with diethyl ether. There was obtained 9 g (82% yield of the titled amine-hydrochloride salt, m.p. 273°C (dec.), which melting point was unchanged when the amine salt was recrystallized from a methanol/diethyl ether mixture. UV sh 215 (22,750), λ max 231 (66,100), sh 263 (2,450), sh 274 (4,400), 283 (6,300), 296 (6,000), sh 310 (1,450), 323 (2,458), sh 335; 337 (3,000). IR $NH^+$/=CH 3125, 3011; $NH_3^+$ 2726, 2606, 2566, 2511, 2470; $NH_3^+$/C=C 1618, 1601, 1584, 1556, 1512, 1499; C=O/other 1266, 1262, 1190, 1125, 1053; aromatic 811, 753. NMR ($CD_3OD$) δ 2.75-3.85 (m,5,$CH_2$,CH), 3.85 (s,3,$OCH_3$), 4.85 (s,2,$NH_2$,-exch), 7.2-7.85 (m,5,aromatic). Mass spec. $M^+$213.

Calcd. for $C_{14}H_{15}NO \cdot HCl$: C, 67.33; H, 6.46; N, 5.61; Cl, 14.20.
Found: C, 67.00; H, 6.53; N, 5.52; Cl, 14.54.

The titled amine free base was crystallized from diethyl ether-petroleum ether (b.p. 30-60°), m.p. 103-105°. UV sh 215 (19,650), λ max 231 (56,700), sh 264 (2,300), sh 275 (3,900), 284 (5,550), 296 (5,250), sh 311 (1,350), 323 (2,150), 338 (2,500). IR NH 3329; =CH 3046; bonded NH ~2800 broad; NH def/C=C 1623, 1598, 1586, 1572, 1510; C=O 1260; C-O/C-N 1322, 1126, 1054, 1042; aromatic 818, 810, 780, 754. NMR ($CDCl_3$) δ 1.51 (s,2,$NH_2$,exch), 2.50-3.50 (m,5,$CH_2$,CH), 3.93 (s,3,$OCH_3$), 7.17-7.75 (m,5,aromatic). Mass spec. $M^+$213.

Anal. Calcd. for $C_{14}H_{15}NO$: C, 78.84; H, 7.09; N, 6.57.
Found: C, 78.43; H, 7.08; N, 6.20.

Example 3 2,(N,N-dimethylamino)-4-methoxy-2,3-dihydro-1H-phenalen-4-ol, and its hydrochloride.

Formaldehyde (aqueous 37% solution, 25.2 ml, 0.03 mole) was added to a solution of 2-amino-4-methoxy-2,3-dihydro-1H-phenalene (2.12 g; 0.01 mole) in 105 ml of methanol, and the resulting solution was stirred for 30 min. The solution was cooled to 0°C, and then solid sodium borohydride, (9.46 g; 0.25 mole) was added slowly during 25 min, maintaining the temperature of 10-15°C. The mixture was then

stirred at room temperature for 2.5 hr and evaporated. The residue was taken up in a diethyl ether/water mixture, the aqueous phase was extracted with diethyl ether, and the combined organic extract was washed with saturated salt solution, dried with magnesium sulfate and evaporated to leave 2.25 g of the titled 2-(dimethylamino) compound as a brown oil.

The hydrochloride salt of this amine was prepared in diethyl ether with hydrogen chloride in diethyl ether, and this salt was crystallized from a methanol/diethyl ether mixture, giving 2.21 g γ80% yield) of the titled amine hydrochloride salt, m.p. 234-235°C, which was raised to 235-236° upon recrystallization. UV sh 214 nm (20,750), λ max 233 (62,650), sh 263 (2,450), 273 (4,300), 284 (6,250), 295.5 (6,000), sh 311 (1,350), 324 (2,500), 337 (3,050). IR OH (trace of water) 3413; =CH 3047, 3004; $NH^+$ 2831, 2553, 2520, 2475, 2449; C=C 1626, 1598, 1585, 1514, 1491; C-O/other 1265, 957; Aromatic 826, 811, 783, 767, 758. NMR ($D_2O$) δ 2.88 (s,6,$Nme_2$), 2.75-3.50 (m,-5,$NCH(CH_2)_2$), 3.94 (s,3,$OCH_3$), 7.10-7.80 (m,5,aromatic).

HR mass spec. Calcd. for $C_{16}H_{19}NO$: 241.1467.

Found: 241.1456.

Anal. Calcd. for: $C_{16}H_{19}NO \cdot HCl$: C, 69.16; H, 7.28; Cl, 12.76; N, 5.04.

Found: C, 69.12; H, 7.31; Cl, 12.84; N, 5.21.

Example 4 2-(Di-n-propylamino)-4-methoxy-2,3-dihydro-1H-phenalene, and its hydrochloride.

The 2-amino-4-methoxy-2,3-dihydro-1H-phenalene hydrochloride salt (4 g; 0.016 mole) was released to its free base form using chloroform and 15% w/v sodium hydroxide in water solution.

A mixture of the free base (3.22 g; 15 mmole), 1-bromopropane (7.98 g; 65 mmole), potassium carbonate (8.98 g; 65 mmole) and 90 ml of acetonitrile was refluxed for 20 hr. GC analysis indicated 84% dialkylated and 16% of monoalkylated material; 3.9 g 1-bromopropane, 4.5 g potassium carbonate and 45 ml acetonitrile were added, and the mixture was refluxed 24 hr. GC indicated 100% dialkylation. The mixture was evaporated, the residue taken up in diethyl ether-water and the aqueous phase was extracted twice more with diethyl ether. The organic layer was washed with saturated salt solution, dried (magnesium sulfate), and evaporated to give 4.2 g of a brown oil.

This oil was subjected to LPLC using a 300 ml Michel-Miller column, 230-400 mesh silica gel and $CHCl_3$. Fractions 1-27 (10 ml each) gave an impurity. Fractions 28-67 (20 ml each) gave no material. Fractions 68-100 (20 ml each) gave 3.53 g (74% yield) of the titled 2-(N,N-di-n-propylamine compound as an oil. NMR ($CDCl_3$): $\delta$ 0.90 (t,6,C-$CH_3$,J=7.1Hz), 1.45 (sext.,4,C-$CH_2$-C,J=7.3$H_2$), 2.59 (t,4,NC-$H_2$,J=7.3H), 2.75-3.25 (m,5,N-CH($CH_2$)$_2$), 3.93 (s,3,)$CH_3$), 7.16-7.72 (m,5,aromatic). The hydrochloride was formed in diethyl ether with ethereal hydrochloride and was crystallized from methanol-diethyl ether, m.p. 183-184°. UV sh 215 (28,600), $\lambda$ max 231 (59,950), sh 264 (2,600), sh 274 (4,450), 284 (6,450), 296 (6,300), 324 (2,600), 337 (3,200). IR=CH 3099, 3068, 3054; $NH^+$2390, 2352; C=C 1625, 1598, 1584, 1514, 1490; C-O/other 1265, 1047, 973; aromatic 818, 760. Mass spec. $M^+$297.

Anal. Calcd. for $C_{20}H_{27}NO$ 1.HCl: C, 71.94; H, 8.45; Cl, 10.62; N, 4.20.

Found: C, 71.60; H, 8.58; Cl, 10.70; N, 4.07.

Example 5 2-(Di-n-propylamino)-2,3-dihydro-1H-phenalen-4-ol, as its hydrobromide salt.

A mixture of 2-(di-n-propyl amino)-4-methoxy-2,3-dihydro-1H-phenylene (1 g; 3.4 mmole) and 20 ml of 48% v/v aqueous hydrogen bromide solution was heated for 45 min in an oil bath, kept at 125°C. The resulting solution was evaporated, and the residue was crystallized from a methanol/diethyl ether mixture to obtain 1 g (82% yield) of the titled hydrobromide salt, m.p. 199-200°C. UV $\lambda$ max 231 nm (56,400), sh 272 (4,200), 281 (5,950), 293 (5,700), sh 328 (2,600), 337 (3,050). IR OH 3243; =CH 3057; $NH^+$ 2760, 2723, 2691, 2632, 2561, 2533; C=C 1632, 1621, 1606, 1590, 1530, 1516, 1488; C=O 1280, 1269; aromatic 830, 825, 820, 755. NMR ($CD_3OD$) $\delta$ 1.06 (t,6,$CH_3$,J=9Hz), 1.82 (sextet,4,C-$CH_2$-C,J=8Hz), 2.90-4.0 (m,10,($CH_2$)$_2$CH-N($CH_2$)$_2$, $^+$OH), 7.08-7.70 (m,5,aromatic).

HR Mass spec. Calcd. for $C_{19}H_{25}NO$: 283.1936.

Found: 283.1938.

Anal. Calcd. for $C_{19}H_{25}NO\cdot HBr$: C, 62.63; H, 7.19; Br, 21.93; N, 3.85.

Found: C, 62.51; N, 7.26; Br, 21.63; N, 3.69.

Example 6 2-Dimethylamino-2,3-dihydro-1H-phenalen-4-ol as its hydrobromide salt.

A mixture of 2-dimethylamino-4-methoxy-2,3-dihydro-1H-phenalene (1.4 g; 5.8 mmole) and 20 ml of 48% aqueous hydrogen bromide solution was stirred and heated in an oil bath maintained at 120°C for 45 min. The reaction mixture was evaporated, and the residue was crystallized from a methanol/diethyl ether mixture to obtain 1.36 g of the title compound, m.p. 238-239°C, which was unchanged upon recrystallization.

Second crop: 130 mg, same m.p. Yield = 84%, UV λ max 232 nm (62,000), sh 261 (2,550), 271 (4,200), 281 (6,050), 293 (5,550), sh 327 (2,500), 337 (2,950). IR OH 3251; =CH 3022; $NH^+$ 2706, 2679, 2574, 2521, 2474; C=C 1631, 1610, 1589, 1518, 1485; C-O/other 1278, 951; aromatic 823, 816, 756. NMR ($CDCl_3$-$CD_3OD$) δ 2.98 (s,6,$NMe_2$), 3.0-3.8 (m,5,$NCH(CH_2)_2$), 7.09-7.70 (m,5,aromatic).

HR mass spec. Calcd. for $C_{15}H_{17}NO$: 227.1310.

Found: 227.1307.

Anal. Calcd. for $C_{15}H_{17}NO \cdot HBr$: C, 58.44; H, 5.89; Br, 25.93; N, 4.55.

Found: C, 58.33; H, 5.92; Br, 25.70; N, 4.63.

5-SUBSTITUENT SERIES

Example 7 2-Amino-5-methoxy-2,3-dihydro-1H-phenalene, and its hydrochloride salt.

A. Sulfonation of Naphthalic anhydride to form the 3-(sodium sulfonyloxy)naphthalic anhydride (where the sodium sulfonyloxy group is in the position equivalent to the 5-position of the phenalene ring system.

A mixture of 1,8-naphthalic anhydride (125 g; 0.63 mole) and 500 ml of fuming sulfuric acid (18-24% sulfur trioxide in sulfuric acid) was heated at 130° for 30 min. The resulting reaction mixture was poured onto 1100 g of ice and cooled in an ice bath for about 3 hr undisturbed to allow the formation of large crystals. The crystals were filtered, dissolved in 500 ml of water, and the solution was basified to about pH 10 with solid sodium hydroxide while cooling in ice. The resulting brown solution was taken to dryness on a rotary evaporator. The residual solid was dried in vacuo at 100°C to give 245 g (theory 180 g) of the crude titled intermediate (A).

B. Preparation of 3-hydroxy-1,8-naphthalic anhydride.

A mixture of potassium hydroxide (986 g; 17.6 mole) and 20.8 ml of water was heated at 185°C in a stainless steel beaker until a solution resulted (ca. 35 min). The crude sodium salt (0.63 mole) from part A hereinabove was added while stirring with a TEFLON rod, and the temperature was raised to 220-225°C for 25 min. A thick yellow suspension resulted. It was cooled to 160°C, 1 kg of ice was added, then 1 l of water. The mixture was cooled to 50°C and filtered to remove some insoluble material. The filtrate was cooled and acidified with concentrated hydrochloric acid to about pH 1 (about 1500 ml). The resulting suspension was stirred to 90°C on the steam-bath for 10 min, cooled, filtered and the sub-titled yellow solid product was washed twice with water and dried overnight at 100°C, 105 g (yield 78%), m.p. 273-274°.

C. Mixture of dimethyl ester of 3-methoxy-1,8-naphthalic acid and dimethyl ester of 3-hydroxy-1,8-naphthalic acid.

Dimethyl sulfate (187 g; 1.48 mole) was added to a suspension of 3-hydroxy-1,8-naphthalic anhydride (51 g; 0.24 mole) from Part B hereinabove in 1200 ml of methanol. A solution of potassium hydroxide (86.8 g; 1.55 mole) in 1200 ml of methanol was added during 40 min, keeping the temperature at 25°C, and stirring was continued at 25°C for 2 hr. Halfway through the addition of the potassium hydroxide solution, a reaction mixture solution was obtained followed by a suspension. The resulting mixture was evaporated in vacuo at 35°C and the residue was taken up in 300 ml each of water and diethyl ether. The aqueous layer was separated and extracted with diethyl ether (2 x 100 ml batches) and the combined ether washes and ether liquid phase was washed with 5% potassium hydroxide (4 x 50 ml portions, ice cold and the extracts were saved), and then with water and saturated sodium chloride solution, and then dried with magnesium sulfate, and evaporated. The residue, 58.8 g (74% yield) the crude sub-titled dimethyl-5-methoxy compound. This intermediate compound is suitable for use in the next, lithium aluminum hydride reduction step.

Crystallization from 250 ml of methanol at -70° gave 40.6 g (62% yield) of colorless crystals, m.p. 70-71°. UV sh 221 (27,800), λ max 235 (33,900), 291 (5,600), 296, (5,650), sh 331 (3,650), 340

(4,000). IR-CH 3073; C=O 1716, 1697; C=C 1619, 1599, 1585, 1512, 1446, 1380; C-O 1287, 1210, 1152; other 892, 859, 801, 781. NMR ($CDCl_3$) δ 3.89 and 3.93 (two s, 9, $OCH_3$), 7.25-7.9 (m,5,aromatic). Mass spec. $M^+$274.

Anal. Calcd. for $C_{15}H_{14}O_5F$: C, 65.69; H, 5.14.

Found: C, 65.31; H, 5.35.

The above potassium hydroxide extract was washed once with ether (discard ether), cooled in ice, acidified with 10% Hydrogen chloride to pH 1, and extracted well with ether. The organic extract was washed with water, saturated salt solution, dried (magnesium sulfate) and evaporated to give 12 g of dimethyl 3-hydroxy-1,8-naphthalene dicarboxylate. The analytical sample was obtained by two recrystallizations from ether; m.p. 153-154°. UV sh 219 (26,950), δ max 236 (30,350), 293 (5,400), 301 (5,450), 344 (4,400). IR OH 3318; =CH 3089, 3065, 3043; C=O 1714, 1710, 1693; C=C 1623, 1578, 1508, 1445; C-O/other 1309, 1279, 1255, 1206, 1148, 1012, 881, 777, 751. NMR ($CDCl_3$) δ 3.88, 3.90 (two s,6,$OCH_3$), 6.6 (s,1,OH exch), 7.45-8.1 (m,5,aromatic). Mass spec. $M^+$260.

Anal. Calcd. for $C_{14}H_{12}O_5$: C, 64.61; H, 4.65.

Found: C, 64.23; H, 4.57.

D. Preparation of 3-methoxy naphthalene-1,8-bis methanol.

A solution of dimethyl-3-methoxynaphthalate ester (crude, 50 g, 0.18 mole) in 800 ml of tetrahydrofuran (THF) was added during 25 min to a solution of lithium aluminum hydride (LAH) (50 g) in 1200 ml of THF, keeping the temperature at 20°C. The mixture was stored at room temperature for 2.5 hr and was decomposed in succession with 50 ml of water, 50 ml of 15% w/v sodium hydroxide in water solution and 150 ml of water, and stirred for one hour at room temperature (RT). The suspension was filtered, the filter cake was washed with THF (3 x 300 ml), the combined filtrate and THF washings was dried with magnesium sulfate and evaporated to leave as residue 31.8 g (81% yield) of the titled diol intermediate, m.p. 155-156°C.

Crystallization from ether gave colorless needles, m.p. 154-155°. UV λ max 233 (11,750), sh 264 (2,950), sh 268 (4,450), 277 (5,650), 293 (4,800), 317 (1,850), 331 (2,350). IR OH 3342, 3249; =CH 3069, 3054; C=C 1616, 1605, 1589, 1509; C-O 1162, 1078, 1063, 1012, 997, 989; aromatic 886, 847, 767. NMR ($CDCl_3$) δ 2.82 (2,-

broad,OH exchangable), 3.91 (3,s,3.91), 5.20 (4,s,$CH_2$), 7.1-7.75 (5,m,aromatic). Mass spec. $M^+$218.

Anal. Calcd. for $C_{13}H_{14}O_3$: C, 71.54; H, 6.47.

Found: C, 71.35; H, 6.46.

E. Preparation of 3-methoxynaphthalene-1,8-bis(methyl bromide).

A solution of phosphorus tribromide (5.68 g; 0.021 mole) in 5 ml of benzene was added during 5 min to a suspension of the diol from part D, hereinabove (2.2 g; 0.01 mole) in 50 ml of benzene. An oily complex was formed. The mixture was stirred at room temperature for 18 hr. Ice water (25 ml) was added, stirring continued for 30 min, and the organic layer was separated. The aqueous layer was extracted with diethyl ether (2 x 25 ml), and the combined organic solution was washed with water, saturated sodium bicarbonate solution (25 ml), saturated salt solution, dried (magnesium sulfate) and evaporated to leave the sub-titled compound as a colorless solid, 2.45 g (72% yield), m.p. 118-122°.

The analytical sample was prepared from diethyl ether at -18°, colorless prisms, m.p. 129-130°. UV sh 230 (33,500), δ max 239 (34,450), sh 296 (7,050), 304 (7,200), 333 (3,950), 344 (4,350). IR=CH 3059, 3052; C=C 1619, 1603, 1511; C-O 1282, 1206, 1706; aromatic 853, 766. NMR ($CDCl_3$) δ 3.91 (s,3,$OCH_3$), 5.22 and 5.24 (s,4,$CH_2$), 7.14-7.80 (m,5,aromatic).

HR mass spec. theory for $C_{13}H_{12}Br_2O$: 341.9256.

Found: 341.9241.

F. Preparation of

(1) 5-Methoxy-2-bis(ethoxycarbonyl)-2,3-dihydro-1H-phenalene, and

(2) 5-Methoxy-2-(ethoxycarbonyl)-2,3-dihydro-1H-phenalene.

A solution of diethyl malonate (64.4 g; 0.401 mole) in 225 g of THF was added during 15 min to a suspension of sodium hydride (HaH) (36.8 g; 0.766 mole of a 50% dispersion in mineral oil, and washed with petroleum ether (having a b.p. of 30-60°C) in 550 ml of THF, and then the mixture was heated at 95°C for 30 min. The mixture was cooled, a solution of the bis(methyl bromide) compound from part E hereinabove (125.5 g; 0.365 mole) in 550 ml of THF was added during 35 min, and then the mixture was refluxed for 3 hr and then evaporated. The resulting residue was taken up in a diethyl ether/water

mixture, and the aqueous phase was separated and washed twice with diethyl ether. The ether washings and diethyl ether phase were combined and washed with water, saturated sodium bicarbonate aqueous solution, and saturated sodium chloride in water solution, and then dried with magnesium sulfate, and evaporated to leave as residue 115 g of the above mixed compounds as a yellow oil. This yellow oil was chromatographed through 2500 g of silica gel using 10% ethyl acetate v/v in cyclohexane mixture as eluting liquid, collecting 40 ml fractions after the initial 4 liters of liquid passed through the column. Fractions 64-82 gave 5.4 g of the sub-titled compound (2) hereinabove.

Crystallization of that titled compound (2) from a diethyl ether/petroleum ether (b.p. 30-60°C) mixture gave more pure compound (2) as colorless rod crystals, m.p. 68.5-70°C.

UV λ max 233 (58,700), sh 259 (2,900), 268 (4,450), 279 (5,800), 291 (5,000), 318 (1,750), sh 328 (1,700), 333 (2,450). IR-CH 3021; C=O 1730; C=C 1619, 1605, 1592, 1508; C-O 1282, 1256, 1195, 1186, 1160, 1017; aromatic 851, 842, 832, 763. NMR ($CDCl_3$) δ 1.29 (t,3,C-$CH_3$,J=7.1Hz), 3.06-3.3 (m,5,$2CH_2$+CH), 3.89 (s,3,$OCH_3$), 4.20 (q,2,-$OCH_2$, J=7.1Hz), 6,95-7.34 (m,5,aromatic). Mass spec. $M^+$270.

Calcd. for $C_{17}H_{18}O_2$: C, 75.53; H, 6.71.

Found: C, 75.33; H, 6.69.

Chromatography fractions 110 to 187 gave 58 g (46% yield) of the above titled diethyl ester compound (1) above, which was suitable for the next process step.

It crystallized from diethyl ether-petroleum ether 30-60°, colorless needles, m.p. 62-63°. UV λ max 232 (53,650), sh 259 (2,950), sh 269 (4,400), 280 (5,800), 292 (5,150), 319 (1,750), sh 328 (1,700), 334 (2,500). IR-CH 3058, 3035; C=O 1742, 1714; C=C 1623, 1603, 1507; C-O 1316, 1287, 1255, 1220, 1200, 1193, 1159, 1098, 1085, 1050, 1034; aromatic 854, 835, 762. NMR ($CDCl_3$) δ 1.12 (t,6,C-$CH_3$,J=7.1Hz), 3.57 (s,4,C-$CH_2$), 3.88 (s,3,$OCH_3$), 4.10 (q,4,$OCH_2$,-J=7.1Hz), 6.97-7.60 (5,m,aromatic). Mass spec. $M^+$342.

Anal. Calcd for $C_{20}H_{22}O_5$: C, 70.16; H, 6.48.

Found: C, 69.96; H, 6.50.

G. Preparation of 5-methoxy-2,2-bis(carboxy)-2,3-dihydro-1H-phenalene.

A mixture of 58 g (0.169 mole) of the 6-methoxy-2,2-bis(ethoxycarbonyl)-2,3-dihydro-1H-phenalene diester from step F hereinabove, 94.6 g of potassium hydroxide (1.69 mole) and 460 ml each of water and methanol was refluxed for 2 hr. A solution resulted after 30 min. The resulting solution was evaporated and the residue was dissolved in 500 ml of water. This resulting solution was extracted once with diethyl ether, the ether wash was discarded, and the washed solution was acidified with concentrated hydrochloric acid solution to pH 1. The resulting oily mixture was stirred for 1 hr to give a suspension. The suspension was filtered, the filter solid was washed with water and dried in vacuo at 50°C overnight to obtain 45.03 g (93% yield of the sub-titled di-carboxylic acid, m.p. 201°C (efferv.).

UV λ max 232 (55,100), sh 258 (2,850), 259 (4,450), 279 (12,250), 291 (5,150), 318 (1,800), 333 (2,550). IR acid OH 3000 b., 2724, 2622, 2522; C=O 1708; C=C 1624, 1606, 1589, 1508; C-O 1288, 1277, 1262, 1246, 1202, 1161; acid OH 935; aromatic 840, 764. NMR ($CDCl_3$) δ 3.58 (s,4,$CH_2$). 3,91 (s with broad base,5,$OCH_3$+OH exchangable), 6.97-7.6 (m,5,aromatic).

HR mass spec. theory for $C_{16}H_{14}O_5$: 286.0841.

Found: 286.0851.

Anal. Calcd. for $C_{16}H_{14}O_5 \cdot 1/4H_2O$: C, 66.08; H, 5.02.

Found: C, 65.89; H, 4.79.

H. Preparation of 2-carboxyl-5-methoxy-2,3-dihydro-1H-phenalene.

The solid di-carboxylic acid, 5-methoxy-2,2-di-carboxy-2,3-dihydro-1H-phenalene, from part G hereinabove, was decarboxylated (one carboxyl group removed) by heating the di-acid in an oil bath at 190-210°C for 50 min. The resulting liquid was cooled, and the solid was ground to a fine powder to obtain 37.1 g (98% yield) of the sub-titled 2-mono-carboxy compound, m.p. 194-196°C.

UV λ max 231 (57,650), sh 258 (2,850), 269 (4,400), 279 (5,750), 290 (5,000), 317 (1,700), 332 (2,400). IR acid OH ~ 3000b, 2690, 2636; C=O 1700-1694; C=C 1624, 1601, 1508; C-O 1295, 1281, 1255, 1216, 1203, 1159; acid OH 941; aromatic 850, 833, 765, 704. NMR ($d_6$DMSO) δ 2.95-3.17 (m,5,2$CH_2$+CH), 3.85 and 3.88 (two s,4,$OCH_3$+OH exchangable), 7.0-7.45 (m,5,aromatic).

HR mass spec. theory for $C_{15}H_{14}O_3$: 242.0943.

Found: 242.0934.

Anal. Calcd. for $C_{15}H_{14}O_3 \cdot 1/6H_2O$: C, 73.46; H, 5.89.

Found: C, 73.53; H, 5.91.

I. Preparation of

(1) 5-Methoxy-2-(tert-butoxycarbonylamino)-2,3-dihydro-1H-phenalene (tert-butyl carbamate derivatives)

and

(2) N,N'-bis(5-methoxy-2,3-dihydro-1H-phenalen-2-yl)urea, as a 0.5 hydrate.

A mixture of the 5-methoxy-2,3-dihydro-1H-phenalen-2-yl-carboxylic acid, from step H hereinabove, (37.1 g; 0.153 mole), 42.1 g (0.153 mole) of diphenyl phosphoryl azide, 17 g (0.168 mole) of triethylamine and 1950 ml of dry tert-butyl alcohol was refluxed for 21 hr. The initially formed solution became a suspension. The solvent was evaporated in vacuo and the residue was taken up in a chloroform/water mixture. The resulting suspension was filtered to remove the above named insoluble urea, (7.29 g;, see below). The filtrate was separated, the aqueous phase was extracted with chloroform, and the combined organic liquid phases was washed with 5% w/v sodium hydroxide aqueous solution (3 x 100 ml), with water and with saturated sodium chloride salt solution and then dried over magnesium sulfate and evaporated. The resulting residual yellow solid, 35.9 g, was extracted with boiling SKELLYSOLVE B brand of hexanes (5 x 200 ml), the resulting solution was concentrated to about 300 ml, clarified with diethyl ether and allowed to crystallize at 0°C to obtain 28.6 g of the above tert-butyl carbamate compound, m.p. 113-115°C.

The second crop: 1.9 g, yield: 64%. UV λ max 233 (60,700), sh 260 (3,000), 269 (4,550), 279 (5,900), 291 (5,200), 317 (1,800), 332 (2,500). IR NH 3356, 3314; =CH/NH 3069, 3050, 3014; impurity: 2178, 2137; C=O 1674; C=C 1627, 1606, 1592; amide II 1518; C-O/C-N 1306, 1275, 1236, 1163, 1050; aromatic 853, 828, 758, 701. NMR ($CDCl_3$) δ 1.41 (s with broad base, 10, t-butyl+NH exchangable), 2.85-3.40 (m,4,$CH_2$), 3.89 (s,3,$OCH_3$), 4.1-4.4 (m,1,CH-N), 6.85-7.40 (m,5,aromatic).

HR mass spec. theory for $C_{19}H_{23}NO_3$: 313.1678.

Found: 313.1684.

The above insoluble urea was crystallized from N,N-dimethylsulfoxide, pale yellow needles, m.p. 308°. UV (DMSO) λ max 273 (9,800), 283 (12,700), 294 (11,300), 320 (3,900), 334 (5,150). IR NH 3331; =CH/NH 3102, 3053; C=O 1618; C=C 1603, 1591, 1519; amide II 1569; C-O/C-N 1258, 1197, 1160; aromatic 847, 823, 751. NMR ($d_6$DMSO) δ 2.7-3.2 (m,4,$CH_2$), 3.83 (s,6,$OCH_3$), 3.9-4.25 (m,2,CH-N), 5.83 and 5.92 (s,2,NH), 6.90-7.7 (m,10,aromatic). Mass spec. $M^+$452.

Anal. Calcd. for $C_{29}H_{28}N_2O_3 \cdot 0.5H_2O$: C, 75.46; H, 6.33; N, 6.07.

Found: C, 75.66; H, 6.17; N, 5.98.

J. Preparation of 2-amino-5-methoxy-2,3-dihydro-1H-phenalene, and its hydrochloride.

Trifluoroacetic acid, 85 ml, was added to 30.5 g (0.0958 mole) of the tert-butyl carbamate compound, prepared as described in part I hereinabove. The resulting solution was stirred for 20 min. Ice was then added, and the resulting mixture was made pH basic with 20% w/w sodium hydroxide in water solution and stirred for 1 hr at room temperature. The mixture was extracted well with chloroform, the chloroform extract was separated and washed with water, with saturated sodium chloride solution; dried with magnesium sulfate, and evaporated to give 18.8 g (92% yield) of the above sub-titled amine as a brown oil.

This amine oil was converted to its hydrochloride salt in methanol with 1.5 N hydrogen chloride in diethyl ether solution to give 18.11 g of the above titled hydrochloride as colorless needle crystals, m.p. 252°C (dec.). It was associated with about 0.25 water of hydration.

UV λmax 233 (58,950), 258 (2,850), 269 (4,300), 278 (5,450), 290 (4,600), 318 (1,650), 333 (2,300). IR $NH_3^+$2786, 2691, 2614, 2531, 2487, 2033; $NH_3^+$/C=C 1621, 1607, 1591, 1515, C-O 1263, 1195, 1168, 1161, 1102, 1026; aromtic 834,751. NMR ($CDCl_3$+$CD_3OD$) δ 3.25-3.45 (m,7,$CH_2$,CH,$NH_2$), 3.89 (s,3,OCH3), 6.85-7.65 (m,5,aromatic).

HR mass spec. theory for $C_{14}H_{15}NO$: 213.1154.

Found: 213.1168.

Anal. Calcd. for $C_{14}H_{15}NO \cdot HCl \cdot 1/4H_2O$: C, 66.27; H, 6.55; Cl, 13.97; N, 5.52.

Found: C, 66.50; H, 6.56; Cl, 14.30; N, 5.42.

K. 2-Dimethylamino-5-methoxy-2,3-dihydro-1H-phenalene, and its maleate salt.

To a 2.46 g (0.0115 mole) portion of 2-amino-5-methoxy-2,3-dihydro-1H-phenalene prepared as described in part J hereinabove, released from its hydrochloride salt, in 120 ml of methanol, there was added 29 ml (0.36 mole) of 37% formaldehyde solution. The mixture was stirred for 15 min. Then 10.8 g (0.288 mole) of sodium borohydride was added portionwise over 15 min, keeping the temperature of the mixture at about 10°C by ice-cooling. The mixture was allowed to warm to room temperature which took about 30 min, and was stirred for another 2.5 hr. The mixture was evaporated in vacuo at 30°C, and the residue was taken up in a diethyl ether/water mixture. The aqueous phase was extracted twice with diethyl ether, and the combined ether phase was washed with water, saturated sodium chloride solution and dried with magnesium sulfate, and the solvent was evaporated to leave as residue 2.76 g of the titled dimethylamine derivative compound.

This amine residue was dissolved in diethyl ether and treated with a solution of 1.33 g (0.0115 mole) of maleic acid in diethyl ether which resulted in the formation of a crude gum maleate amine salt. The resulting crude gum was dissolved in a methanol/diethyl ether mixture and the above named maleate salt crystallized therefrom as colorless needles, m.p. 138-140°C, 3.3 g (81% yield).

UV λmax 233 (59,800), sh 259 (3,400), 269 (4,650), 279 (5,800), 291 (4,950), 318 (1,800), 333 (2,400). IR-CH 3055, 3025; $NH^+$/acid OH 2630, 2365; C=O 1706; C=C/$CO_2$-1624, 1605, 1578, 1536; C-O 1197, 1162; aromatic 874, 830. NMR ($CDCl_3$-$CD_3OD$) δ 2.97 (s,6,$NMe_2$), 3.2-3.7 (m,5,$CH_2$+CH), 3.9 (S,3,$OCH_3$), 6.25 (s,2=CH), 6.95-7.75 (m,5,aromatic). Mass spec. $M^+$241.

Anal. Calcd. for $C_{16}H_{19}NO \cdot C_4H_4O_4$: C, 67.21; H, 6.49; N, 3.92.
Found: C, 67.03; H, 6.59; N, 3.79.

Example 8 2-(Dimethylamino)-2,3-dihydro-1H-phenalen-5-ol, and its hydrobromide salt.

A mixture, the 2-(dimethylamino)-5-methoxy-2,3-dihydro-1H-phenalene, prepared as described in Example 1 hereinabove, 2.07 g (8.6 mmoles), released from its maleate salt, and 35 ml of 48% v/v aqueous hydrogen bromide solution was heated at 125-130°C under a nitrogen

atmosphere for 20 min. A thin layer chromatography (TLC) analysis of a sample of the reaction mixture indicated complete reaction after 15 min. The resulting solution was evaporated. The residue was dissolved in 10 ml of methanol and then diethyl ether was added until the mixture became cloudy. The purple gummy oil which resulted was separated by filtration through a filter aid, CELITE®. The yellow filtrate was diluted with diethyl ether to cloudiness, seeded and allowed to crystallize at room temperature. The resulting crystalline solid product was separated from supernatent liquid and washed with a methanol/diethyl ether (1:3 v/v mixture) to obtain 1.23 g (46% yield) of the titled amine hydrobromide salt, m.p. 226-227°C (dec.).

UV λmax 232 (55,900), sh 262 (2,500), 277 (4,150), 282 (5,600), 294 (5,050), 324 (2,150), 336 (2,600). IR OH 3238; =CH 3025; $NH^+$ 2728, 2689, 2473; C=C 1627, 1608, 1593, 1511, 1496; C-O 1290, 1277, 1268, 1162, 1156, 978; aromatic 868, 775, 771. NMR ($CDCl_3$ + $CD_3OD$) δ 2.92 (s,6,NMe2), 3.25-3.9 (m,6,$CH_2$+CH+OH exchangeable), 6.97-7.60 (5,m,aromatic).

HR mass spec. theory for $C_{15}H_{17}NO$:227.1310.

Found: 227.1330.

Anal. Calcd. for $C_{15}H_{17}NO \cdot HBr$: C, 58.44; H, 5.89; Br, 25.93; N, 4.55.

Found: C, 57.98; H, 5.96; Br, 26.18; N, 4.81.

Example 9 2-(Di-n-propylamino)-5-methoxy-2,3-dihydro-1H-phenalene, and its hydrochloride salt.

A mixture of 2-amino-5-methoxy-2,3-dihydro-1H-phenalene, 2.6 g (0.0122 mole), released from its hydrochloride, 6.46 g (0.0525 mole) of n-propyl bromide, 7.25 g (0.0525 mole) of potassium carbonate and 50 ml of acetonitrile was refluxed for 20 hr. The progress of the amine-alkylation reaction was followed by the aid gas chromatography (GC). Another 3.3 g of n-propyl bromide, 3.6 g of potassium carbonate and 25 ml of acetonitrile were added to the reaction mixture. The mixture was refluxed for 26 hr. GC analysis of a sample of the reaction mixture indicated complete reaction. The mixture was evaporated, and the residue was taken up in a diethyl ether/water mixture. The organic liquid layer was washed with saturated sodium chloride (salt) solution and dried with magnesium sulfate, and evaporated. The residue was dissolved in petroleum ether (bp 30-60°C),

and filtered from some insoluble brown material, and the filtrate was evaporated to leave the titled 2-dipropylamine)-compounds as residue. The residue was dissolved in diethyl ether, treated with ethereal hydrogen chloride which resulted in the formation of the crude gummy solid hydrogen chloride salt. This gummy solid was dissolved in and crystallized from a methanol/diethyl ether mixture to form 2.67 g (66% yield) of the titled compound, as its hydrochloride salt, m.p. 196-197°C.

UV λmax 232 (56,150), sh 259 (3,000), sh 269 (4,450), 279 (5,850), 290 (5,100), 318 (1,850), 333 (2,550). IR-CH 3046; $NH^+$ 2731, 2605, 2529, 2471; C=C 1623, 1602, 1590, 1513; C-O 1262, 1199, 1184, 1023, 999, 977; aromatic 838, 768. NMR ($CDCl_3$) δ 1.04 (t,6,C-CH3, J=7.2 Hz), 1.94 (sextet, 4, C-$CH_2$-C, J = 7.2 Hz), 2.95-3.70 (m,9,$NCH_2$, $NCHCH_2$), 3.90 (s,3,$OCH_3$), 6.90-7.70 (m,5, aromatic). Mass spec. $M^+$297.

Anal. Calcd. for $C_{20}H_{27}NO \cdot HCl$: C, 71.94; H, 8.45; Cl, 10.62; N, 4.20.

Found: C, 71.65; H, 8.49; Cl, 10.99; N, 4.05.

Example 10 2-(Di-n-propylamino)-2,3-dihydro-1H-phenalen-5-ol hydrobromide.

A mixture of 2-(Di-n-propylamino)-5-methoxy-2,3-dihydro-1H-phenalene, (1,65 g; 5.55 mmoles), released from the hydrochloride salt, and 20 ml of 48% aqueous hydrogen bromide solution was heated for 15 min in an oil bath at 125-130°C. A tlc analysis of a sample of the reaction mixture indicated a complete reaction had occurred. The reaction mixture was evaporated, the residue was dissolved in a minimum amount of methanol, diethyl ether was added until the mixture became cloudy, and the mixture was filtered through a filter aid (CELITE) to remove some oily impurity. The filtrate was diluted with diethyl ether and seeded.

There was obtained 1.28 g (63% yield) of the titled compound as its hydrobromide salt, m.p. 233-234°C (dec.).

UV λmax 231 (54,150, sh 262 (2,650), sh 273 (4,200), 282 (5,800), 294 (5,300), 324 (2,250), 336 (2,700). IR OH 3167; $NH^+$2738, 2647, 2520; C=C 1629, 1622, 1605, 1593, 1513; C-O 1279, 1166, 977; aromatic 860, 798, 777. NMR ($CDCl_3$ + $CH_3OD$) δ 1.07 (t,6,C-$CH_3$), 1.90

(sextet,4,C-$CH_2$-C, J=7.2Hz),3.0-4.0 (m,9,$CH_2$-CH-N-$CH_2$), 4.26 (s,1,OH exchangeable), 6.85-7.6 (m,5,aromatic).

HR mass spec. theory for $C_{19}H_{25}NO$: 283.1936.

Found: 283.1927.

Anal. Calcd. for $C_{19}H_{25}NO \cdot HBr$: C, 62.63; H, 7.19; N, 3.85.

Found: C, 62.67; H, 7.14; N, 3.86.

Example 11 Resolution of 2-(di-n-propylamino)-5-methoxy-2,3-dihydro-1H-phenalene into its dextro (+) and levo (-) stereo isomers.

A solution of (+) di-p-toluoyl-D-tartaric acid (141.9 g, 0.351 mole) of one liter of isopropanol was added to a solution of (dl)-2-(di-n-propylamino)-5-methoxy-2,3-dihydro-1H-phenolene (104 g, 0.341 mole) in 350 ml of isopropanol. An additional 400 ml of isopropanol was added and the mixture was warmed to achieve solution. Crystallization began shortly afterwards. The mixture was allowed to stand overnight, and the solid collected by filtration, 206, mp 154-155° (+filtrate A). The solid was recrystallized nineteen times, each time converting a sample to the free base, then hydrochloride and checking the rotation. When $[\alpha]_D^{25}$ + 11.22 (c 0.74, methanol) was observed, the di-p-toluoyl tartarate was recrystallized 4 times without any further change in the rotation of the hydrochloride. The (+) di-p-tolyl-D-tartaric acid salt melted at 162-163° dec., UV, IR and mass spec. were in accord. $[\alpha]_D^{25}$ + 82.41 (c 0.705, MeOH).

Anal. Calcd. for $C_{20}H_{27}NO \cdot C_{20}H_{18}O_8$: C, 70.26; H, 6.63; N, 2.05.

Found: C, 70.20; H, 6.66; N, 1.98.

The original filtrate A (containing the levo(-)isomer) was combined with three additional filtrates, evaporated, and basified to give 58.1 g (0.195 mole) of the free base. Treatment (-) di-p-toloyl-1-tartaric acid (78.9 g, 0.195 mole) as described above, followed by a similar recrystallization sequence gave the (-) di-p-toloyl-1-tartaric acid salt, mp 163-164°C. UV, IR and mass spec. were in accord $[\alpha]_D^{25}$ (C -82.83 (c 0.635, methanol).

Anal. Calcd. for $C_{20}H_{27}NO \cdot C_{20}H_{18}O_8$: C, 70.26; H, 6.63; N, 2.05.

Found: C, 70.26; H, 6.61; N, 1.97.

The above salt is released to the free base and converted to the hydrochloride which had an $[\alpha]_D^{25}$ -11.02 (c 0.735, methanol).

Example 12 Cleavage of methoxy group on resolved isomers.

A. (+) 2-(di-n-propylamino)-5-hydroxy-2,3-dihydro-1H-phenalene.

A mixture of the (+) 2-(di-n-propylamino)-5-methoxy-2,3-dihydro-1H-phenalene free base enantiomer (1.6 g; 5.35 mmole) and 17 ml of 48% aqueous hydrobromic acid was stirred and heated in an oil bath maintained at 125-130°C for 20 min. The suspension was evaporated down to 0.1 mm Hg pressure, and the residue solid was crystallized rapidly from a methanol/diethylether mixture to obtain 1.65 g (84% yield) of the titled (+) enantiomer compound, m.p. 247-248°C (dec.); $[\alpha]_D^{25}$ + 12.08 (co.605, methanol).

The UV, IR, proton NMR and CD analyses were in accord.

Mass spec. Found: 283.1930.

Calcd. for $C_{19}H_{25}NO$: 283.1936.

HPLC on Water's model 660 ($C_{18}$ column, mobile phase 75%-acetonitrile - 10% phosphate buffer, compound dissolved in methanol showed 99.4% of 9.24 min.

Anal. Calcd. for $C_{19}H_{25}NO \cdot Br$: C, 62.63; H, 7.19; Br, 21.94; N, 3.85.

Found: C, 62.27; H, 7.27; Br, 21.74; N, 3.88.

B. (-)-2-(Di-n-propylamino)-5-hydroxy-2,3-dihydro-1H-phenalene.

A mixture of the (-) 2-(di-n-propylamino)-5-methoxy-2,3-dihydro-1H-phenalene free base enantiomer (1.24 g; 4.17 mmoles) and 14 ml of 48% aqueous hydrobromic acid was stirred and heated in an oil bath maintained at 125-130°C for 20 min. The resulting suspension was evaporated in vacuo and the resulting solid was crystallized from a methanol/diethyl ether mixture to obtain 1.24 g (82% yield) of the (-) enantiomer compound, m.p. 247-248°C (dec.) $[\alpha]_D^{25}$-12.02 (c. 0.595, methanol). The UV, IR proton and CD analyses were in accord with the named compound.

Mass spec. Found: 283.1933.

Calcd. for $C_{19}H_{25}NO$: 283.1936.

HPLC on Water's model 660 ($C_{18}$ column, mobile phase 75%-acetonitrile-10% phosphate buffer, compound dissolved in MeOH) showed 99.7%-acetonitrile-10% phosphate buffer, compound dissolved in MeOH) showed 99.7% at 9.38 mμ.

Anal. Calcd. for $C_{19}H_{25}NO \cdot HBr$: C, 62.63; H, 7.19; Br, 21.94; N, 3.86.

Found: C, 62.64; H, 7.30; Br, 21.64; N, 3.98.

Example 13 Resolution of (±) d-2-(di-n-propylamino)-5-methoxy-2,3-dihydro-1H-phenalene into its dextro (+) and Levo (-) isomers.

A solution of (+) di-p-toluoyl-D-tartaric acid (141.9 g, 0.351 mole) of one liter of isopropanol was added to a solution of (+) 2-(di-n-propylamino)-5-methoxy-2,3-dihydro-1H-phenalene (104 g; 0.341 mole) in 350 ml of isopropanol. An additional 400 ml of isopropanol was added and the mixture was warmed to achieve solution. Crystallization began shortly afterwards. The mixture was allowed to stand overnight, and the solid collected by filtration, 206 g, mp 154-155° (+ filtrate A). The solid was recrystallized nineteen times, each time converting a sample of the tartarate salt to the free base, then to the hydrochloride and checking the rotation thereof. When $[\alpha]_D^{25}$ + 11.22 (c 0.74, MeOH) was observed, the di-p-toluoyl tartarate was recrystallized 4 times without any further change in the rotation of the hydrochloride. The (+) di-p-toloyl-D-tartaric acid salt melted at 162-163° dec., UV, IR and mass spec. were in accord. $[\alpha]_D^{25}$ + 82.41 (c 0.705, MeOH).

Anal. Calcd. for $C_{20}H_{27}NO \cdot C_{20}H_{18}O_8$: C, 70.26; H, 6.63; N, 2.05.
Found: C, 70.20; H, 6.66; N, 1.98.

The original filtrate A was combined with three additional filtrates, evaporated, and basified to give 58.1 g (0.195 mole) of the free base. Treatment with (-) di-p-toluyl-1-tartaric acid (78.9 g, 0.195 mole) as described above, followed by a similar recrystallization sequence gave the (-) di-p-toluyl-1-tartaric acid salt, m.p. 163-164°C. UV, IR and mass spec. were in accord $[\alpha]_D^{25}$ (C -82.83 (c 0.635, MeOH).

Anal. Calcd. for $C_{20}H_{27}NO \cdot C_{20}H_{18}O_8$: C, 70.26; H, 6.63; N, 2.05.
Found: C, 70.26; H, 6.61; N, 1.97.

The above salt is released to the free base and converted to the hydrochloride, $[\alpha]_D^{25}$ -11.02 (c 0.735, MeOH).

Example 14 Cleavage of methyl group from (+)-2-(di-n-propylamino)-5-methoxy-2,3-dihydro-1H-phenalene to form (+)-2-(di-n-propylamino)-5-hydroxy-2,3-dihydro-1H-phenalene.

A mixture of the titled 5-methoxy enantiomer free base (+ isomer, 1.6 g, 5.38 mmole) and 17 ml of 48% aq HBr was stirred and heated at 125-130°C (oil bath T) for 20 min. The suspension was

evaporated at 0.1 mm, Hg and the resulting solid was crystallized rapidly from methanol-diethyl ether, 1.65 g (84% yield), m.p. 247-248° dec. $[\alpha]_D^{25}$ + 12.08 (c 0.625, $CH_3OH$).

UV, IR, proton NMR, and CD were in accord.

Mass spec. Found: 283.1930.

Calcd. for $C_{19}H_{25}NO$: 283.1936.

HPLC on Water's model 660 ($C_{18}$ column, mobile phase 75%-acetonitrile - 10% phoaphate buffer, compound dissolved in MeOH) showed 99.4% of 9.24 min.

Anal. Calcd. for $C_{19}H_{25}NO \cdot Br$: C, 62.63; H, 7.19; Br, 21.94; N, 3.85.

Found: C, 62.27; H, 7.27; Br, 21.74; N, 3.88.

<u>Example 15</u> Cleavage of the methyl group from (-)-2-(di-n-propylamino)-5-methoxy-2,3-dihydro-1H-phenalene to form (-)-2-(di-n-propylamino)-5-hydroxy-2,3-dihydro-1H-phenalene.

A mixture of the titled 5-methoxy enantiomer free base (- isomer, 1.24 g, 4.17 mmole) and 14 ml of 48% ac HBr was stirred and heated at 125-130°C (oil bath T) for 20 min. The suspension was evaporated in vacuo and the resulting solid was crystallized from methanol-diethyl ether, 1.24 g (82% yield), m.p. 247-248°C. dec. $[\alpha]_D$ -12.02 (c 0.595, $CH_3OH$). UV, IR, proton NMD and CD were in accord.

Mass spec. Found 283.1933.

Calcd. for $C_{19}H_{25}NO$: 283.1936.

HPLC on Water's model 660 ($C_{18}$ column, mobile phase 75%-acetonitrile-10% phosphate buffer, compound dissolved in methanol showed 99.7% at 9.38 mμ.

Anal. Calcd. for $C_{19}H_{25}NO \cdot HBr$: C, 62.63; H, 7.19; Br, 21.94; N. 3.86.

Found: C, 62.64; H, 7.30; Br, 21.64; N, 3.98.

<u>Example 16</u> (+)2-(Dipropylamino)-2,3-dihydro-1H-phenalen-5-ol monobromide.

This example illustrates a Diels-Alder type process for preparing $R_3$-substituted-2,3-dihydro-1H-phenalen-2-amine compounds of this invention starting from a selected 2-hydroxybenzaldehyde.

A. Preparation of 3-bromo-2-hydroxy-5-methoxybenzaldehyde.

A solution of 2-hydroxy-5-methoxybenzaldehyde (41.08 g, 0.27 mol) and sodium acetate (24.61 g, 0.30 mol) in acetic acid (200 ml) was cooled in ice (crystallization occurs), and a solution of bromine (13.9 ml, 0.27 mol) in acetic acid (100 ml) was added over 5 min and stirred at room temperature for 15 min. A 5% w/v solution of stannous chloride in water (900 ml) prepared by dissolving 45 g of anhydrous stannous chloride ($SnCl_2$) in water and filtering through a filter aid (CELITE) was added. The yellow precipitate (ppt) was filtered, washed with water and cold ethanol, and dried in vacuo at room temperature to give 50.4 g (81%) of the above named benzaldehyde, m.p. 108-109°. A sample (0.48 g) was crystallized from ethanol to give bright yellow crystals (m.p. 109.5-110.5°C). The NMR, IR and UV spectra were consistent with this named compound.

Anal. Calcd. for $C_8H_7BrO_3$: C, 41.59; H, 3.05; Br, 34.59.

Found: C, 41.51; H, 3.01; Br, 34.38.

B. Preparation of 3-bromo-5-methoxy-2[[(4-methylphenyl)sulfonyl]-oxy]-benzaldehyde.

A solution of the 3-bromo-2-hydroxy-5-methoxybenzaldehyde (4.62 g, 0.02 mol) and p-toluenesulfonyl chloride (4.58 g, 0.024 mol) in tetrahydrofuran (150 ml) was cooled in an ice bath, and 15% sodium hydroxide (50 ml) was added dropwise. When the addition was complete, the mixture was stirred an additional two hr in an ice bath. The mixture was extracted twice with diethylether, and the combined ether extracts were washed with 15% NaOH and saturated NaCl solutions and dried ($MgSO_4$). The solvent was removed in vacuo to leave 7.71 g (100%) of the sub-titled tosyl group protected benzaldehyde as a solid. A sample (0.95 g) was crystallized from methanol to give colorless crystals (0.66 g; m.p. 85.5-86.5°C).

NMR ($CDCl_3$-TMS) $\delta$ 2.44 (s, 3, tosyl $CH_3$), 3.84 (s, 3, $OCH_3$), 7.2-7.45 (m, 4, aromatic H), 7.74, 7.85 (m, 2, tosyl 3, 5-H), 10.00 (s, 1, CHO). IR =CH 3084; C=O 1703, 1689; C=C 1592, 1568, 1492; $SO_3$ ester/-other 1375, 1357, 1200, 1181, 1172; =C-O/other 1260; C-O/$\beta$CH 1120, 1088, 1040; $SO_3$ ester/$\gamma$CH 853, 845, 820, 801, 740, 711, 675. UV (ethanol) End Abs., 224 nm (E 27,550), 260 Sl.Sh. (5,900), 290 (1,100), 323 (2,450). Mass spec. $m^+$ at m/z 384, 386.

Anal. Calcd. for $C_{15}H_{13}BrSO_5$: C, 46.77; H, 3.40; S, 8.32; Br, 20.74.

Found: C, 46.74; H, 3.43, S, 8.33; Br, 20.43.

C. Preparation of 2-(2-nitroethenyl)furan.

A mixture of 144 g of furfuraldehyde (1.5 mols), 183 g (3 moles) of nitromethane, 9.0 g (0.1 mol) of β-alanine and 150 ml of absolute ethanol was stirred at reflux for 3 hr at atmospheric pressure. Then the solvent and excess nitromethane were removed in vacuo. The residue compound was mixed with and stirred in diethylether and filtered. This diethylether treatment was repeated several times and the filtrate was evaporated to dryness and the residue was recrystallized from ethanol and washed with isopropanol to give 98 g of the above named product as a yellow-brown solid, 47% yield. This solid was again recrystallized from ethanol to give the sub-titled product as a brownish solid, m.p. 71.5-72.5°C.

Anal. Calcd. for $C_6H_5NO_3$: C, 51.803; H, 3.623; N, 10.069, O, 34.5

Found: C, 51.41; H, 3.64; N, 9.92.

D. Preparation of 2-(2-Nitroethenyl)furan.

A solution of 50 g of 2-(2-nitroethenyl)furan (0.36 mol) from part C, hereinabove, in 1000 ml of tetrahydrofuran was added to a mixture of 37.84 g (1.0 mol) of sodium borohydride in 860 ml of isopropanol while stirring over a 2 hr period. The resulting mixture was stirred for an additional 30 min at room temperature and then cooled in ice. A 10% v/v hydrochloric acid, 250 ml, solution was added slowly. When the acid solution addition was completed, the resulting mixture was diluted with water and diethyl ether. The pH of the aqueous phase of the resulting mixture was above 8. The aqueous phase solution was separated from the diethyl ether liquid phase and again extracted with diethyl ether. The aqueous and diethyl ether liquid phases were separated. The combined diethyl ether liquid phases were washed with water, saturated sodium chloride solution, and then dried over magnesium sulfate. The resulting dried solution was subjected to vacuum to remove solvent to leave as a residue the above named compound as a brown oil. An NMR spectral analysis confirmed the presence of the named compound. Distillation of the brown oil, and the taking of fractions thereof as follows gave the following weight amounts of named product.

| bp/mm (Hg) Pressure | Wt of Product |
|---|---|

| | | |
|---|---|---|
| 1. | (forerun) | 0.41 g |
| 2. | 55-57°C/0.4 | 16.73 g--colorless |
| 3. | 55-57°C/0.04 | 19.21 g--colorless |
| 4. | 56-58°C/1.4 | 4.86 g--colorless |

The total yield was 80 percent. The NMR spectra for samples 2, 3 and 4 were identical and consistent with the named intermediate compound.

E. Preparation of 2-bromo-4-methoxy-2-[3-(2-furanyl)-2-nitro-1-propenyl]-phenol, 4-methylbenzenesulfonate.

A mixture of the substituted benzaldehyde from part B hereinabove (3.20 g, 8.3 mmol), 2-(2-nitroethyl)furan from part D hereinabove (1.40 g, 9.9 mmol), piperidine (0.82 ml, 8.3 mmol) and acetic acid (0.48 ml. 8.3 mmol) in SKELLYSOLVE B hexanes (40 ml) was stirred at reflux for 2 hr. The reaction mixture was diluted to 300 ml with methylene chloride, silica gel (50 g, 70-230 mesh) was added, and the mixture was stirred for 2.5 hr. Ethereal HCl (about 5 mmol of HCl) was added and the mixture was stirred for 30 min. The mixture was filtered, and the silica gel was washed several times with methylene chloride. The combined organic liquid phases were evaporated to dryness to give 3.72 g of a yellow oil. Crystallization from methanol gave a yellow solid (2.74 g, 65%; m.p. 116°C). The NMR, IR and UV spectra were consistent with the titled compound.

Anal. Calcd. for $C_{21}H_{18}BrNO_7S$: C, 49.62; H, 3.57; N, 2.76; S, 6.31; Br, 15.72.

Found: C, 49.24; H, 3.66; N, 2.73; S, 6.30; Br, 15.56.

F. Preparation of 2-[2-amino-3-(2-furanyl)propyl]-6-bromo-4-methoxyphenol, 4-methylbenzenesulfonate (ester), (E)-2-butenedioate (1:1).

A mixture of lithium aluminum hydride (4.10 g, 0.108 mmol) in tetrahydrofuran (THF) (100 ml) was cooled in ice, and 100% sulfuric acid (5.30 g, 0.054 mmol) was added dropwise with stirring over a period of 10 min. A solution of the nitro compound from step E hereinabove (5.49 g, 0.0108 mmol) in THF (70 ml) was added at 0°C over a 15 min period. The reaction was stirred for an additional 10 min and water (4.1 ml), 15% NaOH (4.1 ml), and water (12.3 ml) were consecutively added at 0°C. The suspension was filtered, and the aluminum containing filter cake was washed well with diethylether and THF.

The filtrate was dried ($MgSO_4$), and the solvent was removed in vacuo to leave a yellow oil (5.22 g, 100%). A sample (0.50 g) was mixed with fumaric acid (0.13 g) and crystallized from methanol/diethyl ether to give the sub-titled compound as a colorless solid, 0.53 g; m.p. 185°C. The NMR, IR and UV and mass spectra were consistent with the titled compound.

Anal. Calcd. for $C_{21}H_{22}BrNO_5S \cdot C_4H_4O_4$: C, 50.34; H, 4.39; N, 2.35; S, 5.38; Br, 13.40.

Found: C, 50.08; H, 4.43; N, 2.21; S, 5.18; Br, 13.07.

G. Resolution of 2-[2-amino-3-(2-furanyl)propyl]-6-bromo-4-methoxyphenol, 4-methylbenzenesulfonate (ester).

A 44.68 g (0.0930 mmol) portion of the (±) racemic mixture compound, 2-[2-amino-3-(2-furanyl)propyl]-6-bromo-4-methoxyphenol, 4-methylbenzenesulfonate ester, (prepared on a larger scale, in general as described in part F hereinabove) in 500 ml of hot methanol solution was combined with a solution of (+)-Di-p-toluoyl-D-tartaric acid monohydrate, 37.61 g (0.930 mmol) in 500 ml of hot methanol. The mixture was stirred and allowed to stand at room temperature for 24 hr, then at -10°C for 4 hr) to effect crystallization of the resulting amine salt. After crystallization was complete, the crystals were collected by filtration to give 24.6 g of the tartrate salt. Recrystallization twice more from methanol gave 11.97 g of salt. The optical rotation of the free base was $[\alpha]_D^{25}$ equals +10.3°.

The filtrate from the first crystallization was evaporated to dryness and the residue was partitioned between diethyl ether and 15% w/v sodium hydroxide aqueous solution. The aqueous phase was separated and extracted with diethyl ether. The combined diethyl ether organic liquid phases were washed with 15% w/v sodium hydroxide aqueous solution and saturated sodium chloride solution and then dried with magnesium sulfate. The solvent was removed in vacuo to leave the resolved amine as a yellow brown oil, 34.28 g. This crude oily compound residue was dissolved in hot methanol, 250 ml, and this methanolic solution of the compound was added to a solution of (-)-Di-p-toluoyl-L-tartaric acid, 28.86 g in hot methanol, 400 ml. The mixture was stirred and allowed to stand and cool at room temperature for 24 hr, then at -10°C for 4 hr to crystallize the resulting tartaric salt. After crystallization was complete, the crystals were

collected by filtration to give 22.2 g of the salt. Recrystallization of this salt twice more from methanol gave the tartarate salt as a colorless solid, 12.92 g. Optical rotation of the above-named free base was $[\alpha]_D^{25}$ -9.92°.

H. Preparation of (+) 2-[2-(2,5-dimethyl-1-pyrrol-1-yl)-3-(2-furanyl)propyl]-6-bromo-4-methoxyphenol, 4-methylbenzenesulfonate (ester).

The resolved (+) isomer from Step G hereinabove, 11.76 g was released from its di-p-toluyl-tartaric salt form to give its free base by shaking the tartaric acid salt with a 10% w/v sodium hydroxide aqueous solution, diethyl ether mixture. The ether solution was washed with 10% w/v sodium hydroxide solution and with saturated sodium chloride solution and then dried with magnesium sulfate. The solvent was then removed in vacuo to give 6.75 g of the free base, (+) isomer as a colorless oil.

This free base (+) isomer, oil was combined with 3.30 ml (0.0281 mmol) of acetonylacetone (2 equivalents) and 0.26 ml (3.5 mmol) of propionic acid, and 150 ml of benzene.

This mixture was refluxed through a Dean-Stark trap for 23 hr. Then 0.45 ml of water by-product was collected. The resulting mixture was diluted with diethyl ether and washed with 5% w/v sodium hydroxide solution and with saturated sodium chloride solution and dried ($MgSO_4$). The solvent was removed in vacuo to leave the sub-titled product crude compound as a yellow oil, 8.94 g. Purification by flash chromatography through 4 x 17 cm columns, using 12.5% diethyl ether in hexane mixture as eluting liquid gave the sub-titled compound, 6.80 g (88 percent yield) as a colorless oil. The NMR spectrum was consistent with the sub-titled intermediate compound. The specific rotation $[\alpha]_D^{25}$ for this compound was +58.46.

I. Preparation of the 2,3-dihydro-2-(2,5-dimethylpyrrol-1-yl)-5-methoxy-7,9a-epoxy-3,9-ene-1H-phenalene.

A 6.22 g (0.0114 mmol) portion of the (+) 2-[2-(2,5-dimethyl-1-pyrrol-1-yl)-3-(2-furanyl)propyl]-6-bromo-4-methoxyphenol, 4-methylbenzenesulfonate ester, from step H hereinabove, in purified tetrahydrofuran and maintained under an atmosphere of argon gas, was cooled to 10°C while 700 ml (0.0118 mmol) of 1.68 N phenyllithium was added dropwise over about 12 min. The mixture was stirred at 10°C for about 3.5 hr. Then 300 ml of diethylether was added and the mixture

was washed with water. The resulting aqueous solution phase was separated and back extracted with diethyl ether. The combined diethyl ether organic liquid phases were washed with saturated sodium chloride solution, separated therefrom and dried ($MgSO_4$). The solvent was removed in vacuo to leave as residue the sub-titled compound as a yellow-brown oil, 5.28 g. The presence of the sub-titled compound was confirmed by an NMR spectrum analysis of a sample thereof.

J. Preparation of 2,3-Dihydro-2-(2,5-dimethylpyrrol-1-yl)-5-methoxy-7,9a-epoxy-1H-phenalene.

The 5.28 g (0.0114 mmol) of the epoxy-ring unsaturated compound 2,3-dihydro-2-(2,5-dimethylpyrrol-1-yl)-5-methoxy-7,9a-epoxy-8,9-ene-1H-phenalene, from step I hereinabove, and 0.60 g of 10% w/v palladium-on-carbon catalyst and 150 ml of reagent grade tetrahydrofuran was treated with hydrogen (hydrogenated) at room temperature for 1.75 hr. The mixture was filtered through a filter aid (CELITE) to collect the hydrogenation catalyst. The catalyst/filter aid solid was washed well with diethyl ether. The combined filtrates were evaporated to dryness to give the sub-titled epoxy ring saturated compound as a brown oil. An NMR spectrum analysis of a sample of the oil confirms the presence of the sub-titled compound. This oil intermediate product was purified by flash chromatography using silica gel, 0.40 to 0.0063 mm particle size beads in four 16.5 cm columns using 15% v/v ethyl acetate in hexane as eluting liquid. This procedure gave two fractions or cuts: the first contained 2.60 g (74%) yield), and the second contained 90 mg of the desired sub-titled product, plus some impurities. A third oil contained 0.59 g of the sub-titled compound, obtained by flushing the column with 1:1 v/v hexane:ethyl acetate mixture.

The specific light rotation $[\alpha]_D^{25}$ for the most pure fraction of this compound was +9.90.

K. Preparation of 2-(2,5-dimethylpyrrol-1-yl)-2,3-dihydro-5-methoxy-1H-phenalene.

A 2.60 g (8.40 mmol) portion of the epoxy-ring saturated compound, from step J, hereinabove, in 50 ml of methylene chloride was treated dropwise, while stirring, with 1.1 ml (8.9 mmol) of boron trifluoride etherate over 3 hr at room temperature. The mixture was then stirred for 10 min and 150 ml of diethyl ether, followed by 100

ml of 10% w/v sodium hydroxide solution were added. The resulting mixture was shaken vigorously until an oily precipitate was dissolved. The liquid layers were then separated. The aqueous phase was extracted with diethyl ether. The combined organic liquid phases were dried over magnesium sulfate. The solvent was removed in vacuo to leave as residue 2.35 g of the crude sub-titled compound as a yellow-brown oil. This crude product oil was flash chromatographed over silica gel beads using 5% v/v ethyl acetate in hexane as eluting liquid in a 3.5 by 15 cm column. This procedure gave a poor separation, perhaps because the oil was added to the column in methylene chloride. In any event, 1.85 g of a colorless foamy product was obtained. An NMR spectrum of a sample thereof confirmed the presence of the sub-titled compound.

L. Preparation of 2-amino-5-methoxy-2,3-dihydro-1H-phenalene.

A mixture of 1.79 g (6.14 mmol) of 2-(2,5-dimethylpyrrol-1-yl)-2,3-dihydro-5-methoxy-1H-phenalene, prepared as described in step K hereinabove, 2.57 g (0.037 mmol) (6 equivalents) of hydroxylamine hydrochloride, 2.10 g (0.025 mmol) of sodium bicarbonate (4 equivalents) in 70 ml of absolute ethanol was stirred at reflux temperatures in a heating mantle overnight (17.5 hr). Then an additional 1.29 g of hydroxylamine hydrochloride (3 equivalents) and 1.05 g (2 equivalents) of sodium bicarbonate were added and the refluxing action was continued. After 41.5 hr, an additional 1.29 g of hydroxylamine hydrochloride and 1.05 g of sodium bicarbonate were added and the reflux was continued for seven more hours. A total of 47.5 hr of refluxing the mixture was imposed on the reaction mixture.

The solvent was removed in vacuo and the residue was partitioned between 5% w/v sodium hydroxide solution and diethyl ether. The aqueous phase was extracted with diethyl ether. The combined organic liquid phases were washed with water and extracted with 25 ml of 10% v/v aqueous hydrochloric acid solution.

A precipitate was formed which solubilized by added water, about 50 ml. The aqueous and organic liquid layers were separated and the diethyl ether layer was extracted again with 25 ml of 10% hydrochloric acid solution. The acid extracts were washed with diethyl ether and basified with 40% w/v sodium hydroxide solution while cooling in ice. The resulting milky mixture was extracted three times with diethyl ether. The resulting ether extracts were combined and washed with saturated sodium chloride solution and dried with magnesium sulfate. The solvent was removed in vacuo to leave the sub-titled compound as a colorless oil which solidified upon standing, and weighed 1.03 g (79 percent yield). An NMR spectrum of a sample thereof was consistent for this sub-titled compound.

M. Preparation of 2-(N,N-di-n-propylamino)-5-methoxy-2,3-dihydro-1H-phenalene and its hydrochloride.

A mixture of 0.25 g (1.17 mmol) of 2-amino-5-methoxy-2,3-dihydro-1H-phenalene, prepared as described in part L hereinabove, and 1.28 ml of 1-bromopropane (12 equivalents), and 0.49 g (3.51 mmol) of potassium carbonate in 25 ml of acetonitrile was stirred at reflux while the progress of the amino-nitrogen alkylation reaction was followed by vapor phase chromatography (VPC) analyses of samples of the reaction mixture. Additional 0.64 ml of 1-bromopropane, 0.49 g of potassium carbonate and 10 ml portions of acetonitrile were added after 22 hr of refluxing. Additional quantities of these 3 reagents were added during the total of 53 hr of refluxing, to ensure complete reaction. The solvent was removed in vacuo and the residue was partitioned between water and diethyl ether liquid phases. The aqueous solution phase was separated and extracted again with diethyl ether. The combined diethyl ether organic liquid phases was washed with saturated sodium chloride and dried over magnesium sulfate. The solvent was removed in vacuo to leave as residue the sub-titled 5-methoxy-phenalene-2-amine as a yellow oil 0.36 g.

The crude product oil was dissolved in diethyl ether and the solution was acidified with hydrogen chloride in diethyl ether solution. The resulting precipitate was collected, washed with diethyl ether and crystallized from a mixture of methanol/diethyl ether to give 0.33 g (84% yield) of the sub-titled amine hydrochloride as off-white crystals. The specific rotation, $[\alpha]_D^{25}$ was -13.46°.

N. (-) 2-(Di-n-propylamino)-5-Hydroxy-2,3-dihydro-1H-phenalene, and its hydrobromide salt.

A 24.5 g (0.082 mole) portion of 2-(N,N-di-n-propylamino)-5-methoxy-2,3-dihydro-1H-phenalene and 200 ml of 48% v/v hydrobromic acid was heated in an oil bath at 125-130°C for 20 min, and then the mixture was evaporated to dryness on a rotary evaporating device at

55°C, leaving a very dark solid as residue. This residue was taken up in a mixture of 15% v/v ammonium hydroxide solution and chloroform, the liquid phases were separated, the aqueous phase was extracted with fresh chloroform. The chloroform liquid phases were combined, washed with water, saturated sodium chloride solution and dried over magnesium sulfate. The dried solution was evaporated to remove solvent leaving as residue 23.6 g of the titled compound.

This crude 23.6 g of 2-(di-n-propylamino)-5-hydroxy-2,3-dihydro-1-phenalene purified by chromatography through a silica gel column containing 2.3 kg of 70-230 mesh silica gel beads, using a 2.5% v/v methanol in chloroform solution which also contained 0.25% v/v ammonium hydroxide solution as eluting liquid. The pertinent liquid fractions were combined to obtain the titled -phenalene-5-ol in two crops, (1) 18 g and (2) 2.7 g (taken the following day after the filtrate stood overnight. High performance liquid chromatography (HPLC) analysis of the larger crop showed that it was 98.8% pure; the second crop was shown to be 96.8% pure.

The larger, first crop was converted to its hydrobromide salt by dissolving the 2-(di-n-propylamino)-5-hydroxy-2,3-dihydro-1H-phenalene in diethyl ether, and adding 30% hydrogen bromide in acetic acid until precipitation is complete. The precipitate was filtered from the liquid mixture and recrystallized from a methanol/diethyl ether mixture to obtain the hydrobromide salt as gray needles, m.p. 235-236°C (dec.), 16 g.

Example 17 (+) 2-(Di-n-propylamino)-5-Hydroxy-2,3-dihydro-1H-phenalene.

Following the procedure of Example 16 and using the (-) resolved isomer of 2-[2-amino-3-(2-furanyl)propyl]-6-bromo-4-methoxyphenol, 4-methylbenzenesulfonate (ester) from Example 16, part G, the optical rotation of which is -9.92°, and following generally the reaction steps set forth in parts H to N of Example 16 hereinabove, there is obtained the (+)-2-(di-n-propylamino)-5-hydroxy-2,3-dihydro-1H-phenalene and its hydrobromide salt.

6-SUBSTITUENT SERIES

Example 18 2-Amino-6-bromo-2,3-dihydro-1H-phenalene, and its hydrochloride.

A. Preparation of diethyl-6-bromo-1H-phenalene-2,2(3H)-dicarboxylate.

The diester, diethyl 1H-phenalene-2,2(3H)dicarboxylate, 1.56 g, 5.0 mmol) was dissolved in chloroform (20 ml) and ferric chloride (0.27 g, 1.7 mmol) was added. A solution of bromine (0.86 g, 5.4 mmol) in chloroform (10 ml) was added, and the mixture was stirred at room temperature for 2.5 hr. The reaction mixture was washed with water, saturated sodium bicarbonate, saturated sodium chloride and dried (magnesium sulfate). The solvent was removed in vacuo to leave 2.1 g of the diethyl 6-bromo-1H-phelanene-2,2-(3H)dicarboxylate as a light brown oil. Crystallization from methanol gave a colorless solid (1.48 g, 76%) m.p. 71.5-72°C.). NMR ($CDCl_3$ - TMS) $\delta$ 1.12 (t, J = 7Hz, 6, $CH_3$), 3.57 (S, 2, $CH_2$), 3.62 (S, 2, $CH_2$), 4.10 (q, J = 7Hs, 4, $OCH_2$), 7.0-7.75 (M, 4, aromatic H), 8,07 (dd, J = 1.6 and 8 HZ, 1, C-7 hydrogen); IR = CH 3072, 3056, C = O 1747, 1721, C = C 1611, 1596, 1571, 1501, CO/other 1255, 1226, 1193, 1079, 1058, γCH/other 845, 838, 761; UV (ethanol) 214 sh. (25,400), 231 (59,100), 274 sl. sh. (4,650), 284 (8,100), 295 (10,400), 306, (7,400), 311 (5,650), 320 sh. (1350), 326 (1,150); mass spec. $M^+$ at m/z 390,392.

Anal. Calcd. for $C_{19}H_{19}BrO_4$: C, 58.32; H, 4.89; Br, 20.42.
Found: C, 58.04; H, 4.99; Br, 20.61

B. Preparation of 6-Bromo-1H-phenalene-2,2(3H)-dicarboxylic acid.

The 6-bromo diester from part A hereinabove (185.3 g, 0.47 mol) was dissolved in methanol (1,300 ml) and added to a solution of potassium hydroxide (263.2 g, 4.7 mol) in water (1,300 ml), and the mixture was stirred at reflux for two hr. The methanol was removed in vacuo, and the aqueous mixture was diluted with 500 mls of water and extracted twice with ether. The aqueous solution was acidified with concentrated HCl, and the resulting white precipitate was filtered, washed with water, and dried in vacuo at 50°C. to give 157 g (100%) of the subtitled diacid. NMR (DMSO-$d^6$, TMS) $\delta$3.46 (S, 2, $CH_2$), 3.50 (S, 2, $CH_2$), 7.15-8.05 (M, 5, aromatic hydrogens).

C. Preparation of 6-Bromo-2,3-dihydro-1H-phenalene-2-carboxylic acid.

The diacid from part B hereinabove (50.0 g, 0.149 mol) was covered with a nitrogen atmosphere and heated in an oil bath maintained at 235-240°C. for 25 min during which time the evoluation of

$CO_2$ ceased. The melt solidified upon cooling. The mass was ground to a fine powder via mortar and pestle to give the subtitled 6-bromo mono-carboxylic acid (43.37 g, 95%); m.p. 213-215°C.). The NMR was identical to that of the compound prepared by bromination of 2,3-dihydro-1H-phenalene-2carboxylic acid.

D. Preparation of 1,1-dimethylethyl(6-bromo-2,3-dihydro-1H-phenalen-2-yl)carbamate.

The reaction to convert the 6-bromo-2,3-dihydro-1H-phenalen-2-carboxylic acid from part C hereinabove, to the above subtitled 6-bromocarbamate, was run according to the procedure set forth herein for preparing (2,3-dihydro-1H-phenalen-2-yl)carbamic acid, 1,1-dimethylpropyl ester, (5.00 g, 0.017 mol), diphenylphosphoryl azide (3.7 ml, 0.017 mol), and triethylamine (2.6 ml, 0.019 mol) in t-butanol (60 ml). A (4.74 g) sample of the crude subtitled carbamate ester (8.92 g) was purified by low pressure column chromatography ($SiO_2$, 0.040-0.063 mm; 10:1 cyclohexane/ethyl acetate) to give 1.72 g of a yellow solid. Crystallization from cyclohexane/SKELLYSOLVE B hexanes gave yellow crystals of the subtitled carbamate ester; m.p. 181.5-183°C). NMR ($CHCl_3$, TMS) $\delta$1.42 (s,9,$C(CH_3)_3$, 2.8-3.5 (M, 4, N-C-$(CH_2)_2$), 4.0-4.65 (M, 2, NHCH), 7.0-8.15 (M, 5, aromatic H); IR NH 3333, C = O 1681, C = C 1595, 1571, 1500, Amide II 1524, C-O/C-N/other 1305, 1236, 1173, $\gamma$CH/other 816, 760; UV (ethanol) 214 sh. (24,450), 231 (69,200), 275 sh. (4,850), 285 (8,250), 296 (10,350), 307 (7,300), 312 (6,000), 321 (1,400), 326 (1,350); mass spec. $M^+$ at m/z 361,363.

Anal. Calcd. for $C_{18}H_{20}BrNO_2$: C, 59,68; H, 5.56; N, 3.87; Br, 22.06.

Found: C, 59.68; H, 5.73; N, 3.82; Br, 21.94.

The remainder of the crude product material (4.18 g) was boiled with 150 ml of cyclohexane and the supernatant decanted. This was repeated with 2 x 100 ml of cyclohexane. The combined supernatant was evaporated to dryness and crystallized from methanol to give an additional 1.46 g of the subtitled carbamate ester; (m.p. 178-179.5°C). The total yield was 3.18 g (51%).

E. Preparation of 6-Bromo-2,3-dihydro-1H-phenalen-2-amine.

The 1,1-dimethylethyl 6-bromo-2,3-dihydro-1H-phenalen-2-ylcarbamate ester from part D hereinabove (9.5 g, 0.026 mol) and trifluoroacetic acid (20 ml) were reacted as described in other examples herein to remove the carboylate ester group and to form the 6-bromo-2,3-dihydro-1H-phenalen-2-amine as a deep yellow oil (6.0 g, 87%). A 0.5 g sample of the named amine product was converted to the HCl salt by stirring in 3 ml. of 10% aqueous HCl, filtering and drying in vacuo at 50°C. The yield was 0.53 g (93%) (m.p. > 320°C. NMR of free base ($CDCl_3$ - TMS) δ1.95 (S, 2, exch, $NH_2$), 2.65-3.65 (M, 5, N-$CH(CH_2)_2$), 6.9-7.75 (M, 4, aromatic H), 8.00, 8.10 (d, 1, $C_7$ H); UV (ethanol) $-CH/NH_3^+$ 3119,3057, 3037, $NH_3^+$ 2794, 2698, 2618, 2533, 2491, 2025, $NH_3^+/C{-}C$ 1620, 1595, 1574, 1530, 1519, 1507, γCH819/755; UC (ethanol) 214 sh. (23,800), 231 (68,200), 274 sh. (4,550), 284 (7,700), 294 (9,800), 306 (6,900), 311 (5,000), 320 sh. (1,200), 326 (1,150); mass spec. $M^+$ for free base at m/z 261, 263.

Anal. Calcd. for $C_{13}H_{12}BrN{\cdot}HCl$: C, 52.29; H, 4.39; N, 4.69; Cl, 11.87; Br, 26.76.

Found: C, 52.06; H, 4.37; N, 4.68; Cl, 11.62; Br, 26.85.

Example 19 2-Dimethylamino-6-bromo-2,3-dihydro-1H-phenalene, and its hydrochloride.

A solution of 2-amino-6-bromo-2,3-dihydro-1H-phenalene (1.93 g; 7.36 mmole) released from its hydrochloride in ethyl acetate with 15% sodium hydroxide in water solution), formic acid (3.57 g; 0.073 mole), and 37% formalin solution (3.59 g; 0.044 mole) was refluxed for 30 min, cooled in ice and then 20% sodium hydroxide solution was added. The resulting reaction mixture was extracted well with diethyl ether. The ether extract was washed with water and with saturated sodium chloride solution, and then separated from the aqueous wash liquids and dried with magnesium sulfate. The dried organic liquid mixture was evaporated to give 2 g of the titled amine as a brown oil. This oil amine could be crystallized from petroleum ether (b.p. 30-60°C.) at -70°C. The amine m.p. was 47-48°C. UV sh 214 (26.850), λmax 230 (61,750), 286 (7,950), 296 (9,950), 307 (7,200), 312 (6,000), sh 321 (1,750), 326 (1,500). IR −CH 3063, 3042, 3034; NCH 2781; C=C 1609, 1594, 1501, 1497; C-N 1034; aromatic 812,752. NMR ($CDCl_3$) δ2.42 (s, 6, $NMe_2$), 2.5-3.35 (m, 5, $CH_2$-CH-$CH_2$), 7.02-8.10 (m, 5, aromatic).

HR mass spec. Calcd.: 289.0467.

Found: 289.0478.

Calcd. for $C_{15}H_{16}BrN$: C, 62.07; H, 5.56; Br, 27.54; N, 4.83.
Found: C, 62.02; H, 6.08; Br, 27.17; N, 4.72.

The titled hydrochloride was prepared in diethyl ether with ethereal HCl, m.p. 277-278°C dec., unchanged on recrystallization from methanol-ether. UV sh 213 nm (28,550), λmax 229(62,350), sh 274 (4,550), 284 (7,700), 294 (9,900), 305.5 (7,050), 311 (5,000), sh 320 (1,250), 326 (1,050). IR =CH 3063, 3034, 3020, 3009; $NH^+$ 2643, 2571, 2524, 2478; C=C 1612, 1598, 1572, 1505, 1493; other 966; aromatic 815, 758. NMR in $CDCl_3$ + $CD_3OD$: δ 2.94 (s, 6, $NMe_2$), 3.0-3.75 (m, 5, N-$CH(CH_2)_2$), 7.1-8.2 (m, 5, aromatic). Mass spectrum $M^+$289, 291.

Anal. Calcd. for $C_{15}H_{16}BrN\cdot HCl$: C, 55.15; H, 5.25; Cl, 10.85; Br, 24.46; N, 4.29.

Found: C, 55.32; H, 5.63; Cl, 10.57; Br, 24.38; N, 4.20.

Example 20 1-(6-Bromo-2,3-dihydro-1H-phenalen-2-yl)azetidine.

A mixture of 2-amino-6-bromo-2,3-dihydro-1H-phenalene (3,67 g; 14 mmole), 1,3-dibromopropane (3,39 g; 16.8 mmole), potassium carbonate (4.3 g; 31 mmole) and 120 ml of acetonitrile was refluxed for 21 hr. The reaction mixture was then evaporated and the residue was taken up in a chloroform/water mixture. The aqueous layer was extracted with chloroform. The combined chloroform layers were washed with water and saturated sodium chloride aqueous solution, and dried with magnesium sulfate. The dried organic liquid phase was evaporated. A Beilstein test of the residue for halogen was positive. The above oil residue was heated with 15% sodium hydroxide w/v in water solution at 95°C for two hr and worked up as above.

The resulting crude oil product (3.4 g) was subjected to low performance liquid chromatography (LPLC) using a 300 ml. column packed with 230-400 mesh size silica gel particles and using 3% v/v methanol in chloroform mixture, also containing 0.5% v/v ammonium hydroxide solution as eluting liquid. Twenty ml. fractions were collected. Fractions 1 to 19 gave no pertinent material. Fractions 20 to 31 gave 1.18 g of the titled -azetidine derivative compound as a brown oil. This oily product was dissolved in diethyl ether, decanted away from some amorphous material, filtered through a filter aid (CELITE) to clarify the solution, and then concentrated to a small volume, diluted with petroleum ether (b.p. 30-60°C), and

 

allowed to crystallize. There was obtained 0.67 g of the titled-azetidine compound, m.p. 102-103°C.

Second crop 0.21 g of the same m.p. UV sh 214 nm (24,650), sh 225 (50,850), λmax 229 (64,750), sh 274 (4,550), 284 (7,850), 295 (9,900) 306 (7,100), 312 (5,600), sh 320 (1,450), 326 (1,350). IR =CH 3063, 3041, 3007; N-CH 2784; C=C 1611, 1595, 1572, 1501, 1482; C-N 1192, 1030; aromatic 825, 810, 801, 766, 760. NMR ($CDCl_3$) δ 2.10 (quint., 2, C-$CH_2$-C, J=7.1 Hz), 2.75-3.5 (m, 5, N-CH($CH_2$)$_2$), 3.33 (t, 4, $CH_2$-N-$CH_2$, J=6.9 Hz).

HR mass spec. Calcd. 301.0467.

Found: 301.0478.

Anal. Calcd. for $C_{16}H_{16}BrN$: C, 63.58; H, 5.34; Br, 26.44; N, 4.64.

Found: C, 63.15; H, 5.37; Br, 26.01; N, 4.52.

Example 21 1-(6-Bromo-2,3-dihydro-1H-phenalene-2-yl)pyrrolidine.

A mixture of 2-amino-6-bromo-2,3-dihydro-1H-phenalene (0.88 g, 3.35 mmole), 1.4-diiodobutane (1.04 g; 3.35 mmole), potassium carbonate (1.38 g, 0.01 mole) and 20 ml of acetonitrile was refluxed for 22 hr, and then evaporated. The residue was taken up on a chloroform/-water mixture and filtered to remove a small amount of solid. The chloroform liquid layer was separated from the aqueous phase and washed with water, with saturated sodium chloride aqueous solution and then dried with magnesium sulfate, and evaporated to give 0.86 g of the crude titled -pyrrolidine derivative compound as a brown oil.

This brown oil was dissolved in 25 ml of petroleum ether (b.p. 30-60°C) filtered, and the solution filtrate was concentrated to a 5 ml residue and cooled to -18°C to give 0.65 g of the purified titled -pyrrolidine compound as pale yellow prism crystals, m.p. 87-88°C. UV sh 214 nm (25,600), λ max 229 (66,500), 285 (8,050), 295 (10,150), 306 (7,300), 311 (5,900), sh 320 (1,500), 326 (1,400). IR=CH 3066, 3022; N-CH 2806, 2781; C=C 1611, 1595, 1501; C-N 1148, 1067; aromatic 822, 761. NMR ($CDCl_3$) δ 1.85 (center of multiplet, 4, C-$CH_2$-$CH_2$-C), 2.75 (center of a multiplet, 4, $CH_2$-N-$CH_2$) 2.5-3.5 (m, 5, N-CH($CH_2$)$_2$) 7.0-8.15 (m, 5, aromatic). Mass spec. $M^+$315, 317.

Anal. Calcd. for $C_{17}H_{18}BrN$: C, 64.56; N, 5.74; Br, 25.27; N, 4.43.

Found: C, 64.36; H, 5.77; BR, 25.01, N, 4.42.

Example 22 1-(6-Bromo-2,3-dihydro-1H-phenalen-2-yl)piperadine.

A mixture of the 2-amino-6-bromo-2,3-dihydro-1H-phenalene (0.88 g; 3.35 mmole), potassium carbonate (1.38 g; 10 mmole) and 20 ml of acetonitrile was refluxed for 20 hr and then evaporated. The residue was taken up in a chloroform/water mixture and the organic liquid layer was separated and washed with water, saturated sodium chloride salt solution, and then dried with magnesium sulfate. The dried organic liquid layer was evaporated to leave the titled compound as a crude oil residue. This crude oil product was dissolved in boiling petroleum ether (b.p. 30-60°C), filtered and concentrated to a 5 ml residue and cooled at -18°C to give 0.6 g of the titled -piperadine compound as yellow prism crystals, m.p. 65-66°C.

UV sh 214 (26,200), λmax 229 (57,950), sh 276 (4,850), sh 287 (8,150), 297 (10,350), 307 (7,600), 312 (6,600), sh 321 (2,200), 326 (1,750). IR =CH 3082, 3064, 3035; N-CH 2809, 2763, 2738; C=C 1609, 1592, 1498; C-N 1206, 1122, 1109; aromatic 838, 820, 794, 759. NMR ($CDCl_3$) δ 1.57 (center of multiplet, 6, C-$CH_2CH_2CH_2$-C), 2.68 (center of multiplet, 4, $CH_2$-N-$CH_2$), 2.80-3.50 (m, 5, N-CH($CH_2$)$_2$), 7.01-8.10 (m, 5, aromatic). Mass spec. $M^+$329, 331.N.

Anal. Calcd. for $C_{18}H_{20}BrN$: C, 65.46; H, 6.10; Br, 24.20; N, 4.24.

Found: C, 65.12; H, 6.13; Br, 23.87; N, 4.27.

Example 23 2,3-Dihydro-N-methyl-6-(2-methyl-1,3-dioxan-2-yl)-1H-phenalen-2-amine- and its Z-2-butendioate (1:1) salt.

A. Preparation of 6-Acetyl-2,3-dihydro-1H-phenalen-2-yl carbamic acid ethyl ester.

Aluminum trichloride (17.7 g; 0.173 mole) was added over 10 min to a solution of 2-(ethoxycarbonylamino)-2,3-dihydro-1H-phenalene (9.55 g; 0.037 mole) in 190 ml of 1,2-dichloroethane and acetyl chloride (3.61 g; 0.046 mole) keeping the temperature at room temperature. The mixture was stirred for one hr at room temperature. Ice was added, followed by 300 ml of 10% hydrochloric acid solution and diethyl ether, and the mixture was stirred for one hr. The organic liquid layer was separated. The aqueous layer was extracted with diethyl ether using three 100 ml portions of diethylether. The combined ether extract was washed with saturated sodium bicarbonate

solution (2 x 100 ml), with saturated salt solution, dried with magnesium sulfate and evaporated. The residual solid was crystallized from ethanol to give 7.32 g of the subtitled compound, m.p. 146-147°C, which was unchanged on recrystallization.

Second crop, 1.06 g, m.p. 143-145°C, yield 76%. UV λmax 214 nm (ε 31,000), sh 221 (27,950), 243 (20,900), 317 (8,100), sh 327 (7,600). IR NH/=CH 3363, 3074, 3041; other 2165; 2136; C=O (carbamate) 1706; C=O (ketone) 1673; C=C 1595, 1580; amide II 1523; C-O/C-N 1267, 1224, 1220; aromatic 844, 766. NMR ($CDCl_3$) δ 1.20 (t, 3, O-C-$CH_3$, J~ 8 Hz), 2.70 (s, 3, $COCH_3$), 2.9-3.6 (m, 4, N-CH($CH_2$)$_2$, 4.10 (quads, 2, $OCH_2$, J~ 8 Hz), 4.40 (center of m, 1, N-CH), 4.75 (center of broad, d, 1, NH), 7.2-8.7 (m, 5, aromatic). Mass spec. $M^+$297.

Anal. Calcd. for $C_{18}H_{19}NO_3$: C, 72.70; H, 6.44; N, 4.71.

Found: C, 72.49; H, 6.43; N, 4.83.

B. Preparation of 2,3-dihydro-6-(2-methyl-1,3-dioxan-2-yl)-1H-phenalen-2-yl carbamic acid ethyl ether.

A mixture of 6.3 g (0.021 mole) of the 6-acetyl-2-(ethoxycarbonylamino)-2,3-dihydro-1H-phenalene, 5.26 g (0.085 mole) of ethylene glycol, 50 mg of p-toluenesulfonic acid and 70 ml of benzene was refluxed for 6 hr using a water separator. Benzene was evaporated and the residue was taken up in a diethyl ether/water mixture. The ether liquid layer was separated and washed twice with water with saturated sodium bicarbonate solution, with saturated sodium chloride solution, dried with magnesium sulfate and evaporated. The residue was extracted with three 100 ml portions of boiling 20% v/v diethyl ether in petroleum ether (b.p. 30-60°C), the extract was concentrated to about 50 ml, the residue was cooled in ice, diethyl ether was added to clarify the liquid and the residue was allowed to crystallize to give 2.51 g of the crude subtitled compound, m.p. 89.5-91°C. The filtrate was evaporated and the residue, 5 g, was chromatographed on 500 g of silica gel using 10% v/v ethyl acetate in cyclohexane mixture as eluting liquid, collecting 30 ml fractions. Fractions 91 to 123 gave 2 g of additional, subtitled product, which was crystallized from a diethyl ether/petroleum ether (b.p. 30-60°C) mixture at -70°C to give an additional 1.7 g of the subtitled compound, m.p. 90-91°C.

UV λ max 211 nm (ε 27,750), 224 (54,700), 231 (89,550), sh (4,600), 281 (7,800), 291 (9,700), 302 (6,750), sh 307 (4,300), sh 317 (956), 322 (714). IR NH/=CH 3110, 3069; C=O 1687; C=C 1611, 1600, 1487; amide II 1534; C-O/C-N 1303, 1287, 1233, 1198, 1190, 1043, 1037; aromatic 836, 813, 767. NMR ($CDCl_3$) δ 1.2 (t, 3, carbamate $CH_3$, J= ~8 Hz), 1.90 (s, 3, ketal $CH_3$), 2.90-3.50 (m, 4, N-$CH(CH_2)_2$), 3.70-4.20 (m, 6, ketal $CH_2$, carbamate $CH_2$), 4.35 (center of m, 1, NCH), 4.70 (center of broad d, 1, NH), 7.10-8.55 (m, 5, aromatic). Mass spec. $M^+$341.

Anal. Calcd. for $C_{20}H_{23}NO_4$: C, 70.36; H, 6.79; N, 4.10

Found: C, 70.26; H, 6.90; N, 3.95.

C. 2,3-Dihydro-N-methyl-6-(2-methyl-1,3-dioxan-2-yl)-1H-phenalen-2-amine and its (Z)-2-butenedioate (1:1).

A solution of 2,3-dihydro-6-(2-methyl-1,3-dioxan-2-yl)-1H-phenalen-2-yl carbamic acid ethyl ester (2.5 g; 7.4 mmole) in 30 ml of THF was added during 3 min to a solution of LAH (2.5 g) in 100 ml of THF, and the mixture was refluxed 3 hr. It was cooled in ice and was treated in succession with 2.5 ml water, 2.5 ml 15% NaOH, and 7.5 ml water. The suspension was stirred one hr at room temperature, filtered, the cake was washed with THF, and the combined filtrate evaporated to give 2 g of the titled amine compound. NMR ($CDCl_3$) δ 1.87 (s, 3, C-Me), 2.55 (s, 3, N-Me), 2.80-3.40 (m, 5, N-$CH(CH_2)_2$), 3.65-4.15 (m, 4, $OCH_2CH_2O$), 7.10-8.50 (m, 5, aromatic).

HR mass spec. Calcd. for $C_{18}H_{17}NO_2$: 283.1572.

Found: 283.1553.

The maleic acid salt of this amine was prepared in diethyl ether witn an equimolar amount of maleic acid and was recrystallized from methanol/diethyl ether, m.p. 193-194°C. UV λ max 211 nm (ε 44,100), sh 218 (44,750), 224 (61,700), 231, (87,650), sh 269 (4,650), sh 281 (7,650), 290 (9,250), sh 301 (6,500), sh 308 (3,750), sh 316 (895), 322 (679). IR $NH^+$/acid OH 2794, 2731, 2626, 2505; $CO_2^-$/C=C/CH def. 1628, 1601, 1578, 1514, 1462, 1350; C-O 1192, 1043, 1037, 987; aromatic 875. Mass spec. $M^+$283.

Anal. Calcd. for $C_{18}H_{21}NO_2 \cdot C_4H_4O_4$: C, 66.15; H, 6.31; N, 3.51.

Found: C, 66.27; H, 6.38; N, 3.46

<u>Example 24</u> [2,3-Dihydro-2-(methylamino)-1H-phenalen-6-yl]-(phenyl)methanone, and its methanesulfonate salt.

A. A preparation of 6-benzoyl-2,3-dihydro-1H-phenalen-2-yl carbamic acid ethyl ester.

Benzoyl chloride, 3.51 g (0.025 mole) was added to a solution of 5.1 g (0.02 mole) of 2-(ethoxycarbonylamino)-2,3-dihydro-1H-phenalene in 100 ml of ethylene chloride. While keeping the temperature of the mixture at 20-25°C, 9.33 (0.025 mole) of aluminum trichloride was added portionwise over 5 min. The mixture was stirred at room temperature for two hr. The mixture was cooled in ice, ice was added followed by 250 ml of 10% v/v hydrochloric acid solution. The resulting mixture was extracted with diethyl ether and the organic liquid phase was washed twice with aqueous sodium bicarbonate solution, once with saturated sodium chloride solution, then dried with magnesium sulfate and evaporated. The residual brown oily solid, 6 g, was subjected to LPLC purification using a 635 ml Michel-Miller column and 230-240 mesh silica gel. Elution of the column with a 5% v/v ethyl acetate in cyclohexane mixture as elution liquid separated impurities. Fractions 1 to 50 (30 ml each) contained impurities. Elution with 20% v/v ethylacetate in cyclohexane mixture (fractions 51 to 79 (40 ml each) gave impurities. Fractions 80 to 100 gave 4.1 g of the subtitled ketone. Crystallization from a minimum of ethanol at 0°C gave 2.9 g of the more pure, subtitled compound, m.p. 111.5-113°C. The analytical sample melted at 113-114°-C. UV sh 214 (39,200), λ max 225 (51,650), 250 (20,000), sh 285 (6,550), 3020 (5,950). IR NH/=CH 3333, 3084, 3064, 3056, 3041; C=O (carbamate) 1712; C=O (ketone 1650); C=C 1595, 1582, 1510; amide II 1531; C-O/C-N/other 1278, 1267, 1237, 1225, 1060, 1046, 958; aromatic 838, 827, 765, 709. NMR ($CDCl_3$) δ 1.22 (t, 3, $CH_3$, J=7.1 Hz). 2.9-3.65 (m, 4, $CH_2$-C-$CH_2$), 4.11 (quartet, 2, $OCH_2$, J=7.1 HZ), 4.25-4.80 (m, ~2, $NH^+$, CH-N), 7.10-8.05 (m, 10, aromatic). Mass spec. $M^+$359.

Anal. Calcd. for $C_{23}H_{21}NO_3$: C, 76.86; H, 5.89; N, 3.90.
Found: C, 76.90; H, 5.93; N, 3.86.

B. Preparation of [2,3-Dihydro-2-(methylamino)-1H-phenalen-6-yl](phenyl)methanol.

A solution of 1.91 g (5.3 mmoles) of 6-benzoyl-2,3-dihydro-1H-phenalen-2-yl carbamic acid ethyl ester, from part A hereinabove, in 20 ml of THF was added during 5 min to a solution of 1.9 g of LAH in 65 ml of THF and refluxed one hr. The mixture was cooled in ice,

decomposed in succession with 1.9 ml of water, 1.9 ml of 15% w/v sodium hydroxide in water solution and 5.7 ml of water. The mixture was stirred at room temperature for one hr, the resulting suspension was filtered and the filter solid was washed with THF. The filtrate was dried with magnesium sulfate and evaporated to give 1.7 g of the subtitled compound as a solid, which solid was used directly in the next step. An IR spectrum of a sample of this product showed the absence of any starting material containing the carbonyl group absorption. UV λ max 213 nm (27,950), sh 227 (42,250), 232 (50,400), sh 271 (4,050), sh 284 (6,900), 292.5 (8,350), sh 302 (6,100), sh 308, (4,600), sh 317 (1,250), sh 322 (841). NMR ($CDCl_3$) δ 2.47 (s, 3, $CH_3$), 2.60-3.40 (m, 5, $NCH(CH_2)_2$), 6.45 (s, 1, CH-O), 7.0-8.0 (m, 10, aromatic). Mass spec. $M^+$303.

C. [2,3-Dihydro-2-(methylamino)-1H-phenalen-6-yl]-(phenyl)methanone, and its methanesulfonate salt.

Jones Reagent [J. Chem. Soc., (London) 1946, pp. 39-45 (chromic trioxide/water/sulfuric acid], 1.39 ml was added dropwise during 3 min to a solution of the [2,3-dihydro-2-(methylamino)-1H-phenalen-6-yl](phenyl)methanol, from part B hereinabove, 1.7 g (5.6 mmole) in 100 ml of acetone. The reaction was followed by thin layer chromatography (TLC) procedures. After 15 min, two additional drops of Jones Reagent were added. The mixture was stirred for 5 min, ice was added followed by 15% w/v sodium hydroxide. Acetone was evaporated in vacuo at 30°C, the product was extracted well with diethyl ether. The ether extract was washed with saturated sodium chloride, aqueous solution, dried with magnesium sulfate and evaporated to give 1.1 g of the above-titled compound, as a crude oily solid. This oily solid was subject to LPLC using a 110 ml Michel-Miller column, containing 230-240 mesh silica gel, and using chloroform as eluting liquid, collecting 10 ml fractions. Fractions 1 to 22 give impurities. Elution of the column with a 10% v/v methanol in chloroform containing 2% v/v ammonium hydroxide mixture gave no material in Fractions 22 to 29. Fractions 30 to 43 gave 0.67 g of the purified herein titled amine. An IR spectrum showed carbonyl (-C(O)-) absorption at 1650. NMR ($CDCl_3$) δ 257 (s, 3, $CH_3$); 2.7-3.5 (m, 6, N-$CH(CH_2)_2$); 7.0-8.05 (m, 10, aromatic).

A solution of methanesulfonic acid (213 mg) in diethyl ether was added to the solution of the above amine oil in ether. The resulting gum was crystallized from a methanol/diethyl ether mixture to give 0.63 g, m.p. 153-154°C of the titled amine methanesulfonate salt. UV sh 213 (38,750), λ max 225 (50,100), 249 (19,150), 288 (6,650), 309 (5,850), sh 321 (5,700). IR =CH 3021; $NH_2^+$ 3000 b., 2743, 2429; C=O 1659, C=C/$NH_2^+$ 1632, 1618, 1595, 1579, 1513; $SO_3$ 1215, 1167, 1139, 1049; aromatic 813, 777, 764, 716, 687. Mass spec. $M^+$301.

Calcd. for $C_{21}H_{19}NO \cdot CH_3SO_3H$: C, 66.47; H, 5.83; N, 3.54; S, 8.07.

Found: C, 66.38; H, 5.70; N, 3.34; S, 8.19.

Example 25 1,[2,3-dihydro-2-(methylamino)-1H-phenalen-6-yl]-ethanone and its monohydrochloride.

A mixture of 2,3-dihydro-N-methyl-6-(2-methyl-1,3-dioxan-2-yl)-1Hphenalen-2-amine, from Example 23 hereinabove (0.3 g; 1.06 mmole), 3 ml of diethyl ether and 3 ml of concentrated HCl was stirred at room temperature for two hr. Ice was added, the mixture was basified with 15% NaOH, and extracted well with ether. The ether extract was washed with water, saturated salt solution, dried ($MgSO_4$) and evaporated to give 0.25 of the titled compound as an oil, nmr ($CDCl_3$) δ 1.47 (s, 1, NH exch), 2.51 (s, 3, $COCH_3$), 2.69 (s, 3, NMe), 2.75-3.50 (m, 5, N-$CH(CH_2)_2$), 7.10-8.70 (m, 5, aromatic).

The hydrochloride of this titled amine was prepared with ethereal HCl, and was crystallized from methanol/diethyl ether, 0.251 g, m.p. 213-214° UV λ max 215 nm (ε 29,250), sh 222 (27,950), 242 (19,400), 313 (7,250), IR =CH 3038, 3020; $NH^+$ 2791, 2749, 2707, 2478, 2424; C=O 1681, C=C/$NH_2^+$ 1597, 1586; 1512, aromatic 829, 811, 769. NMR ($D_2O$) δ 2.70 (s, 3, $COCH_3$), 2.8 (s, 3, $NCH_3$), 3.0-3.7 (m, 5, N-$CH(CH_2)_2$), 7.15-8.35 (m, 5, aromatic). Mass spec. $M^+$239.

Anal. Calcd. for $C_{16}H_{17}NO \cdot HCl \cdot 1/3\ H_2O$: C, 68.20; H, 6.67; Cl, 12.58: N, 4.97.

Found: C, 68.53; H, 6.47; Cl, 12.78; N, 4.96.

Example 26 N-[2,3-Dihydro-6-(2-methyl-1,3-dioxolan-2-yl)-1H-phenalen-2-yl]dimethylamine.

Sodium cyanoborohydride ($NaCNBH_3$) (0.126 g; 2 mmole) was added to a solution of 2,3-dihydro-N-methyl-6-(2-methyl-1,3-dioxan-2-yl)-1H-phenalen-2-amine, free base from Example 23 hereinabove (0.283 g;

1 mmole) and 0.5 ml of aqueous 37% formaldehyde solution in 5 ml of acetonitrile and 2 ml of methanol. The pH was adjusted to 7 with acetic acid and the mixture stirred for 30 min. The pH was adjusted again and the mixture stirred for 3 hr. Additional 0.5 ml of formaldehyde and 0.126 g $NaCNBH_3$ were added, pH adjusted to 7 and the mixture stirred overnight. It was evaporated at 35°C in vacuo, taken up in water and the aqueous phase was extracted with ether. The combined ether was washed with water, saturated salt solution, dried ($MgSO_4$) and evaporated to give 0.29 g of the titled -dimethylamine compound as a yellow oil, UV λ max 211 (ε 21900), sh 225 (42,300), 231 (65,300), sh 269 (3,550), 281 (6,150), 291 (7,550), 301 (5,350), sh 307 (3,350), sh 316 (842) 322 (601). IR showed boron and carbonyl impurities. The titled amine showed =CH 3101, 3069; CH 2954, 2889; NCH 2779; C=C 1600, 1514; CH/C-O/C-N 1189, 1133, 1103, 1038; aromatic 866, 831, 811, 768. NRM ($CDCl_3$) δ 1.85 (s, 3, C-$CH_3$), 2.45 (s, 6, $NMe_2$) 2.90-3.35 (m, 5, N-CH($CH_2$)$_2$), 3.60-4.10 (4, $OCH_2CH_2O$), 7.10-8.50 (m, 5, aromatic). Impurities were present.

HR mass spec. Calcd for $C_{19}H_{23}NO_2$: 297.1729.

Found: 297.1720.

Example 27 1-[2,3-Dihydro-2-(dimethylamino)-1H-phenalen-6-yl]-ethanone butanedioate (1:1).

A mixture of the crude N-[2,3-dihydro-6-(2-methyl-1,3-dioxan-2-yl)1H-phenalen-2-yl]-N,N-dimethylamine, from Example 26 hereinabove (1.5 g) 30 ml of 10% HCl, and 10 ml of diethyl ether was stirred at room temperature for 3 hr. The suspension was filtered, and the solid washed with water, and then diethyl ether to separate the by-product impurity, 0.28 g, m.p. 145-147°C efferv. This boron containing by-product was not elucidated.

The filtrate was cooled, basified with 15% NaOH, the product was extracted well with diethyl ether, the ether extract was washed with saturated salt solution, dried ($MgSO_4$) and evaporated to give the titled product as an oil 0.75 g. IR showed a strong C=O at 1675. NMR($CDCl_3$) δ 2.45 (s, 6, $NMe_2$), 2.70 (s, 3, C-$CH_3$), 2.75-3.70 (m, 5, N-CH($CH_2$)$_2$), 7.05-8.70 (m, 5, aromatic).

A solution of the above amine oil (0.62 g; 2.44 mmole) in 2 ml. of methanol was added to a solution of succinic acid (0.288 g; 2.44 mmole) in 2 ml. methanol. Diethyl ether was added to cloudiness

and the mixture was cooled to give 0.37 g of the titled amine salt, m.p. 126°-127°C. dec. UV λ max 214 nm (ε 28,950), 223 (27,600), sh 228 (26,650), 243 (18,150), 316 (7,000). IR -CH 3104, 3048; $NH^+$/acid OH 2716, 2586, 2436, 1895; C=O (acid) 1720; C=O (ketone) 1672; C=C 1597, 1516; $CO_2^-$/CO 1413, 1324, 1294, 1265, 1190; aromatic 829, 813, 775, 743. Mass spec. $M^+$253.

Anal. Calcd. for $C_{17}H_{19}NO \cdot C_4H_6O_4 \cdot 1/3\ H_2O$: C, 66.82; H, 6.85; N, 371.

Found: C, 66.82; H, 6.95; N, 3.51.

Example 28 [2-(Di-methylamino)-2,3-dihydro-α-methyl-1H-phenalen-6-yl]methanol and its hydrochloride, or named another way - 1-[2-(Dimethylamino)-2,3-dihydro-1H-phenalen-6-yl]-1-ethanol, and its hydrochloride salt.

A solution of the 6-acetyl compound, 1-[2,3-dihydro-2-(dimethylamino)-1H-phenalen-6-yl]ethanone free base, from Example 27 hereinabove, (0.3 g; 1.2 mmole) in 20 ml of diethyl ether was added during 5 min to a solution of lithium aluminum hydride (LAH), 0.18 g., in 10 ml of diethyl ether and the mixture was refluxed for 30 min. The mixture was then cooled, and treated in succession with 0.2 ml of water, 0.2 ml of 15% sodium hydroxide in water solution, and 0.6 ml of water. The suspension was stirred at room temperature for 30 min, filtered, and the filtrate was dried with magnesium sulfate and evaporated to give 0.2 g of the title compound as an oil. An IR spectral analysis indicated absence of the starting material carbonyl group. NMR ($CDCl_3$) δ 1.65 (d,3,$CH_3$,J ~8 Hz), 2.43 (s,6,$NMe_2$), 2.65-3.50 (m,5,N-CH($CH_2$)$_2$), 5.6 (quartet, 1,CHO,J ~8 Hz), 7.10-8.0 (m,5,-aromatic).

The hydrochloride of this amine was prepared in ethereal HCl, and was crystallized from MeOH-ether at -10°, m.p. 241-242° dec. UV λmax 212 (25,300), sh 225 (47,050), 231 (65,150), sh 282 (6,750),291 (8,500), sh 300 (6,250), sh 308 (3,700), sh 317 (1,250), 322 (951). IR OH 3351; -CH 3069, 3037; $NH^+$ 2640, 2574, 2526, 2477; C=C 1602, 1515, 1492; C-O/C-N 1159, 1071, 1016, 967; aromatic 829, 810, 758.

HR Mass spec. Calcd. for $C_{17}H_{21}NO$: 255.1623.

Found: 255.1615.

Example 29 [2,3-Dihydro-2-(N,N-dimethylamino)-1H-phenalen-6-yl]-(phenyl)methanone.

A. Preparation of [2,3-dihydro-2-(N-methyl-N-ethoxycarbonylamino)-1H-phenalen-6-yl]-(phenyl)methanone.

A 1 g (2.8 mmole) portion of [2,3-dihydro-2-(N-ethoxycarbonylamino)-1H-phenalen-6-yl]-(phenyl)methanone was added to a suspension of sodium hydride (0.134 g; 2.8 mmole) of a 50% w/v dispersion in mineral oil, washed with petroleum ether (b.p 30-60°C) in 20 ml of N,N-dimethylformamide (DMF) and the mixture was stirred for one hr. Methyl iodide (0.79 g; 5.6 mmole) was added and the reaction mixture was stirred for 19 hr. TLC analysis of the reaction mixture sample indicated incomplete reaction. An additional 1 ml of methyl iodide was added and the mixture was heated at 65-70°C for several hours. The mixture was evaporated, and the residue was taken up in a chloroform/water mixture. The organic liquid layer was separated and washed with water, saturated sodium chloride solution, dried with magnesium sulfate and evaporated. The residue, 0.9 g., was subjected to LPLC separation procedure using a 110 ml Michel-Miller column containing 230-400 mesh silica gel and a 5% v/v ethyl acetate in cyclohexane mixture as eluting liquid, collecting 10 ml fractions. Fractions 1 to 30 gave no material. Fractions 31 to 80, using 7% ethyl acetate in cyclohexane as eluting liquid gave no material. Fractions 81 to 94 gave 0.452 g of the sub-titled product. Fractions 95 to 103 gave no material. Fractions 104 to 122 gave 0.203 g of the starting material.

The methylated, sub-titled product was crystallized from cold ethanol to obtain the above named product as small, yellow prism crystals, m.p. 154-155°C. UV sh 214 nm (ε 37,450), λmax 226 (49,650), 251 (19,400), 289 (6,500), 313 (5,800), 322 (5,950). IR - CH 3089, 3059, 3039; C = O (carbamate) 1686; C = O (ketone) 1650; C = C 1594, 1580, 1543, 1512, 1490; C-O/C-N 1301, 1276, 1254, 1231, 1142, 965; aromatic 824, 770, 725, 702. NMR ($CDCl_3$), δ 1.27 (t,3,C-$CH_3$, J=7.1 Hz), 2.97 (s,3,N-$CH_3$), 3.05-3.55 (m,4,N-C($CH_2$)$_2$) 4.19 (quadr., 2,$OCH_2$, J=7.1 Hz), 4.7 (center of multiplit,1,CH-N), 7.10-8.0 (m,10,aromatic). Mass spec. $M^+$372.

Anal. Calcd. for $C_{24}H_{23}NO_3$: C, 77.19; H, 6.21; N, 3.75.

Found: C, 77.42; H, 6.35; N, 3.64.

B. [2,3-Dihydro-2-(N,N-dimethylamino)-1H-phenalen-6-yl]-(phenyl)-methanol.

A solution of the [2,3-dihydro-2-(N-methyl-N-ethoxycarbonylamino)-1H-phenalen-6-yl]-(phenyl)methanone from part A hereinabove (0.42 g; 1.1mmole) in 5 ml of THF was added during 2 minutes to a solution of 0.42 g of LAH in 10 ml of THF. The mixture was refluxed for 1 hr; cooled in ice, and treated in succession with 0.5 ml of water, with 0.5 ml of 15% w/v sodium hydroxide solution, and 1.5 ml of water. The mixture was stirred 1 hr at room temperature, filtered, the filter cake was washed well with THF, and the combined organic filtrate and wash liquid was evaporated to leave as residue 0.27 g of the titled di-methyl amine compound. The IR spectrum showed no carbonyl from the starting material. UV λmax 214 nm (ε 32,350), sh 228 (45,650), 232 (54,050), sh 271 (4,300), sh 284 (7,750), 293 (9,450), sh 302 (6,900), sh 309 (5,400), sh 319 (1,450). NMR ($CDCl_3$) δ 2.39 (s,6,$NME_2$), 2.55-3.40 (m,5,N-$CH(CH_2)_2$), 6.45 (s,1,CH-O), 6.95-8.00 (m,10,aromatic). Mass spec. $M^+$317.

Example 30 [2,3-Dihydro-2-(dimethylamino)-1H-phenalen-6-yl)-(phenyl)methanone, and its (Z)-2-butenedioate (maleate) salt.

Jones reagent (chromium trioxide/water/conc. sulfuric acid), (0.2 ml; 0.55 mmole) was added dropwise to a solution of the methanol, [2,3-dihydro-2-(N,N-dimethylamino)-1H-phenalen-6-yl]-(phenyl)-methanol, from Example 27 hereinabove (0.26 g; 0.82 mole) in 10 ml of acetone. The resulting mixture was stirred for 30 min, and then analyzed by the procedures. Another drop of Jones reagent was added, the resulting mixture was stirred for 15 min and then ice was added. The mixture was made pH basic, the acetone was evaporated in vacuo at 30°C and the product was extracted well with diethyl ether. The resulting ether extract was washed with saturated sodium chloride solution, dried with magnesium sulfate and evaporated. The residue, 0.18 g., was subjected to LPLC purification on a 12 ml. Michel-Miller column, containing 230-240 mesh silica gel, using chloroform as eluting liquid. Five ml fractions were collected. Fractions 20 to 25 gave 0.142 g of the desired, titled amine compound. NMR ($CDCl_3$) δ 2.46 (s,6,$N(CH_3)_2$; 2.90-3.50 [m,5,N-$CH(CH_2)_2$]; 7.10-8.05 (m,10,aromatic).

The maleate salt of diamine was prepared in diethyl ether using an equimolar amount of maleic acid, and was crystallized from a

methanol/diethyl mixture, m.p. 168-169°. UV λ max 211 (56,600), 221 (55,650), 249 (20,100), 288 (6,700), 310 (5,900), sh 319 (5,850). IR-CH 3068, 3056, 3038, 3022; $NH^+$/acid OH 2607, 2464; C=O (maleate) 1700; C=O (ketone) 1653; $CO_2$-/C=C 1596, 1587, 1579, 1483, 1358; C-O 1278, 1019, 1001; aromatic 871, 832, 813, 759, 707. Mass spec. $M^+$315.

Anal. Calcd. for $C_{22}H_{21}NO \cdot C_4H_4O_4$: C, 72.37; H, 5.84; N, 3.25.
Found: C, 72.44; H, 5.83; N, 3.25.

Example 31 [2,3-Dihydro-2-(dimethylamino)-1H-phenalen-6-yl]-(phenyl)methanone, and its (Z)-2-butenedioate (maleate) salt.

Jones reagent (chromium trioxide/water/conc. sulfuric acid), (0.2 ml; 0.55 mmole) was added dropwise to a solution of the methanol, [2,3-dihydro-2-(N,N-dimethylamino)-1H-phenalen-6-yl]-(phenyl)-methanol, from Example 30 hereinabove (0.26 g; 0.82 mole) in 10 ml of acetone. The resulting mixture was stirred for 30 min, and then analyzed by the procedures. Another drop of Jones reagent was added, the resulting mixture was stirred for 15 min and then ice was added. The mixture was made pH basic, the acetone was evaporated in vacuo at 30°C and the product was extracted well with diethyl ether. The resulting ether extract was washed with saturated sodium chloride solution, dried with magnesium sulfate and evaporated. The residue, 0.18 g, was subjected to LPLC purification on a 12 ml Michel-Miller column, containing 230-240 mesh silica gel, using chloroform as eluting liquid. Five ml fractions were collected. Fractions 20 to 25 gave 0.142 g of the desired, titled amine compound. NMR ($CDCl_3$) δ 2.46 (s,6,$N(CH_3)_2$; 2.90-3.50 [m,5,$N-CH(CH_2)_2$]; 7.10-8.05 (m,10,aromatic).

The maleate salt of diamine was prepared in diethyl ether using an equimolar amount of maleic acid, and was crystallized from a methanol/diethyl mixture, m.p. 168-169°. UV λmax 211 (56,600), 221 (55,650), 249 (20,100), 288 (6,700), 310 (5,900), sh 319 (5,850). IR -CH 3068, 3056, 3038, 3022; $NH^+$/acid OH 2607, 2464; C=O (maleate) 1700; C=O (ketone) 1653; $CO_2$-/C=C 1596, 1587, 1579, 1483, 1358; C-O 1278, 1019, 1001; aromatic 871, 832, 813, 759, 707. Mass spec. $M^+$315.

Anal. Calcd. for $C_{22}H_{21}NO \cdot C_4H_4O_4$: C, 72.37; H, 5.84; N, 3.25.

Found: C, 72.44; H, 5.83; N, 3.25.

Example 32

One thousand tablets for oral use, each containing 40 mg of 2-(N,N-di-n-propylamino)-2,3-dihydro-1H-phenalen-5-ol succinate as the essential active ingredient are prepared from the following ingredients:

| | |
|---|---|
| Essential active ingredient | 40 gm |
| Dicalcium phosphate | 150 gm |
| Methylcellulose, USP (15 cps) | 6.5 gm |
| Talc | 20 gm |
| Calcium stearate | 2.0 gm |

The essential active ingredient and dicalcium phosphate are mixed well, with 7.5% aqueous solution of methylcellulose, passed through a No. 8 screen and dried carefully. The dried granules are passed through a No. 12 screen, mixed with the talc and stearate and compressed into tablets. These tablets are useful in the treatment of psychoses in adult humans at a dose of 1 tablet 1-4 times a day as needed.

Example 33

One thousand two-piece hard gelatin capsules for oral use, each capsule containing 20 mg of (±) 2-(N,N-propylamino)-2,3-dihydro-1H-phenalen-5-ol succinate as the essential active ingredient are prepared from the following ingredients:

| | |
|---|---|
| Essential active ingredient | 20 gm |
| Lactose, USP | 100 gm |
| Starch, USP | 10 gm |
| Talc, USP | 5 gm |
| Calcium stearate | 1 gm |

The finely powdered materials are mixed thoroughly, then filled into hard gelatin capsules of appropriate size.

One capsule 4 times daily is useful for the treatment of psychoses in adult humans.

Example 34

One-piece soft elastic capsules for oral use, each containing 100 mg (+)-2-(N,N-di-n-propylamino)-2,3-dihydro-1H-phenalen-5-ol succinate as the essential active ingredient are prepared in the usual

manner by first dispersing the active material in sufficient corn oil to render the material capsulatable.

One capsule two times daily is useful in the treatment of psychoses in adult humans.

Example 35

An aqueous oral preparation containing in each teaspoonful (5 ml) 80 mg of (±) 2-(N,N-di-n-propylamino-5-methoxy-2,3-dihydro-1H-phenalene succinate as the essential active ingredient is prepared from the following ingredients:

| | |
|---|---|
| Essential active ingredient | 160 gm |
| Methylparaben, USP | 7.5 gm |
| Propylparaben, USP | 2.5 gm |
| Saccharin | 12.5 gm |
| Glycerine | 3000 ml |
| Tragacanth powder | 10 gm |
| Orange oil flavor | 10 gm |
| Orange II | 7.5 gm |
| Deionized water, g.s. to | 10000 ml |

The foregoing aqueous preparation is useful in the treatment of psychoses at a dose of 1 teaspoonful 4 times daily.

Example 36

One thousand tablets for oral administration, each containing 10 mg of (±) 2-(N,N-di-n-propylamino)-2,3-dihydro-1H-phenalen-5-ol succinate as the essential active ingredient are prepared from the following ingredients:

| | |
|---|---|
| Essential active ingredient, micronized | 10 gm |
| Lactose | 150 gm |
| Starch | 15 gm |
| Magnesium stearate | 1.5 gm |

The ingredients are thoroughly mixed and slugged. The slugs are broken down by forcing through a screen and the resulting granules are then compressed into tablets.

These tablets are useful in reducing post-surgical pain in dogs at a dose of 1-3 tablets depending on the weight of the animal and its condition.

Example 37

A sterile, aqueous suspension for intramuscular injection and containing in each milliliter 50 mg of (±)-2-(N,N-di-n-propylamino)-2,3-dihydro-1H-phenalen-5-ol succinate as the essential active ingredient is prepared from the following ingredients:

| | |
|---|---|
| Essential active ingredient | 5 gm |
| Polyethylene glycol 4000, USP | 3 gm |
| Sodium chloride | 0.9 gm |
| Polysorbate 80, USP | 0.4 gm |
| Sodium metabisulfite | 0.1 gm |
| Methylparaben, USP | 0.18 gm |
| Propylparaben, USP | 0.02 gm |
| Water for injection, q.s. to | 100 ml |

The preceding sterile injectable is useful in the treatment of schizophrenia at a dose of one-half to 2 ml.

Examples of other 2-amino-2,3-dihydro-1H-phenalene compounds within the scope of this anti-psychotic drug method of use and/or compound per se invention which can be made by methods described above include the following.

2,3-dihydro-methyl-1H-phenalen-2-amine,

6-bromo-2,3-dihydro-N,N-dimethyl-1H-phenalen-2-amine,

2-(dimethylamino)-2,3-dihydro-1H-phenalen-6-ol,

6-bromo-2,3-dihydro-N,N-di-n-propyl-1H-phenalen-2-amine,

2,3-dihydro-N-methyl-6-(2-methyl-1,3-dioxan-2-yl)-1H-phenalen-2-amine,

2,3-dihydro-N,N-dimethyl-1H-phenalen-2-amine,

2,3-dihydro-N,N-dimethyl-6-(2-methyl-1,3-dioxan-2-yl)-1H-phenalen-2-amine,

2,3-dihydro-N,N-di-n-propyl-1H-phenalen-6-ol,

1-(2,3-dihydro-2-(N,N-dimethylamino)-1H-phenalen-6-yl)-1-ethanol,

1-(6-bromo-2,3-dihydro-1H-phenalen-2-yl)azetidine,

2,3-dihydro-N,N-dimethyl-5-(phenylmethoxy)-1H-phenalen-2-amine,

1-[2,3-dihydro-2-(methylamino)-1H-phenalen-6-yl]ethanone,

2-(N,N-di-n-propylamino)-2,3-dihydro-1H-phenalen-5-ol,

2,3-dihydro-5-(phenylmethoxy)-N,N-(di-n-propyl)-1H-phenalen-2-amine,

[2,3-dihydro-2-(N,N-dimethylamino)-1H-phenalen-6-yl]phenone,

which can also be named

[α-5-(dimethylamino)-5,6-dihydro-4H-phenalen-1-yl]phenylmethanone,

[2,3-dihydro-2-(methylamino)-1H-phenalen-6-yl]phenone,

2,3-dihydro-2-(N-methylamino)-6-phenylmethoxy)-1H-phenalen-2-amine,

2,3-dihydro-2-(N,N-dimethylamino)-1H-phenalen-5-ol,

2,3-dihydro-4-methoxy-1H-phenalen-2-amine,

2,3-dihydro-4-methoxy-N,N-dipropyl-1H-phenalen-2-amine,

1-(6-bromo-2,3-dihydro-1H-phenalen-2-yl)pyrrolidine,

6-bromo-2,3-dihydro-1H-phenalen-2-amine,

2-(N,N-di-n-propylamino)-2,3-dihydro-1H-phenalen-4-ol,

1-(6-bromo-2,3-dihydro-1H-phenalen-2-yl)piperadine,

2,3-dihydro-5-methoxy-1H-phenalen-2-amine,

2,3-dihydro-5-methoxy-2-(N,N-dimethyl)-1H-phenalen-2-ylamine,

2,3-dihydro-4-methoxy-N,N-dimethyl-1H-phenalen-2-amine,

2-(N,N-dimethylamino)-2,3-dihydro-1H-phenalen-4-ol,

2-(N,N-di-n-propylamino)-2,3-dihydro-1H-phenalen-5-ol, acetate ester,

2,3-dihydro-5-(phenylmethoxy)-1H-phenalen-2-amine,

2-(N,N-di-n-propylamino)-2,3-dihydro-1H-phenalen-5-ol, 2,2-dimethyl propanoate ester,

2-(N,N-di-n-propylamino)-2,3-dihydro-1H-phenalen-5-ol, benzoate ester,

2-(N,N-di-n-propylamino)-2,3-dihydro-1H-phenalen-5-ol, heptanate ester,

2-(N,N-di-n-propylamino)-2,3-dihydro-1H-phenalen-5-ol, ethyl carbonate (ester),

2-(N,N-di-n-propylamino)-2,3-dihydro-1H-phenalen-5-ol, phenylacetate ester,

2,3-dihydro-6-(phenoxymethyl)-1H-phenalen-2-amine,

5-chloro-2,3-dihydro-1H-phenalen-2-amine,

5-chloro-2,3-dihydro-N,N-di-n-propylamino-1H-phenalen-2-amine,

5-chloro-2,3-dihydro-N,N-dimethyl-1H-phenalen-2-amine,

N,N,-diethyl-2,3-dihydro-1H-phenalen-2-amine,

2,3-dihydro-N-n-propyl-1H-phenalen-2-amine,

2,3-dihydro-N-ethyl-1H-phenalen-2-amine,

4-[5-hydroxy-2,3-dihydro-1H-phenalen-5-yl]morpholine,

5-trifluoromethyl-N,N-di-n-propyl-2,3-dihydro-1H-phenalen-2-yl-amine,

5-trifluoromethyl-N-isopropyl-2,3-dihydro-1H-phenalen-2-amine,

6-fluoro-2-(N-tert-butylamino)-2,3-dihydro-1H-phenalene,

6-chloro-2-(N-di-alkylamino)-2,3-dihydro-1H-phenalene,

6-bromo-N-cyclopentyl-2,3-dihydro-1H-phenalen-2-amine,

1-[5-ethylthio-2,3-dihydro-1H-phenalen-2-yl]pyrrolidine,

5-(n-propylsulfinyl)-N,N-diethyl-2,3-dihydro-1H-phenalen-2-amine,

6-(n-butylsulfonyl)-N,N-di-ethenyl-2,3-dihydro-1H-phenalen-2-amine,

5-nonoyloxy-N-cyclobutyl-2,3-dihydro-1H-phenalen-2-amine,

4-methoxycarbonyl-N,N-dimethyl-2,3-dihydro-1H-phenalen-2-amine,

6-(2-ethyl-1,3-dioxan-2-yl)-N-isobutyl-2,3-dihydro-1H-phenalen-2-amine,

6-(1-hydroxybut-1-yl)-2-N,N-diethyl-2,3-dihydro-1H-phenalen-2-amine,

5-hexanoyl-N,N-di-n-butyl-2,3-dihydro-1H-phenalen-2-amine,

2,3-dihydro-2-(N,N-diethylamino)-1H-phenalen-5-ol, benzoate ester,

2,3-dihydro-2-(N,N-di-n-propylamino)-1H-phenalen-6-ol, phenylacetate ester,

2,3-dihydro-2-(N,N-dimethylamino)-1H-phenalen-5-ol, benzyl ether,

1-[2,3-dihydro-5-benzoyl-1H-phenalen-2-yl]azetidine,

2,3-dihydro-2-(N,N-dimethylamino)-1H-phenalen-5-ol, 3,4-dichloro phenylacetate ester,

2,3-dihydro-2-(N,N-di-n-propylamino)-1H-phenalen-5-yl, 4-methyl phenylmethyl ether,

2,3-dihydro-2-(N-cyclohexylamino)-1H-phenalen-6-yl, 4-methylthio phenyl ether, and the like.

<u>Example 38</u> 2,3-Dihydro-6-methyl-N,N-dipropyl-1H-phenalene-2-amine.

A. Preparation of 6-Bromo-N-(2,5-dimethylpyrrol-1-yl)2,3-dihydro-1H-phenalen-2-amine.

The 6-bromo-2,3-dihydro-1H-phenalen-2-amine hydrochloride from Example 18 (10 g) was partioned between ether and dilute sodium hydroxide to give the free base (8.45 g, 0.0322 mol). The amine was combined with acetonylacetone (2,5-hexanedione; 7.5 mls, 0.064 mol), propionic acid (0.58 ml, 7.8 mmol), and benzene (150 mls), and the mixture was refluxed through a Dean-Stark trap for 17 hours. Silica gel (80 mls, 0.040-0.063 mm) was added, and the solvent was removed in vacuo. The residue was slurried with ether, and the solvent was again removed in vacuo to leave a powdery solid. The powder was placed on a flash chromatography column that was wetted with 2.5% ethyl acetate in hexane, and the sub-titled compound was eluted with the same solvent to give a yellow oil (9.08 g, 83%). NMR ($CDCl_3$, TMS) δ2.32 (s,6,pyrrole $CH_3$), 3.0-4.0 (m,4,N-$C(CH_2)_2$), 4.35-4.85 (m,1,N-CH), 5.82 (s,2,pyrrole H), 7.0-8.2 (m,5,aromatic H). IR =CH 3100, 3094, 3068, 3061, 3033, 3017; C=C 1611, 1594, 1573, 1520, 1501; C=C/C-N/other 1400, 1382, 1295; γCH/other 846, 813, 794, 757, 749. UV (ethanol) 215 nm sl. sh. (E 33,810), 226 (58,300), 230 (68,910), 276 sl. sh. (5,240), 286 (8,940), 297 (11,310), 308 (8,000), 313 (6,790), 321 (1,690), 327 (1,420). Mass spec. m+ at m/z 339,341.

Exact mass Calcd. for $C_{19}N_{18}$ 79BrN: 339.0623.

Found: 339.0607.

B. Preparation of 6-methyl-N-(2,5-dimethylpyrrol-1-yl)2,3-dihydro-1H-phenalen-2-amine.

The bromide, from part (A), (6.52 g, 0.0192 mol) and 1,2-Bis(diphenylphosphino)ethanenickel dichloride (0.21 g, 0.40 mmol) in diethylether (75 mls) was degassed with argon and cooled in ice. Methylmagnesium bromide (3.0 M in ether, 14.1 mls, 0.0423 mol) was added over a period of 7 minutes. The mixture was refluxed for 25 hours and poured onto ice (100 mls). Diethylether and 10% HCl (40 mls) were added, and the layers were separated. The aqueous layer was extracted with ether, and the combined organics were washed with sat. $NaHCO_3$ and sat. NaCl and dried ($MgSO_4$). The solvent was removed in vacuo to leave the sub-titled compound as a yellow oil (4.91 g, 93%). NMR ($CDCl_3$, TMS) δ2.32 (s,6,pyrrole-$CH_3$), 2.66 (s,3,Ar-$CH_3$), 3.1-4.05 (m,4,N-$C(CH_2)_2$), 4.4-4.9 (m,1,N-CH), 5.82 (s,2,pyrrole H), 7.2-8.0 (m,5,aromatic H). IR =CH 3101, 3068, 3030; C=C 1612, 1599, 1576, 1518; C=C/C-N/other 1396, 1296; γCH 818, 759. UV (ethanol) 213

nm (E 33,850), 226 sh. (58,800), 231 (67,400), 272 sh. (4,570), 283 (7,740), 293 (9,870), 305 (6,980), 310 sh. (4,460), 319 sh. (1,170), 325 (1,260). Mass spec. m+ at m/z 275.

Exact mass Calcd. for $C_{20}H_{21}N$: 275.1671.

Found: 275.1674.

C. Preparation of 6-methyl-2,3-dihydro-1H-phenalen-2-amine, and its hydrochloride.

A mixture of the pyrrole from part (B) (4.38 g, 0.0159 mol), hydroxylamine hydrochloride (6.63 g, 0.095 mol), and sodium bicarbonate (5.34 g, 0.0636 mol) in absolute ethanol (180 mls) was stirred at reflux under nitrogen for 52 hours. Additional quantities of hydroxylamine hydrochloride (3.31 g, 0.0476 mol) and sodium bicarbonate (2.67 g, 0.318 mol) were added after 26 and 46 hours. The solvent was removed in vacuo, and the residue was partitioned between dilute sodium hydroxide and ether. The aqueous layer was extracted twice with ether, and the combined organics were washed with sat. NaCl and dried ($MgSO_4$). The solvent was removed in vacuo to leave the sub-titled amine as a yellow oil which solidified on standing (3.10 g, 99%). A sample (1.0 g) was dissolved in ether, and excess ethereal HCl was added. The precipitate was filtered, washed with ether, and crystallized from methanol/ether to give a colorless solid (0.78 g; m.p. 331-334 (°C). NMR (DMSO-$d^6$, 300 MHz) δ2.615 (s,3,$CH_3$); 3.15-3.48 (m,4,N-$C(CH_2)_2$); 3.58-3.72 (m,1,N-CH); 7.242, 7.266, 7.305, 7.329 (AB,J=7.1 Hz,2,$C_4$ and $C_5$ aromatic H); 7.371, 7.394 (d,J=6.9 Hz,1,$C_9$ aromatic H); 7.482, 7.510, 7.534 (dd,J=6.9 Hz and 8.3 Hz,1,$C_8$ aromatic H); 7.878, 7.905 (d,J=8.3 Hz,1,$C_7$ aromatic H); 8.543 (br. s,3,NH+). IR =CH/NH+ 3117, 3059; NH+ ~3000-2783, 2703, 2619, 2534, 2493, 2028; C=C/NH+ 1645, 1620, 1605, 1596, 1531, 1520; γCH 826, 757, 745, UV (ethanol) 213 nm (E 24,400), 225 sl. sh. (51,900), 231 (68,070), 271 sl. sh. (3,980), 281 (6,770), 291 (8,540), 291 (8,540), 302 (6,000), 309 (3,070), 318 (891), 325 (984). Mass spec. m+ for free base at m/z 197.

Exact mass Calcd. for $C_{14}H_{15}N$: 197.1204.

Found: 197.1212.

Anal. Calcd. for $C_{14}H_{15}N.HCl$: C, 71.94; H, 6.90; N, 5.99; Cl, 15.17.

Found: C, 72.27; H, 6.79; N, 6.14; Cl, 15.27.

D. Preparation of the above titled compound of this Example.

A mixture of the free base amine of U-75,088A from part C (0.50 g, 2.53 mmol), 1-bromopropane (2.77 mls, 0.0304 mol), potassium carbonate (1.05 g, 7.60 mmol), and acetonitrile (50 mls was stirred at reflux for a total of 53 hours. The reaction was followed by VPC (3% SE-30,1z8" x 3',20 mls/min flow, programmed: 100°C, 1 minute; 100°C to 250°C at 20°C/min; 250°C, 10 min). The product showed 6.92 min (95%) and 7.86 min (5%). Additional quantities of 1-bromopropane (1.39 mls, 0.0153 mol) and potassium carbonate (1.05 g, 7.60 mmol) were added at 27 hours. The solvent was removed in vacuo, and the residue was partioned between ether and dilute base. The aqueous layer was extracted twice with ether, and the combined organics were washed with saturated NaCl and dried ($MgSO_4$). The solvent was removed in vacuo to leave the sub-titled N,N-dipropylamine as a yellow oil (0.80 g). Purification by gravity chromatography ($SiO_2$, 0.063-0.200 mm; 1% $CH_3OH$, 0.1% $NH_3$, $CHCl_3$) gave the titled amine as a yellow oil (0.46 g, 65%). NMR ($CDCl_3$, 300 MHz) δ0.922 (t,J=7.3 Hz,6,N-C-C-$CH_3$); 1.517 (sextet,J=7.5 Hz, 4, N-C-$CH_2$); 2.573-2.623 (m,5 lines,4,N-$CH_2$); 2.643 (s,3,Ar-$CH_3$); 2.98-3.40 (m,5,N-CH($CH_2$)$_2$); 7.128, 7.152, 7.208, 7.232 (AB,J=7.0 HZ,2,$C_4$ and $C_5$ aromatic H); 7.246, 7.269 (d,J=6.8 Hz,1,$C_9$ aromatic H); 7.395, 7.419, 7.423, 7.446 (dd,J=6.9 Hz and 7.3 Hz,1,$C_8$ aromatic H); 7.798, 7.826 (d,J=8.4 Hz,1,$C_7$ aromatic H). IR =CH 3110, 3067, 3029, C-H 2957, 2931, 2871, 2808; C=C/other 1679, 1645; C=C 1612, 1598, 1516; C=C/C-H def. 1458, 1380; C-N/other 1068; γCH 819, 756. UV (Ethanol) 214 nm sh. (E 27,660), 230 (54,750), 273 sl. sh. (4,200), 285 sh. (7,100), 294 (8,820), 304 sh. (6,700), 311 (4,880), 319 sl. sh. (1,800), 326 (1,650). Mass spec. m+ at m/z 281.

Exact mass Calcd. for $C_{20}H_{27}N$: 281.2143.

Found: 281.2137.

Example 39 2,3-Dihydro-N,N,6-trimethyl-1H-phenalen-2-amine and its maleate salt.

A. Preparation of ethyl (2,3-dihydro-6-methyl-1H-phenalen-2-yl carbonate (U-75,090).

The free base of 6-methyl-2,3-dihydro-1H-phenalen-2-amine hydrochloride from Example 38, part C (1.60 g, 8.11 mmol) and triethylamine (1.5 mls, 0.011 mol) were dissolved in tetrahydrofuran (50 mls)

and a solution of ethyl chloroformate (0.97 g, 8.92 mmol) in tetrahydrofuran (20 mls) was added dropwise under an atmosphere of nitrogen. The mixture was stirred for 2 hours, diluted with ether, and washed with dilute HCl, Sat. $NaHCO_3$, and Sat. NaCl. The solvent was removed in vacuo to leave a brown solid (2.04 g). Crystallization from acetonitrile gave the sub-titled carbamate ester as a tan solid (U-75,090; 1.26 g, 58%; m.p. 116.5-117.5°C). IR NH 3307; =CH/NH 3065, 3033; C=O 1704, 1682; C=C 1599; C=C/other 1545; =C-O/=C-N/other 1303, 1286, 1233; C-O 1043; γCH 826, 767. UV (ethanol) 213 nm (E 25,920), 226 sl. sh. (45,500), 230 (70,010), 275 sl. sh. (5,360), 282 (7,170), 292 (9,100), 304 (6,350), 310 (3,860), 318 (1,080), 325 (1,250). Mass spec. m+ at m/z 269.

Exact mass Calcd. for $C_{17}H_{19}NO_2$: 269.1416.
Found: 269.1418.

Anal. Calcd. for $C_{17}H_{19}NO_2$: C, 75.81; H, 7.11; N, 5.20.
Found: 76.09; H, 7.06; N, 5.21.

B. Preparation of 2,3-dihydro-N,6-dimethyl-1H-phenalen-2-amine, and its (Z)-2-butenedioate (1:1) salt.

A solution of the urethane (carbamate ester from part A) (U-75,090; 1.00 g, 3.71 mmol) in tetrahydrofuran (20 mls) was added dropwise to a suspension of lithium aluminum hydride (1.0 g, 0.026 mol) in tetrahydrofuran (30 mls). The mixture was stirred at room temperature for 1 hour and refluxed on the steam bath for 45 minutes. The mixture was cooled in ice and water (1.0 ml), 15% NaOH (1.0 ml), and water (3.0 ml) were added in succession. The mixture was diluted with ether and stirred at room temperature for 30 minutes. The mixture was filtered and the aluminum cake was washed well with ether. The filtrate was dried ($MgSO_4$), and the solvent was removed in vacuo to leave the sub-titled amine as a pink oil (0.85 g; 100%) which solidified on standing in the refrigerator. A sample (0.45 g) was mixed with maleic acid (0.27 g) and the resulting salt was crystallized from ethanol (-10°C) and washed with ether to give an off-white solid (0.58 g; m.p. 169-170°C). NMR (DMSO-$d^6$, 300 MHz) δ2.608 (s,3,Ar-$CH_3$); 2.730 (s,3,N-$CH_3$); 3,10-3.55 (m,4,N-C$(CH_2)_2$); 3.63-3.73 (m,1,N-CH); 6.028 (s,2,maleic acid CH=CH); 7.257, 7.281, 7.312, 7.336 (ab, J=7.2 Hz, 2, $C_4$ and $C_5$ aromatic H); 7.386, 7.409 (d,J=6.9 Hz,1,$C_9$ aromatic H); 7.490, 7.513, 7.518, 7.541 (dd, J=6.9 Hz and 8.4 Hz,1,$C_8$

aromatic H); 7.882, 7.910 (d,J=8.3 Hz,1,$C_7$ aromatic H); 8.709 (br. s,2,NH+). IR =CH 3017; NH+/acid OH 2731, 2489; C=O/C=C/$CO_2$- 1642, 1614, 1601, 1573; CH def./$CO_2$- 1466; $CO_2$-/other 1354; maleate 991, 870, 699; γCH 834, 769. UV (ethanol) 213 nm (E 40,730), 225 sl. sh. (57,200), 230 (68,600), 273 sl. sh. (4,480), 281 (7,000), 291 (8,620), 302 (6,040), 309 (3,160), 318 (903), 324 (970). Mass spec. m+ for free base at m/z 211.

Exact mass Calcd. for $C_{15}H_{17}N$: 211.1361.
Found: 211.1367.

Anal. Calcd. for $C_{15}H_{17}N.C_4H_4O_4$: C, 69.71; H, 6.47; N, 4.29.
Found: C, 69.64; H, 6.49; N, 4.19.

C. Preparation of 2,3-dihydro-N,N,6-trimethyl-1H-phenalen-2-amine, and its (1:1) butenedioate (maleate) salt.

A solution of sodium cyanoborohydride (0.92 g, 0.015 mol) in methanol (20 mls) was added dropwise to a solution of the free base of the N-methylamine from part B (0.34 g, 1.6 mmol), maleic acid (0.19 g, 1.6 mmol), and 37% aqueous formaldehyde (1.2 mls, ~0.015 mol) in methanol (25 mls). The mixture was stirred at room temperature for 4 hours, and the solvent was removed in vacuo. The residue was partitioned between dilute sodium hydroxide and ether. The aqueous layer was extracted again with ether, and the combined organics were extracted twice with 5% hydrochloric acid. The extracts were washed with ether and basified with 40%-sodium hydroxide. The free base was extracted twice with ether, and the extracts were washed with saturated NaCl and dried ($MgSO_4$). The solvent was removed in vacuo to leave the titled N,N-6-trimethyl-1H-phenalen-2-amine a pink oil (0.18 g, 50%). The compound was dissolved in ether, and an ether solution of maleic acid (0.10 g) was added. The precipitate was filtered and washed with ether to give a colorless solid (0.24 g; m.p. 268-168.5°C). NMR (DMSO-$d^6$, 300 MHz) δ2.493 (s,3,Ar-$CH_3$); 2.795 (s,6,N-$CH_3$); 3.10-3.40 (m,4,N-C($CH_2$)$_2$); 3.60-3.75 (n=m,1,N-CH); 5.900 (s,2,maleic acid CH=CH); 7.140, 7.164, 7.204, 7.228 (ab,J=7.1 HZ,2,$C_4$ and $C_5$ aromatic H); 7.267, 7.291 (d,J=7.1 Hz,1,$C_9$ aromatic H); 7.380, 7.403, 7.408, 7.431 (dd,J=7.0 Hz and 8.4 Hz,1,$C_8$ aromatic H); 7.769, 7.796 (d,J=8.3 Hz,$C_7$ aromatic H). IR =CH 3034; NH+/acid OH 2631, 2430; C=O 1706; C=C/$CO_2$- 1617, 1600, 1570, 1540, 1513, 1467; $CO_2$- 1359; γCH (maleate) 874; γCH 828, 765. UV

(ethanol) 213 nm (E 40,300), 226 sl. sh. (55,860), 230 (66,000), 271 sl. sh. (4,300), 281 (6,950), 291 (8,620), 302 (6,050), 309 (3,200), 318 (925), 325 (932). Mass spec. m+ for free base at m/z 225.

Exact mass Calcd. for $C_{16}H_{19}N$: 225.1517.

Found: 225.1510.

Anal. Calcd. for $C_{16}H_{19}N.C_4H_4O_4$: C, 70.36; H, 6.79; N, 4.10.

Found: C, 69.97; H, 6.75; N, 4.06.

Example 40 2,3-Dihydro-N,N-di-n-propyl-6-methylthio-1H-phenalen-2-amine.

A. Preparation of 2,3-dihydro-6-methylthio-N-(2,5-dimethylpyrrol-1-yl)-1H-phenalen-2-amine.

A mixture of the 6-bromo-N-(2,5-dimethylpyrrol-1-yl)-2,3-dihydro-1H-phenalen-2-amine from Example 38 part A (8.15 g, 0.024 mol) and sodium methanethiolate (3.55 g, 0.051 mol) in hexamethylphosphoramide (50 mls) was heated in an oil bath maintained at 100°C for 35 min. and cooled. Methyl iodide (4.5 mls, 0.018 mol) was added, and the mixture was stirred at room temperature overnight. Water was added, and the resulting solution was extracted twice with ether. The extracts were washed twice with water and once with saturated NaCl. The solution was dried ($MgSO_4$), and the solvent was removed in vacuo to leave a black oil (5.58 g). The compound was dissolved in methylene chloride, silica gel (30 g, 0.040-0.063 mm) was added, and the solvent was removed in vacuo. The resulting powder was added to a flash chromatography column that was wetted with 2.5% ethyl acetate/hexane, and the column was eluted with the same. The purest fractions were combined to give the sub-titled methylthio compound as a yellow oil (0.64 g) which crystallized on standing. The compound was crystallized from ethanol to give a brown solid (0.49 g, m.p. 122-125°C). NMR ($CDCl_3$, 300 MHz) δ2.335 (s,6,pyrrole $CH_3$; 2.563 (s,3,S-$CH_3$); 3.25-3.47 and 3.74-3.90 (m,4,N-C($CH_2$)$_2$); 4.59-4.72 (m, 1,N-CH); 5.834 (s,2,pyrrole H); 7.223, 7.249, 7.376, 7.401 (ab,J=7.65 Hz,2,$C_4$ and $C_5$ aromatic H); 7.312, 7.336 (d,J=7.02 Hz,1,$C_9$ aromatic H); 7.474, 7.478, 7.502, 7.526 (dd, J=7.02 Hz and 8.46 Hz,1,$C_8$ aromatic H); 8.204, 8.232 (d,J=8.52 Hz,1,$C_7$ aromatic H).

The remaining fractions containing the desired compound were combined to give a yellow-brown oil (3.53 g).

B. Preparation of 2,3-dihydro-6-methylthio-1H-phenalen-2-amine, and its hydrochloride salt.

A mixture of the pyrrole, from part (a) hereinabove, (3.53 g, 0.0115 mol), hydroxylamine hydrochloride (4.79 g, 0.069 mol), and sodium bicarbonate (3.86 g, 0.046 mol) in ethanol (100 mls) was stirred at reflux for 31 hours. Additional quantities of hydroxylamine hydrochloride (2.40 g, 0.034 mol) and sodium bicarbonate (3.86 g, 0.046 mol) were added at 6 and 24 hours. The solvent was removed in vacuo, and the residue was partitioned between dilute sodium hydroxide and diethylether. The aqueous layer was extracted again with ether, and the combined organics were extracted with 10% HCl (25 mls). A yellow precipitate formed which was filtered, washed with water, ether, acetonitrile, and ether and dried to give the subtitled amine salt as a light yellow-green solid (2.07 g). A sample (0.50 g) was crystallized from acetic acid, washed with ether, and dried in vacuo at 60°C to leave a light green solid (0.39 g, m.p. ~310°C dec). NMR (DMSO, 300 MHz) δ2.567 (s,3,S-$CH_3$); 3.14-3.49 (m,4,C-$C(CH_2)_2$ + $H_2O$); 3.62-3.78 (m,1,N-CH); 7.33-7.47 (m;3;$C_4$, $C_4$, and $C_9$ aromatic H); 7.520, 7.543, 7.548, 7.571 (dd,J=7.0 Hz and 8.4 Hz,1,$C_8$ aromatic H); 8.018, 8.046 (d, J=8.2 Hz,1,$C_7$ aromatic H).

C. Preparation of 2,3-dihydro-N,N-di-n-propyl-6-methylthio-1H-phenalen-2-amine.

A mixture of the primary amine from part (B) hereinabove (0.75 g, released to the free base with 15% NaOH/ether; 0.58 g, 2.53 mmol), 1-bromopropane (1.4 mls, 0.016 mol), and potassium carbonate (1.05 g, 7.6 mmol) in acetonitrile (25 mls) was stirred at reflux under nitrogen for a total of 49 hours. Additional quantities of 1-bromopropane (1.4 mls, 0.016 mol) were added after 19 hours and 42 hours. The solvent was removed in vacuo, and the residue was partitioned between ether and water. The aqueous layer was extracted again with ether, and the combined organics were washed with saturated NaCl and dried ($MgSO_4$). The solvent was removed in vacuo to leave a brown oil. Purification by gravity chromatography ($SiO_2$; 9:1 hexane/ethyl acetate, 1% Methanol, 0.1% ammonia) gave the titled methylthiophenalen-2-amine as a reddish-brown oil (U-76,779, 0.56 g, 71%). NMR ($CDCl_{13}$, TMS) δ0.90 (t,J=7 Hz,6,N-C-C-$CH_3$), 1.49 (sextet,J=7 Hz,4,N-C-$CH_3$), 2.51 (s,3,S-$CH_3$), 2.25-2.65 (m,4,N-$CH_2$), 2.8-3.2 (m,5,N-

$CH(CH_2)_2$), 7.0-7.55 (m,4,aromatic H), 8.14 (dd,J=1.2 Hz and 8 Hz,1,$C_7$ aromatic H). IR =CH 3057, 3032, 3020; C-H 2957, 2931, 2870; N-C-H 2809; C=C 1608, 1594, 1572, 1565; C=C/C-H def. 1462, 1382; C-N/βCH/-other 1107, 1074, 1059; γCH 811, 759. UV (Ethanol) 226 nm (E 37,400), 244 sh. (14,020), 305 sh. (8,870), 314 (10,080), 334 sh. (6,380). Mass spec. m+ at m/z 313.

Example 41 5-Hydroxy-2-methyl-2,3-dihydro-N,N-di-n-propyl-1H-phenalene-2-amine, and its hydrobromide.

A. Preparation of methyl 5-methoxy-2,3-dihydro-1H-phenalen-2-carboxylate.

A mixture of 5-methoxy-2,3-dihydro-1H-phenalen-2-carboxylic acid (5.00 g, 0.0206 mol), potassium carbonate (3.42 g, 0.0248 mol), and methyl iodide (6.5 mls, 2.28 g/ml, 0.10 mol) in acetone (100 mls) was stirred at reflux for 5 hours. Methyl iodide (3.2 mls, 0.050 mol) was again added, and the mixture was refluxed for 3 hours. The solvent was removed in vacuo, and the residue was partioned between water and diethylether containing a little tetrahydrofuran. The aqueous layer was extracted with ether, and the combined organics were washed with saturated NaCl and dried ($MgSO_4$). The solvent was removed in vacuo to leave a solid. Crystallization from methanol gave the sub-titled ester as off-white crystals (4.29 g, 81%; m.p. 96-98°C).

B. Preparation of methyl 5-methoxy-2-methyl-2,3-dihydro-1H-1H-phenalen-2-carboxylate.

A solution of diisopropylamine (2.3 mls, 0.722 g/ml, 0.016 mol) in tetrahydrofuran was cooled to -78°C, and n-butyllithium (10.2 mls, 1.47 N, 0.015 mol) was added dropwise over a period of 4 minutes. The mixture was stirred at -78°C for 10 minutes, and the cold bath was removed for 15 minutes. The solution was again cooled to -78°C, and a solution of the ester from part (A) above (3.5 g, 0.014 mol) in tetrahydrofuran (80 mls) was added over a period of 15 minutes. The mixture was stirred at -78°C for 30 minutes, and the cold bath was removed for 25 minutes. The anion solution was again cooled to-78°C, and methyl iodide (2.6 mls, 2.28 g/ml, 0.41 mol) was added rapidly. After 5 minutes, the mixture was removed from the cold bath, and stirred at room temperature overnight. Ether, saturated NaCl, and water were added, and the layers were separated. The

aqueous layer was extracted with ether, and the combined organics were washed with saturated NaCl and dried ($MgSO_4$). The solvent was removed in vacuo to leave the sub-titled ester a brown oil (3.79 g, 100%). A sample (0.60 g) was crystallized from methanol at -78°C to give a tan solid (0.52 g, m.p. 76-82°C).

C. Preparation of 5-methoxy-2-methyl-2,3-dihydro-1H-phenalen-2-carboxylic acid.

A mixture of the ester from part (B) above (3.00 g, 0.0111 mol), 15% sodium hydroxide (30 mls), and methanol (35 mls) was stirred at reflux for 3 hours. The methanol was removed in vacuo, and the aqueous remains was diluted with water, washed with diethylether, and acidified with conc. HCl while cooling in ice. The precipitate was filtered, washed with water, and dried in vacuo to leave a light amber solid (2.74 g, 96%). A sample (0.55 g) was crystallized from acetonitrile to give the sub-titled acid as an amber solid, m.p. 216-217.5°C.

D. Preparation of 5-methoxy-2-methyl-2,3-dihydro-1H-phenalen-2-carbonylazide.

The acid from part (C) above (2.20 g, 8.58 mmol) was suspended in acetone (40 mls) and triethylamine (1.6 mls, 0.726 g/ml, 0.011 mol) was added. The mixture was stirred for 10 minutes during which time a homogeneous solution was achieved. The mixture was cooled in ice, and a solution of ethyl chloroformate (1.07 mls, 1.135 g/ml, 0.011 mol) in acetone (20 mls) was added over a period of 5 minutes. The mixture was stirred at 0°C for 1 hour, and a solution of sodium azide (0.84 g, 0.013 mol) was added over a 1 minute period. The mixture was stirred at 0°C for 3 hours, and the solvent was concentrated in vacuo. The residue was partioned between water and ether, and the aqueous layer was extracted again with ether. The combined organics were washed with saturated NaCl and dried ($MgSO_4$). The solvent was removed in vacuo to leave a yellow oil (2.83 g). IR shows the sub-titled carbonyl azide at 2135 $cm^{-1}$ and 1710 $cm^{-1}$.

E. Preparation of benzyl 5-methoxy-2-methyl-2,3-dihydro-1H-phenalen-2-carbamate (benzyl urethane).

The acyl azide from part (D) (2.83 g, 8.58 mmol) was dissolved in toluene (40 mls), and the solution was stirred on the steam bath for 30 minutes. IR shows the isocyanate at 2250 $cm^{-1}$ and no acyl

azide. Benzyl alcohol (2.7 mls, 1.45 g/ml, 0.026 mol) was added, the mixture was stirred in an oil bath maintained at 110°C for 17 hours, and the solvent was removed in vacuo to leave a yellow oil (5.22 g). TLC (4:1 hexane/ethyl acetate) shows a mixture of benzyl alcohol and the desired urethane. Purification by flash chromatography ($SiO_2$, 0.040-0.063 mm; 15% ethyl acetate/hexane) gave two cuts of the desired band of the sub-titled carbamate ester. The purest fractions were combined to give a slightly yellow oil (1.59 g). The less pure fraction were combined to give a yellow oil (1.66 g).

F. Preparation of 5-methoxy-2-methyl-2,3-dihydro-1H-phenalen-2-amine, and its maleic acid salt (1:1).

The urethane from part (E) above (containing some benzyl alcohol, 3.12 g) was combined with acetic acid (80 mls) and 10% palladium on carbon (0.50 g), and the mixture was hydrogenated in a Parr apparatus using an initial hydrogen pressure of 50 psi for 17 hours. The product mixture was filtered through Celite™, and the catalyst was washed well with ethanol. The combined filtrate was evaporated to dryness, and the residue was partitioned between water and 10% sodium hydroxide. The aqueous solution was extracted again with ether, and the combined organics were washed with saturated NaCl and dried ($MgSO_4$). The solvent was removed in vacuo to leave the sub-titled amine as an oil (1.52 g, 78% from the acid). A sample (0.40 g) was combined with maleic acid (0.21 g) in ether, and the precipitate was filtered, washed with ether, and crystallized from methanol/ether to give the sub-titled amine salt as a colorless solid (0.47 g, m.p. 176-177°C).

G. Preparation of 5-methoxy-2-methyl-2,3-dihydro-N,N-di-n-propyl-1H-phenalen-2-amine and its hydrochloride hydrate salt (1:1:0.25).

A mixture of the primary amine from part F (1.08 g, 4.75 mmol), potassium carbonate (1.97 g, 0.014 mol), and 1-iodopropane (1.85 mls, 1.743 g/ml, 0.019 mol) in acetonitrile (30 mls) was stirred at reflux for a total of 31 hours and at room temperature for 30 hours. Additional quantitites of 1-bromopropane (.093 mls) were added at 9, 14, and 24 hours. An additional quantity of potassium carbonate (1.31 g) was added at 9 hours. The solvent was removed in vacuo, and the residue was partitioned between water and ether. The aqueous solution was extracted again with ether, and the combined organics

were washed with saturated NaCl and dried ($MgSO_4$). The solvent was removed in vacuo to leave the sub-titled N,N-di-n-propylamine as a yellow oil (1.25 g, 84%). The compound was dissolved in ether, and excess ethereal HCl was added. The gummy precipitate was triturated twice with ether and crystallized from methanol/ether to give the sub-titled amine salt as off-white crystals (1.22 g, m.p. 221.5-222.5°C).

H. Preparation of 5-hydroxy-2-methyl-2,3-dihydro-N,N-di-n-propyl-1H-phenalen-2-amine hydrobromide.

A mixture of the 5-methoxy ether amine salt from part (G) (0.65 g, 1.86 mmol) and 48% hydrobromic acid (distilled, 10 mls) was heated under and atmosphere of nitrogen in an oil bath maintained at 110°C for 10 minutes and diluted with water (5 mls). The mixture maintained at 110°C for 10 minutes and diluted with water (5 mls). The mixture was cooled in ice, and the precipitate was filtered, washed with cold water and several times with ether, and dried to give the titled amine salt as a green solid (0.55 g, 78%). Crystallization from methanol/ether gave a gray solid (m.p. 237-238°C).

<u>Example 42</u> 5-Hydroxy-2,3-dihydro-2,N,N-trimethyl-1H-phenalen-2-amine, and its hydrobromide salt hydrate (1:1:033).

A. Preparation of ethyl (5-methoxy-2-methyl-2,3-dihydro-1H-phenalen-2-yl)carbamate (urethane).

A solution of ethyl chloroformate (1.67 g, 0.0154 mol) in ether (50 mls) was added dropwise to a solution of the primary amine 5-methoxy-2-methyl-2,3-dihydro-1H-phenalen-2-amine from Example 41, part (F) (2.92 g, 0.0128 mol) and triethylamine (2.7 mls, 0.726 g/ml, 0.0193 mol) in ether (150 mls) at room temperature with stirring. The mixture was stirred at room temperature for 4 hours, water was added, and the stirring was continued for 30 minutes. The mixture was acidified with 10% HCl, and the layers were separated. The ether solution was washed with 10% NaOH, saturated NaCl, and dried ($MgSO_4$). The solvent was removed in vacuo to leave an oil (3.29 g). Purification by flash chromatography (18% ethyl acetate/hexane) gave the sub-titled carbamate ester (urethane) as a colorless oil (3.2 g, 84%).

B. Preparation of 5-methoxy-2,N-dimethyl-2,3-dihydro-1H-phenalen-2-amine, and its hydrochloride.

A solution of the urethane from part (A) above (3.10 g, 0.0104 mol) in tetrahydrofuran (100 mls) was added over a period of 5 minutes to a suspension of lithium aluminum hydride (3.00 g, 0.079 mol) in tetrahydrofuran (150 mls) at room temperature. The mixture was heated to reflux on the steam bath for 1 hour, cooled, and treated successively with water (3 mls), 15% NaOH (3 mls), and water (9 mls). The mixture filtered, and the aluminum cake was washed well with ether. The filtrate was dried ($MgSO_4$), and the solvent was removed in vacuo to leave the sub-titled amine as a light green oil (2.50 g, 100%). A sample (1.00 g) was dissolved in ether, and excess ethereal HCl was added. The precipitate was filtered, washed with ether, and crystallized from methanol/ether to give the sub-titled amine salt as a light green solid (0.96 g, m.p. 258-259°C).

C. Preparation of ethyl (5-methoxy-2,N-dimethyl-2,3-dihydro-1H-phenalen-2-yl)carbamate.

A solution of ethyl chloroformate (0.81 g, 7.46 mmol) in ether (50 mls) was added dropwise to a solution of the secondary amine from part (B) (1.50 g, 6.22 mmol) and triethylamine (1.3 mls, 0.726 g/ml, 9.32 mmol) in ether 100 mls at room temperature with stirring. The mixture was stirred at room temperature for 4 hours, water (100 mls) was added, and the stirring was continued for 15 minutes. The layers were separated, and the aqueous layer was extracted again with ether. The combined ether solution was washed saturated NaCl and dried ($MgSO_4$). The solvent was removed in vacuo to leave a green oil (1.86 g). Purification by flash chromatography ($SiO_2$, 15% ethyl acetate/-hexane gave the sub-titled carbamate ester (urethane) as a light green oil (1.69 g, 87%).

D. Preparation of 5-methoxy-2,N,N-trimethyl-2,3-dihydro-1H-phenalen-2-amine, and its hydrochloride hydrate salt (1:1:0.2).

A solution of the urethane from part (C) (1.50 g, 4.79 mmol) in tetrahydrofuran (70 mls) was added over a period of 2 minutes to a suspension of lithium aluminum hydride (1.50 g, 0.040 mol) in tetrahydrofuran (50 mls) at room temperature. The mixture stirred at room temperature for 2 hours, heated to reflux on the steam bath for 10 minutes, cooled, and treated successively with water (1.5 mls), 15% NaOH (1.5 mls), and water (4.5 mls). The mixture filtered, and the aluminum cake was washed well with ether. The filtrate was dried

($MgSO_4$), and the solvent was removed in vacuo to leave the sub-titled amine as a light green oil. The compound was dissolved in ether, and excess ethereal HCl was added. The precipitate was filtered, washed with ether, and crystallized from methanol/ether to give the sub-titled amine hydrochloride salt as a colorless solid (1.12 g, m.p. 247-248°C).

E. Preparation of 5-hydroxy-2,N,N-trimethyl-2,3-dihydro-1H-phenalen-2-amine, and its hydrobromide hydrate salt.

A mixture of the 5-methyl ether amine from part (D) (0.60 g, 2.1 mmol) and 48% hydrobromic acid (distilled, 9 mls) was heated in an oil bath maintained at 110-115°C for 9 minutes. The mixture was diluted with water and cooled in ice. Filtration removed a dark precipitate, and crystallization began. The crystals were filtered, washed with water and ether, and dried in vacuo to leave the titled 5-hydroxy-amine hydrobromide salt as a light green solid (0.45 g, m.p. 195-196°C).

<u>Example 43</u> 2-Hydroxy-2,3-dihydro-2,N-dimethyl-1H-phenalen-2-amine, and its hydrobromide.

A mixture of the amine hydrochloride from Examples 42, part (B) (0.50 g, 1.80 mmol) and 48% hydrobromic acid (8 mls) was heated in an oil bath maintained at 110-115°C for 12 minutes. The mixture was cooled in ice and diluted in half with water. The crystals were filtered, washed with ether, and dried. The filtrate was evaporated to dryness, and the residue was combined with the crystals. Crystallization from methanol/ether gave the titled 5-hydroxy-2-amine hydrobromide salt as a light purple solid (0.44 g, 79%, m.p. 277-278°C).

STRUCTURAL FORMULAE

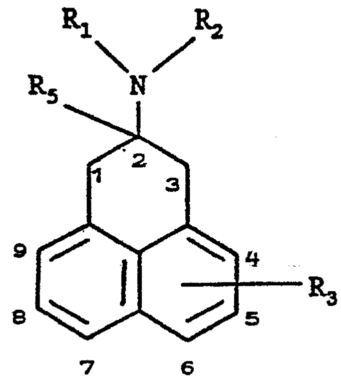

(I)

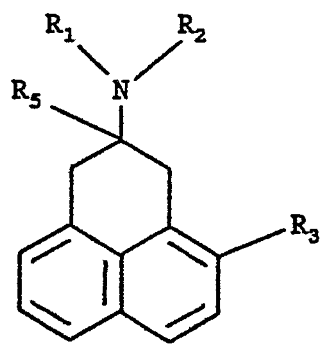

(II)

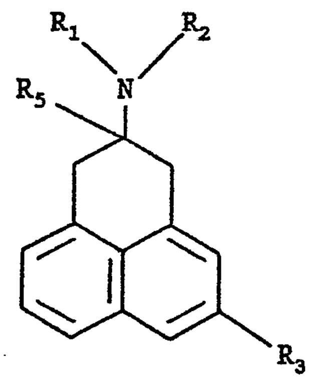

(III)

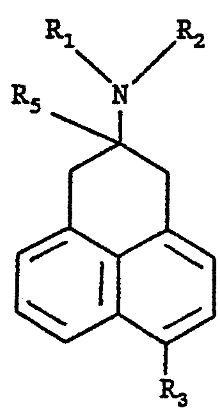

(IV)

STRUCTURAL FORMULAE (continued)

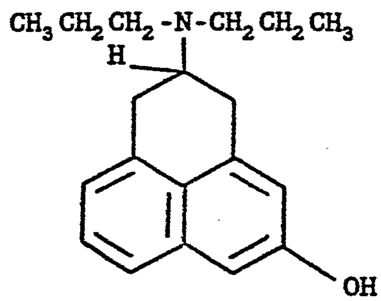

(V)

PROCESS CHART A

(Diels-Alder Route)

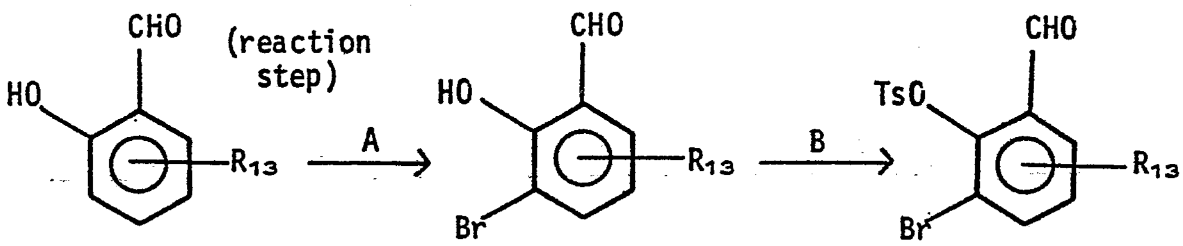


A-1 A-2 A-3

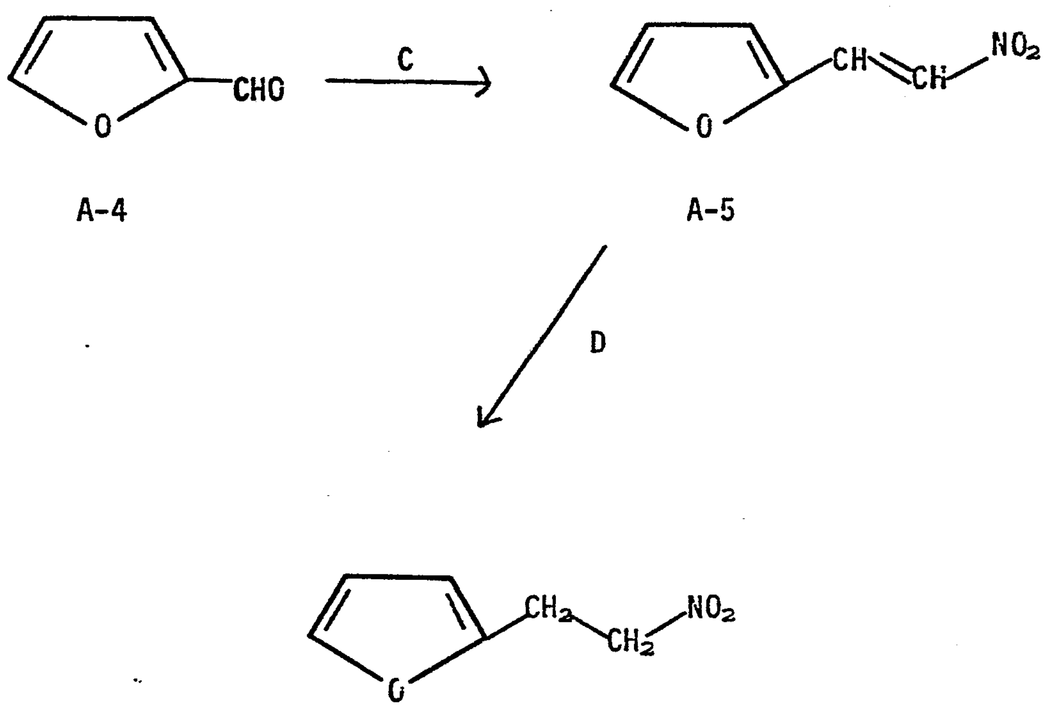


A-4 A-5

A-6

PROCESS CHART A (continued)

A-3 + A-6

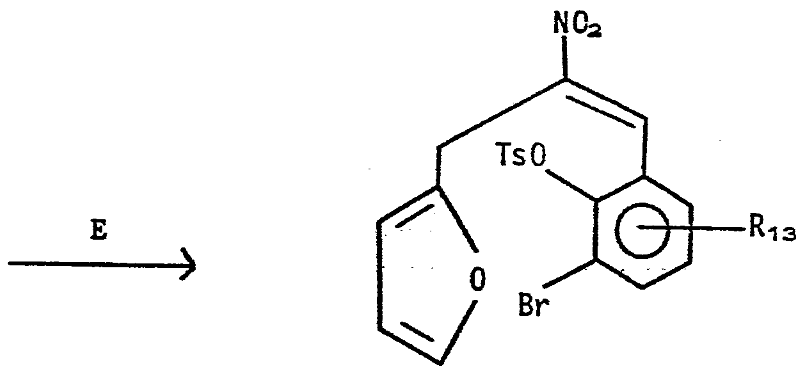


A-7

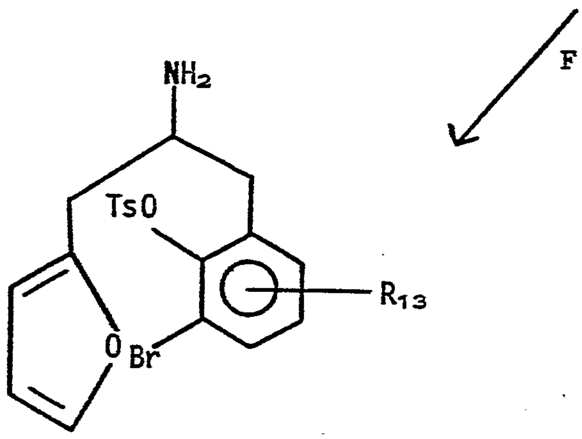


A-8

[Resolvable into its (+) and (-) isomers]

A-8

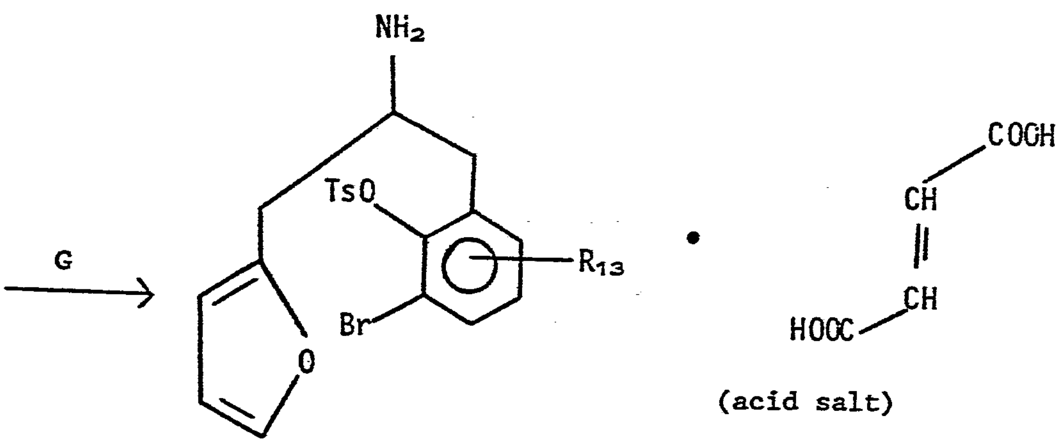


(acid salt)

A-9

(+) and (-) steero forms

PROCESS CHART A (continued)

(+) A-9
and/or
(-) A-9

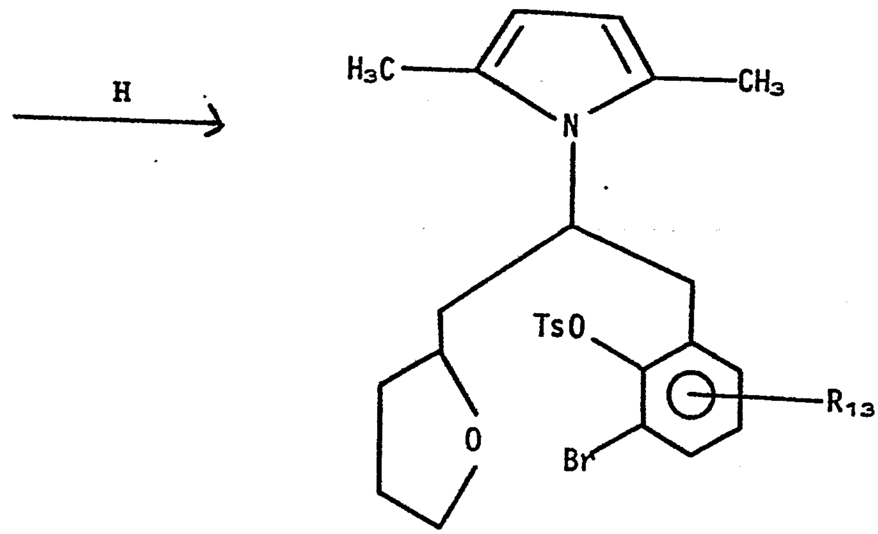


(+) or (-) A-10

A-10

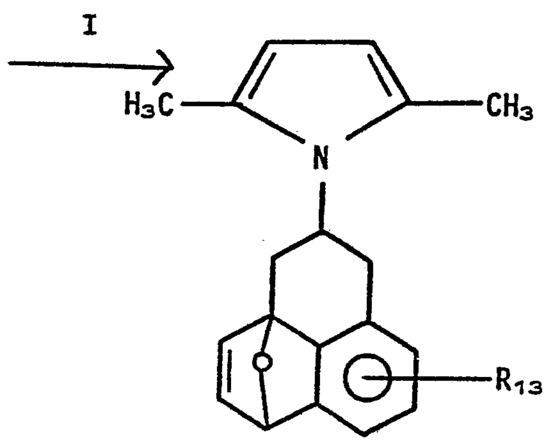


A-11

A-11

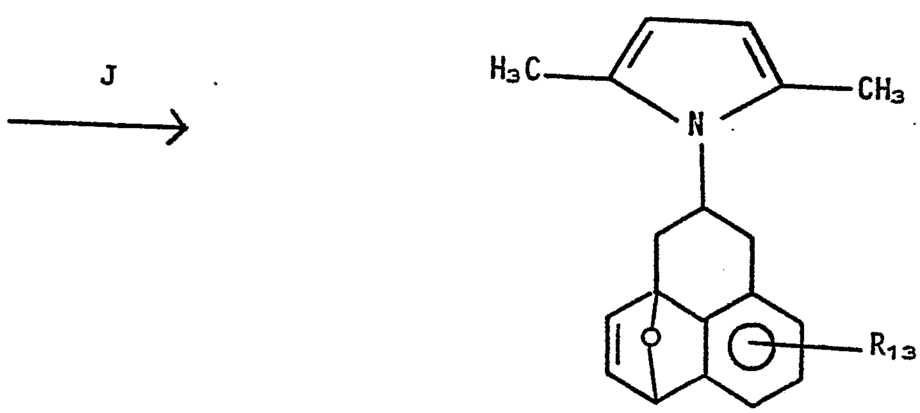


A-12

PROCESS CHART A (continued)

A-12 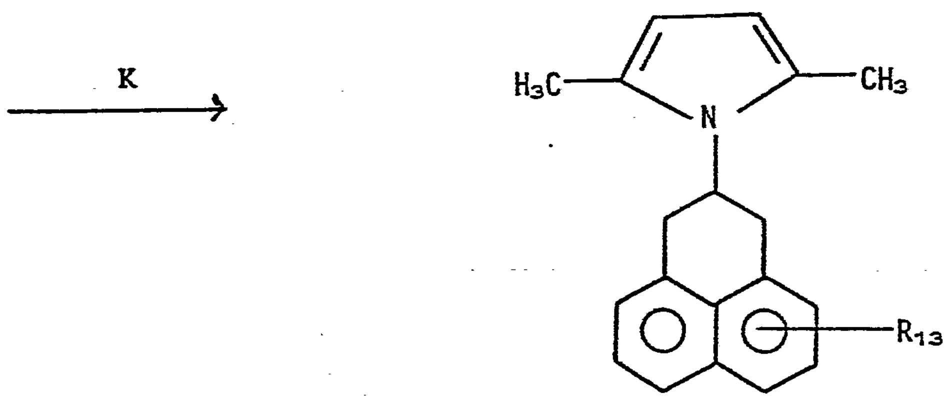


A-13

A-13 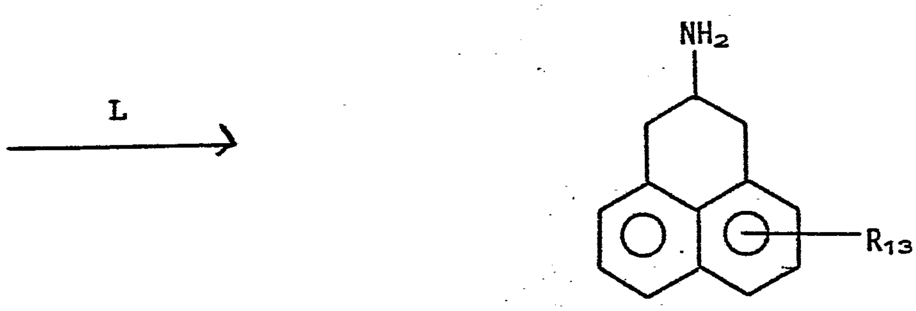


A-14

A-14 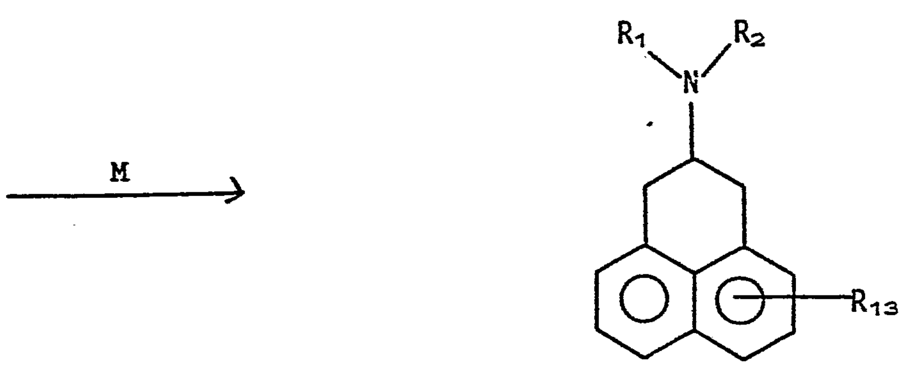


A-15

PROCESS CHART A (continued)

A-15

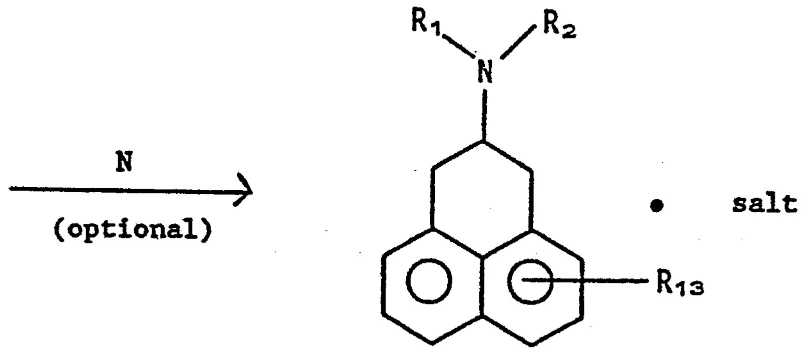


A-16

PROCESS CHART B
(from Naphthalenes)

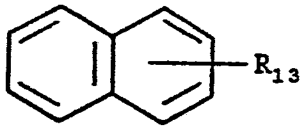

+

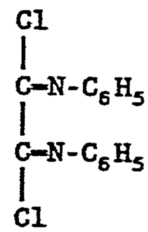

+ $AlCl_3$

B-1

B-2

B-3

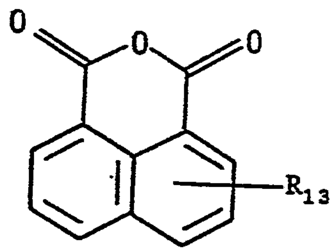

B-4

B-4

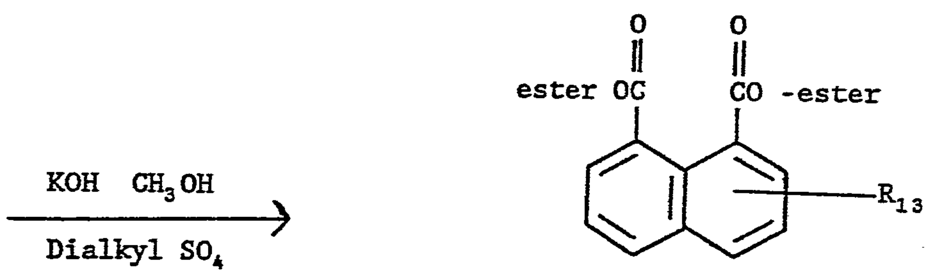


B-5

PROCESS CHART B (continued)

B-5

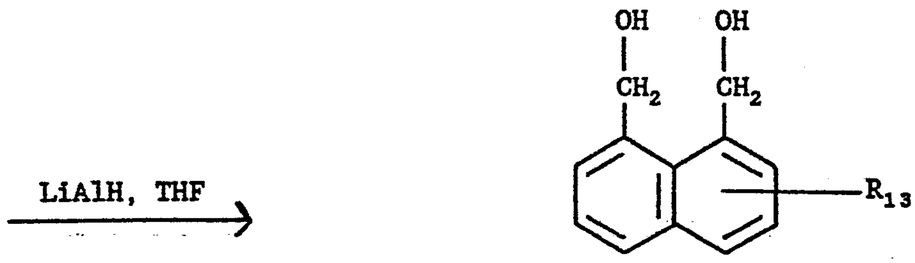


B-6

B-6

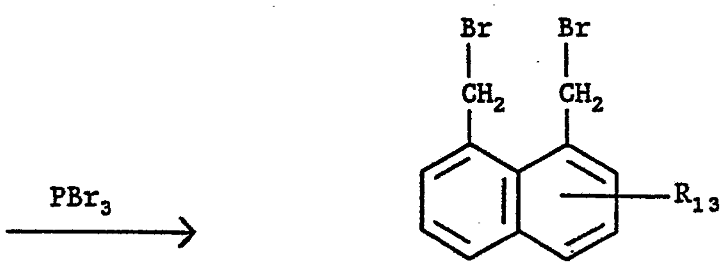


B-7

B-7

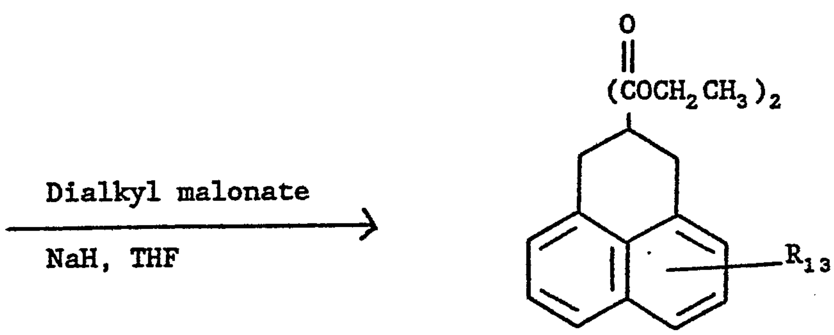


B-8

+

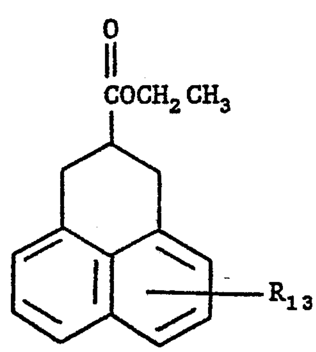

B-9

PROCESS CHART B (continued)

B-8/B-9

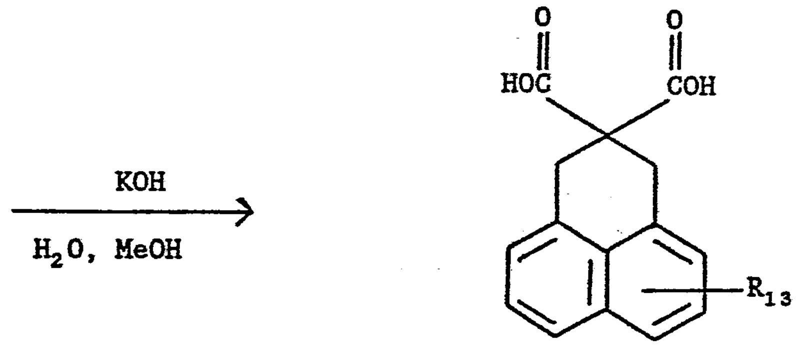


B-10

B-10

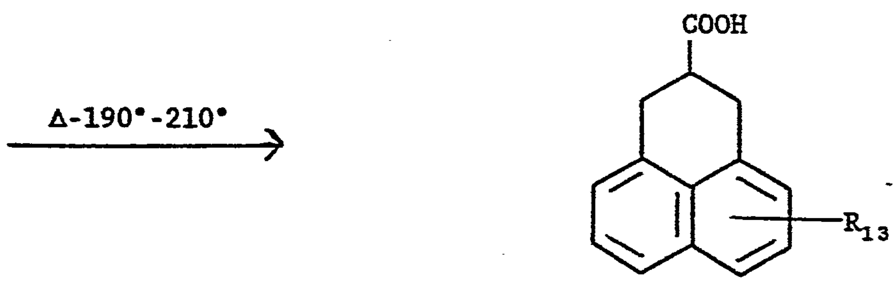


B-11

B-11

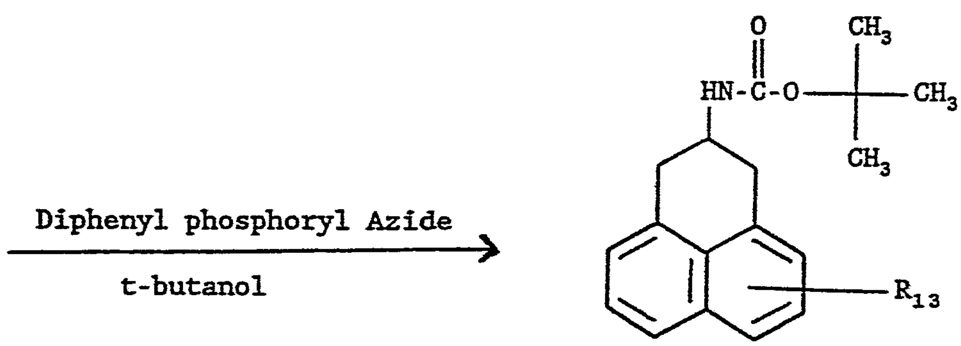


+

HN-dimer(urea) thereof

B-12

PROCESS CHART B (continued)

B-12

Trifluoroacetic Acid

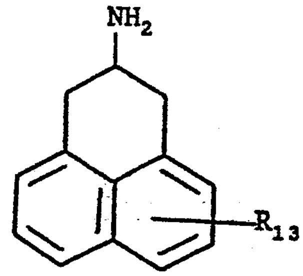

B-13

B-13

(optional)
1) HCHO, $NaBH_4$ or
2) $R_1X$, $R_2X$, or
3) $X-R_1R_2-X$

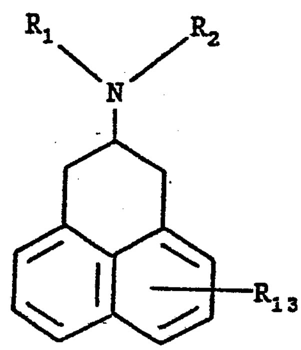

B-14

B-14

Selected Acid
(optional)

B-14 • Acid Salt

CHART C
(for Example 38)

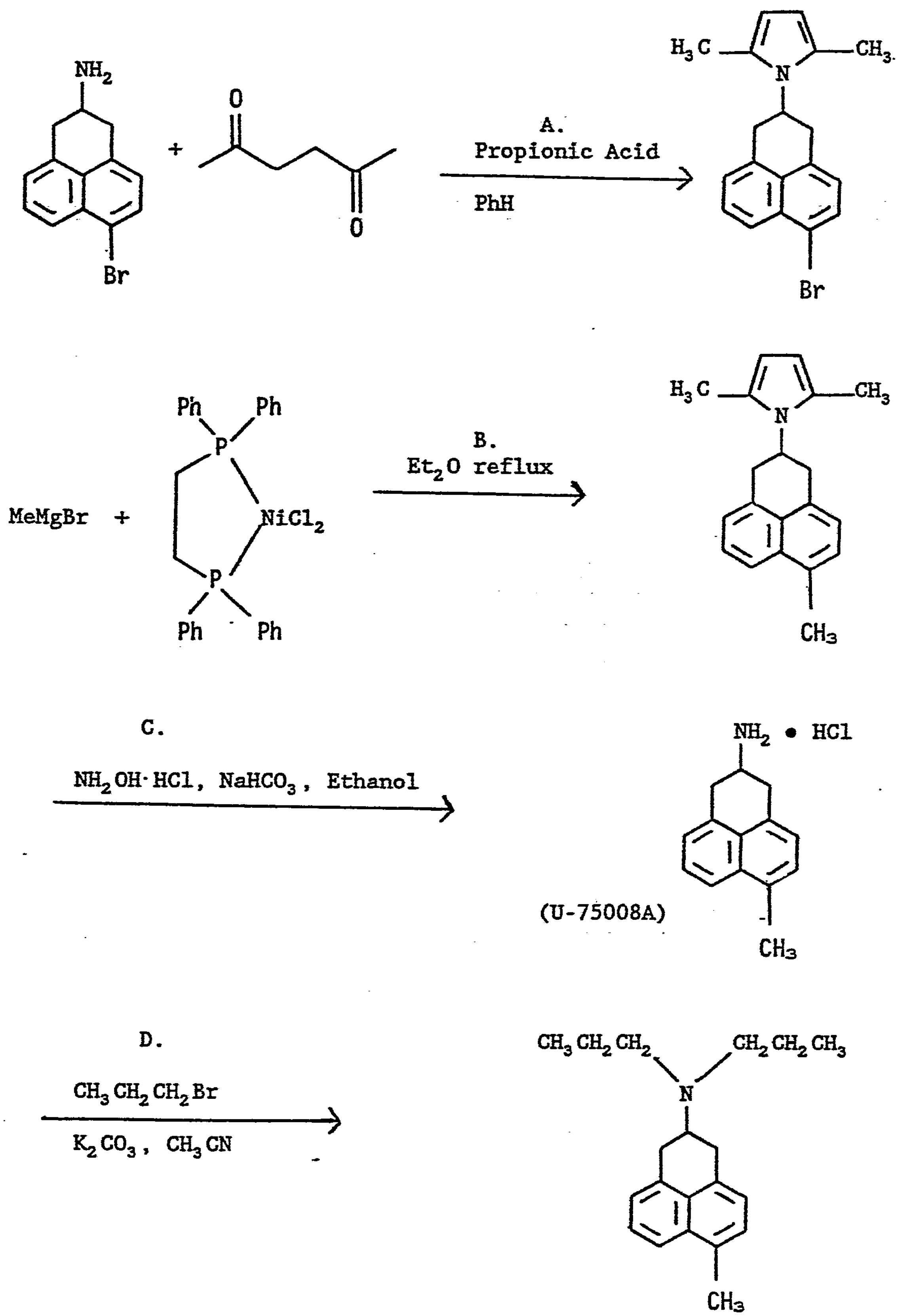


(U-75008A)

CHART D
(for Example 39)

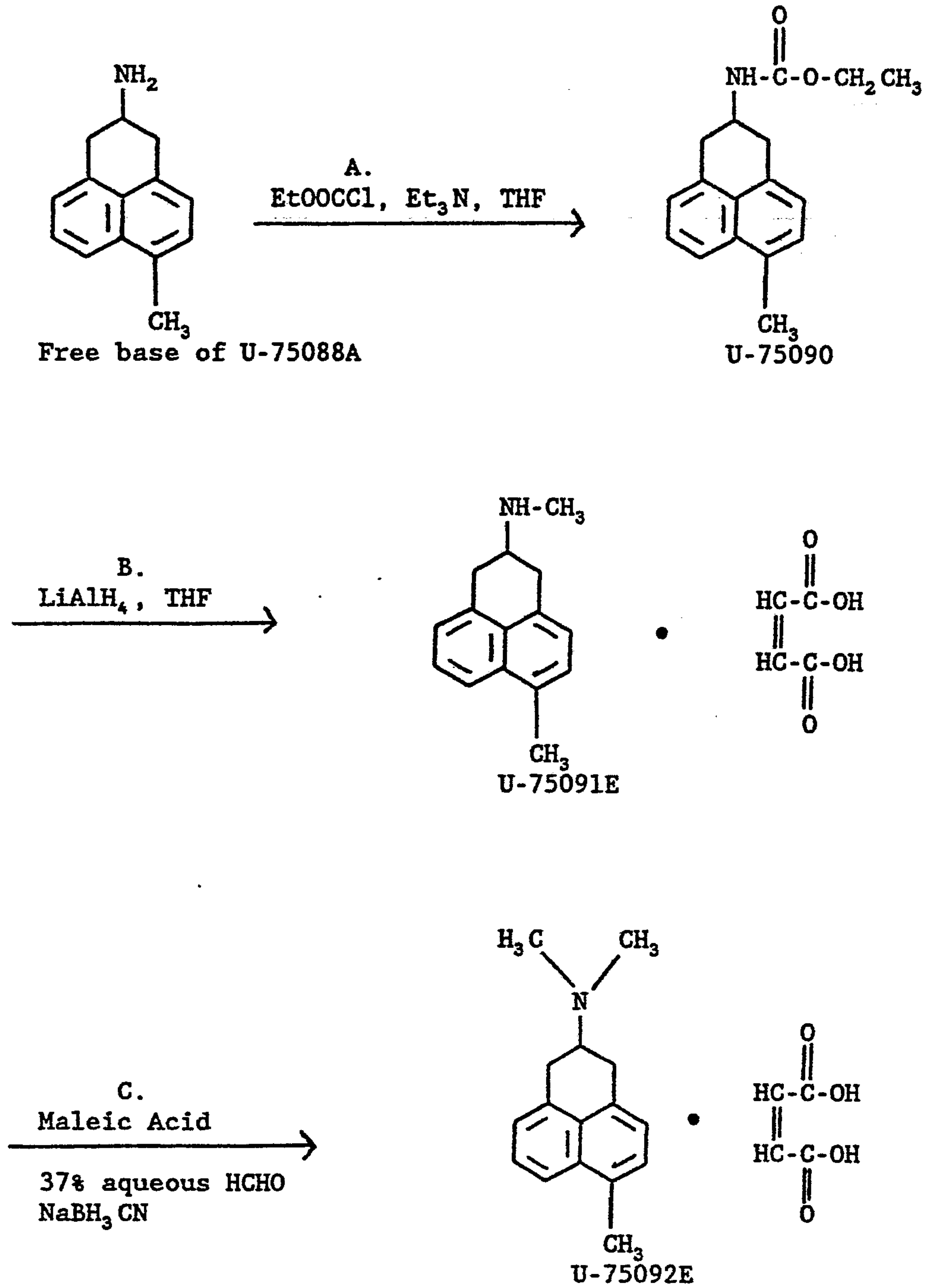


Free base of U-75088A

U-75090

U-75091E

U-75092E

CHART E
(for Example 40)

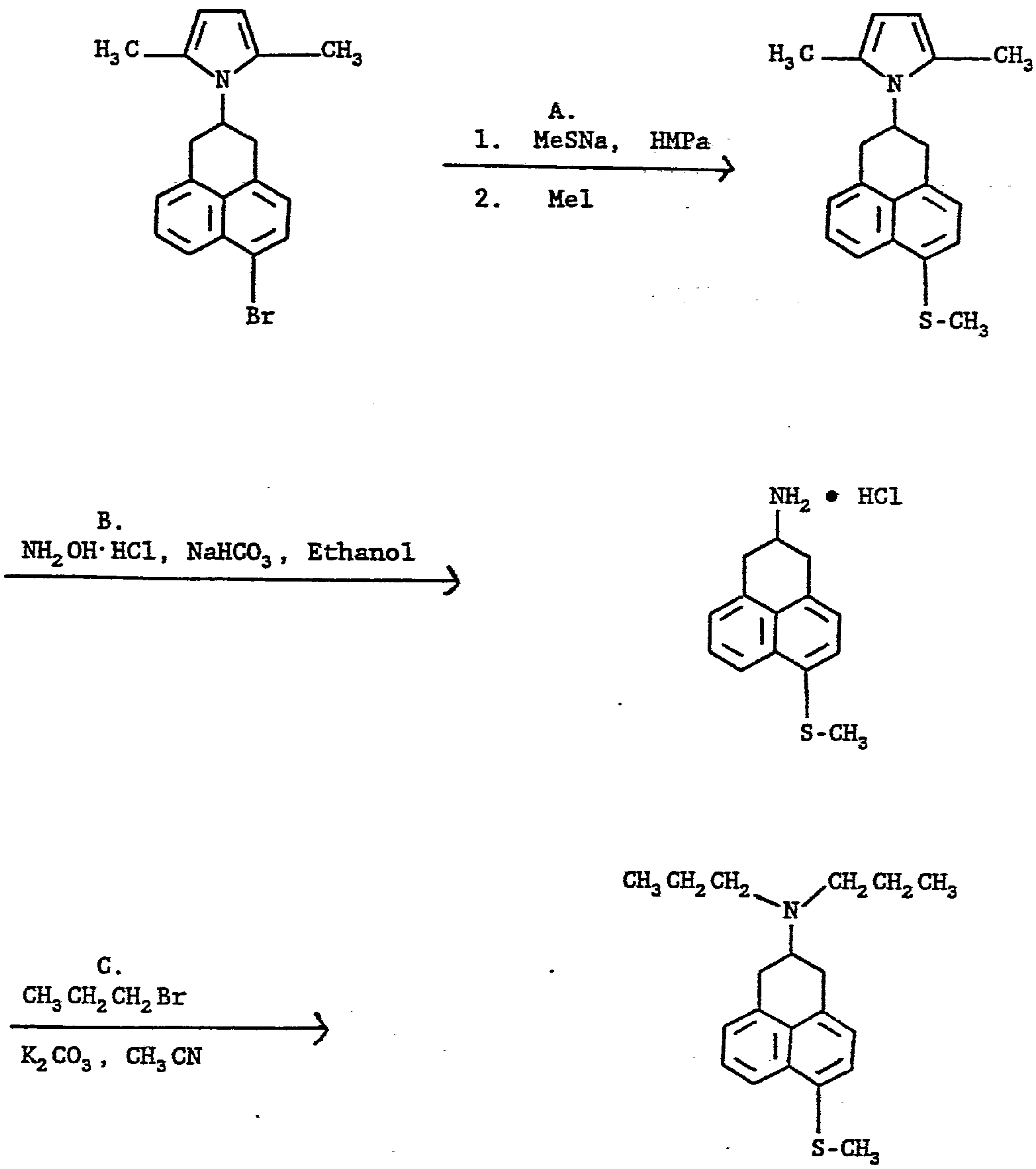

CHART F
(for Example 41)

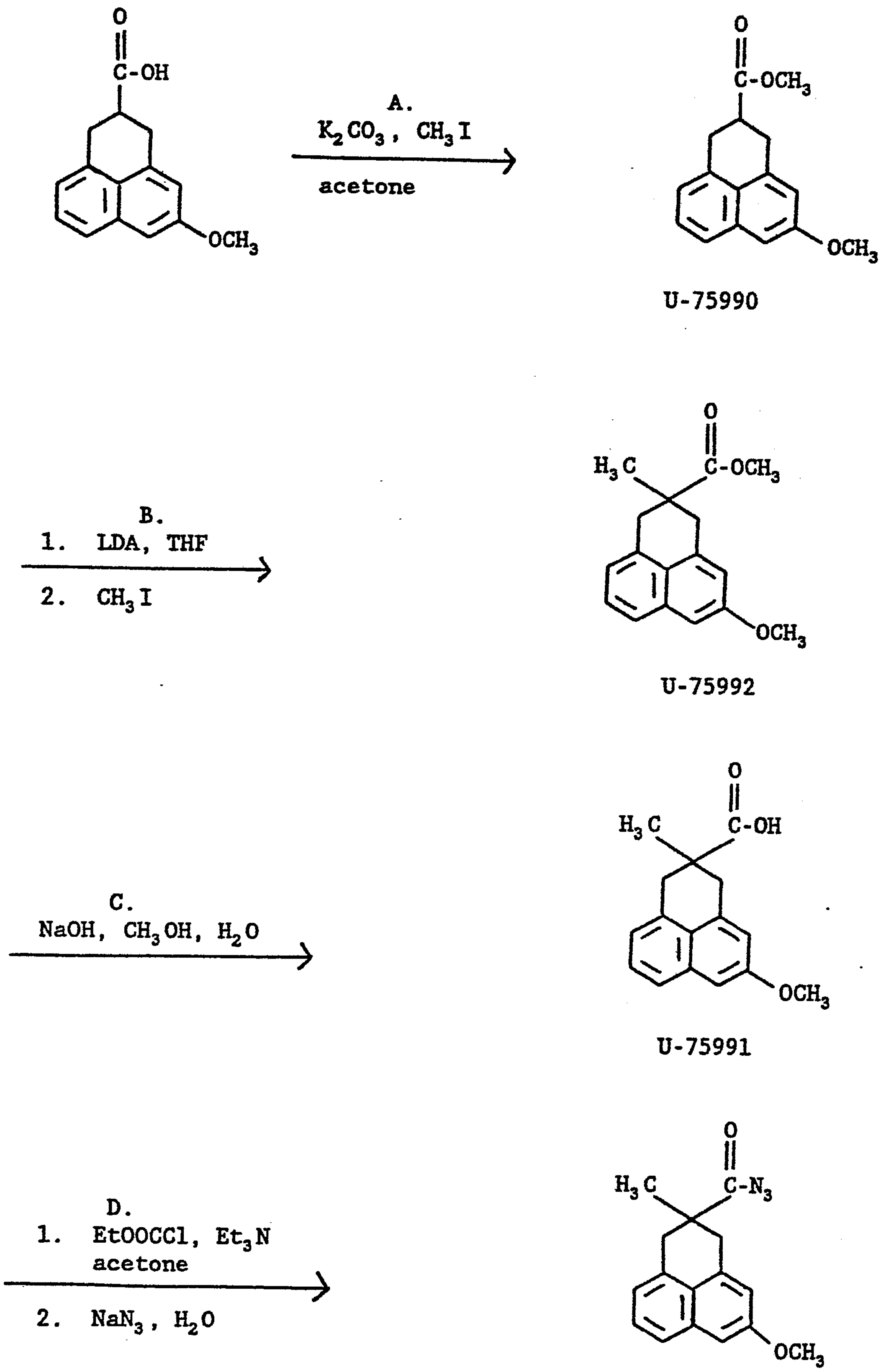


U-75990

U-75992

U-75991

CHART F (continued)

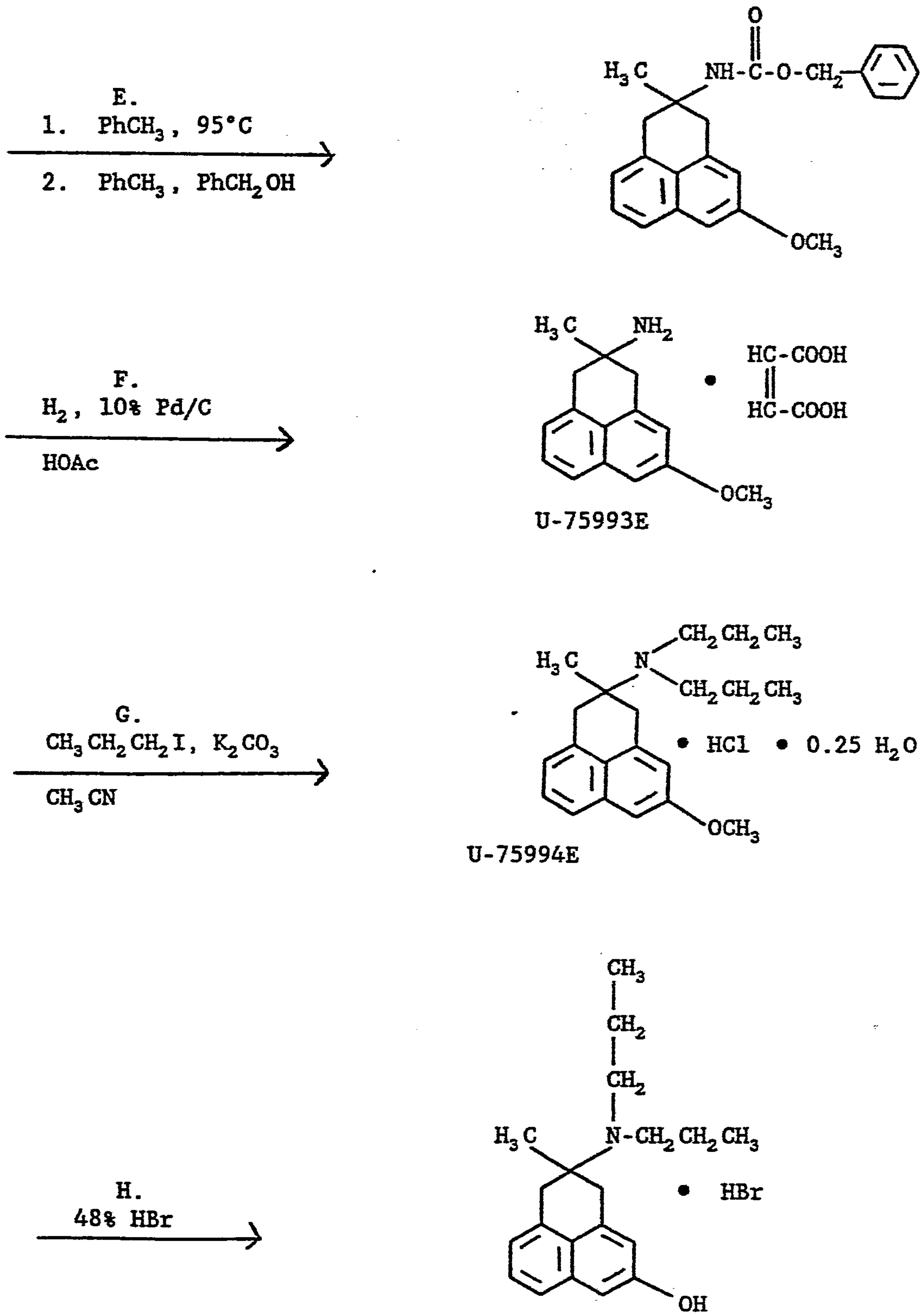


U-75993E

U-75994E

U-75995B

CHART G
(for Example 42)

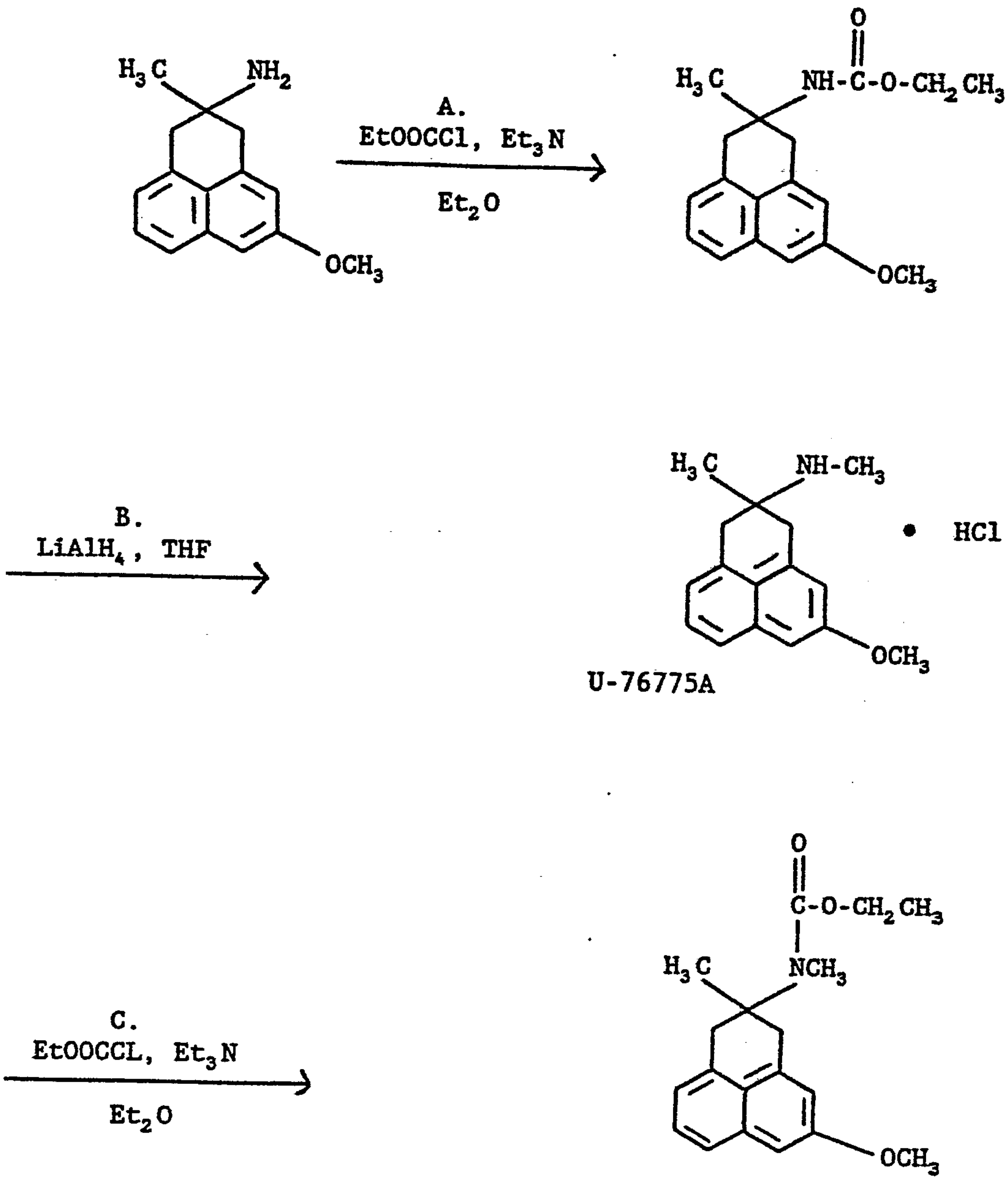


U-76775A

CHART G (continued)

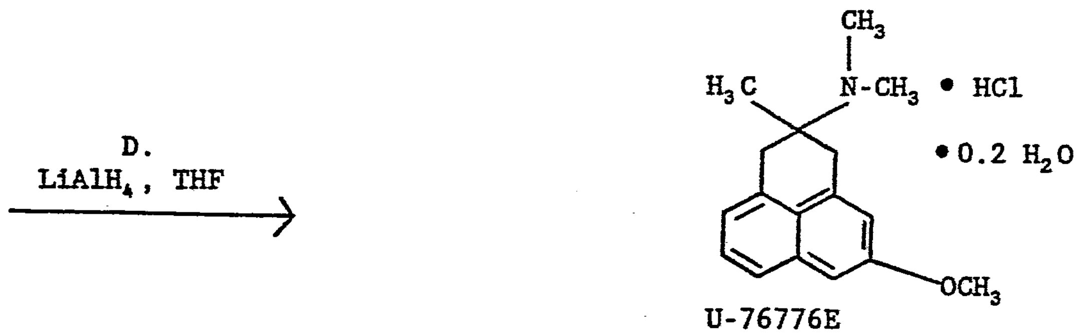


U-76776E

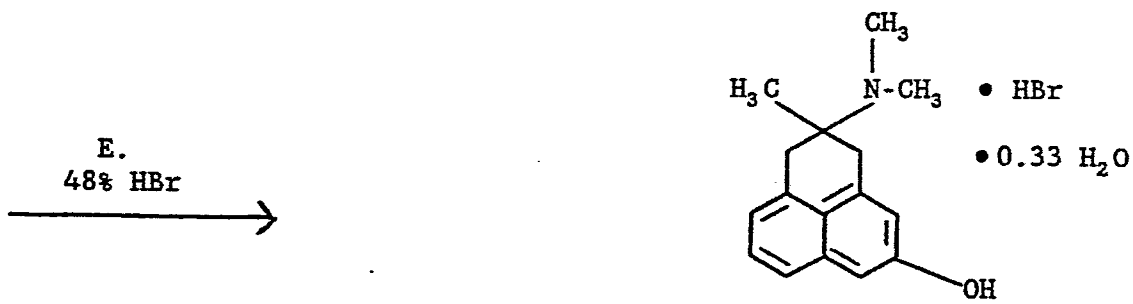


U-76778E

TABLE I

HYPOTHERMIA AND ANTIPSYCHOTIC TESTS

| | ED$_{50}$, mg/kg | | |
|---|---|---|---|
| Example No. | Hypothermia | Apomor. Antag. | Dopamine Binding Assay % Decrease from Control |
| 38, part C | >50 | 35 | -30.62 |
| 38, title compound | 18 | >50 | -82.08 |
| 39, part B | 13 | 35 | -55.37 |
| 39, part C | 5.3 | 21 | -42.52<br>-68.87 |
| 40 | 25 | 12.5 | No Data |
| 41 | >50 | >50 | -55.18 |

TABLE 2

EFFECTS OF U-64,273a ON DOPAMINE NEURONS

A. ANTAGONIZE AMPHETAMINE DEPRESSIONS

| | MAXIMUM EFFECT (per cent reversal) | ED50 (μg/kg) |
|---|---|---|
| Haloperidol | 164% | 8 |
| Clozapine | 105% | 2000 |
| U-64,273A | 69% | 5 |
| TDHL | 57% | 132 |

B. DEPRESS DA NEURON FIRING RATES

| | MAXIMUM EFFECT (per cent decrease | ED50 (μg/kg) |
|---|---|---|
| Apomorphine | 100% | 10 |
| (-)PPP | 68% | 137 |
| TDHL | 53% | 21 |
| U-64,273A | 30% | 70 |

Table 2. Dopamine neuron firing rates were recorded according to published procedures and drugs were injected intravenously in chloral hydrate anesthetized rats. A. Ability of drugs to reverse the depression in firing rates of dopamine neurons by 1 to 2 mg/kg amphetamine. The maximum effect is the maximum amount of the amphetamine effect that was reversed, even when doses were increased beyond dose causing maximum effect. An effect greater than 100% indicates excitation of the neuron beyond its' control rate whereas an effect less than 100% indicates partial agonist effects. B. Depression of dopamine neurons by full agonist apomorphine and partial agonists TDHL, (-)PPP and U-64,273A. Maximum effect is maximum depression that could be realized even after increasing doses beyong the dose causing maximum effect. For both A and B, the ED50 is the dose causing 50% of the respective antagonistic (A) or agonist (B) effect.

TABLE 3

BEHAVIORAL EFFECTS OF U-64,273A

| | HALOPERIDOL | CLOZAPINE | APOMORHINE | U-64273A |
|---|---|---|---|---|
| A. RAT | | | | |
| Conditioned Avoidance | .03 | 3 | 10 (p) | 30 (p) |
| Catalepsy | 1 | i.a. | i.a. | i.a. |
| Turning after 6-OH DOPA | | | 0.1 | 30 |
| B. MONKEY | | | | |
| Apomorphine Discrimination | | | 0.03 | 1.0 (p) |
| Disrupt Time Estimation | .01 | | 0.1 | 0.1,0.3 |
| C. ANTAGONIZE AMPHETAMINE | | | | |
| Mouse Toxicity | .7 | 2 | | >50 |
| 6-OH DA Turning, rat | | 30 | i.a. | 3 |
| RAT locomotor activity | | | | i.a. |
| D. ANTAGONIZE APOMORPHINE | | | | |
| Cage Climbing Mouse | 0.1 | 6 | | 5 |
| Rat Stereotypy | 0.3 | | | i.a. |
| Monkey Discrimination | 0.01 | >30 | | 0.3 (p) |
| Dog Emesis | 0.02 | >3 | | 2.3 |
| Locomotor activity, reserpinized mouse, 3 mg/kg | 85% (CPZ) | 52% | | 47% antag. |
| Locomotor activity, rat | | | | i.a. |

Table 3. Active doses (mg/kg) of haloperidol, clozapine, apomorphine and U-64,273A on various behavioral endpoints, the conditions of the experiments being identical for each of the drugs. (p) indicates partial activity. i.a. indicates that the compound is inactive on the indicated test. CPZ indicates that the data is for chlorpromazine, not haloperidol. A. Behavioral effects in the rat. B. Behavioral effects in the monkey. C. Doses required to antagonize the indicated behavioral effects of amphetamine. D. Doses required to antagonize the indicated behavioral effects of apomorphine.

TABLE 4

EFFECTS OF U-64,273a ON NEUROCHEMICAL ENDPOINTS

| TEST | CHLORPROMAZINE | CLOZAPINE | U-64,273A |
|---|---|---|---|
| A. Dopamine metabolites | | | |
| HVA | 225% | 118% | 135% |
| DOPAC | 375% | 110% | 80% |
| 3MT | 365% | 104% | 50% |
| B. Plasma Prolactin | | | |
| B. Plasma Prolactin | 1057% | 42% | 13% 1% (10) |
| C. Serotonin Metabolites | HALOPERIDOL | | |
| 5HTP | 92% | 89% | 37% |
| 5HIAA | 115% | 117% | 76% |
| 5HT | 103% | 111% | 128% |

Table 4. The endpoints measure dopamine metabolites HVA, DOPAC, and 3MT as a percentage of control (A) or plasma prolactin (B) following 3 mg/kg of chlorpromazine, or 30 mg/kg clozapine or U-64,273A in the Sprague-Dawley rat. C. Serotonin metabolites as a percentage of control following 1 mg/kg haloperidol, 10 mg/kg of clozapine and 30 mg/kg U-64,273A.

TABLE 5

BINDING PROPERTIES OF U-64,273A

| | CHLORPROMAZINE | CLOZAPINE | | U-64,273A |
|---|---|---|---|---|
| A. Binding, 1μM | | | | |
| spiperone | -81.6 | -81.6 | | -46.2 |
| muscarinic | -96.4 | -96.4 | | -11.7 |
| alpha-1 | -98.6 | -98.6 | | i.a. |
| alpha-2 | -83.4 | -83.4 | | -37.5 |
| LSD | -76.8 | -76.8 | | -67.3 |
| B. Mouse | | | | |
| In Vivo Spiperone, (%C) | 34% | 38% | 71% | 39% |

Table 5. A. The percentage of ligand displaced (- indicates ligand was displaced) from rat brain homogenates in the presence of 1 μM chlorpromazine, clozapine, or U-64,273A. B. The percentage of radioactive spiperone remaining bound to brain homogenates obtained from animals previously injected with chlorpromazine, 5 mg/kg clozapine, 15 mg/kg or U-64,273A, 50 mg/kg.

REFERENCES

1. Bunney, B.S. and Aghajanian, G.K., "Antipsychotic drugs and central dopaminergic neurons: A model for predicting therapeutic efficacy and n incidence of extrapyramidal side effects". In PREDICTABILITY IN PSYCHOPHARMACOLOGY: PRECLINICAL AND CLINICAL CORRELATIONS. A. Subilovsky, S. Gershon and B. Beer, Eds., Raven Press, N.Y. 1975, pp 225-245.

2. Welch, J.J., Kim, H.S., and Liebman, J. Amphetamine-induced increases in dopaminergic single cell firing rate after haloperidol pretreatment. Correlation with extrapyramidal side effects. Neurophramacol. 19:371-377, 1980.

3. Tamminga, C.A., "Atypical neuroleptics and novel antisychotic drugs". In NEUROLEPTICS: NEUROCHEMICAL, BEHAVIORAL AND CLINICAL PERSPECTIVES, (J.T. Coyle and S.J. Enna, Eds.) Raven Press, N.Y., 1983.

4. Kehr, W., Wachtel, H., and Schneider, H.H., Dopaminergic and intidopaminergic properties of ergolines structurally related to lisuride. Acta Pharmac. Suec. Suppl. 2:98-110, 1984.

5. Clark, D., Hrorth, S. and Carlsson, A. Dopamine-receptor agonists: Mechanisms underlying autoreceptor selectivity. I: Review of the evidence. J. Neural Transmission 62:171-207.

CLAIMS

1. A method for treating psychoses in a human or valuable, warm blooded animal patient which comprises administering to such psychotic patient an anti-psychotic dose of a 2-amino-3,4-dihydro-1H-phenalene compound of the formula

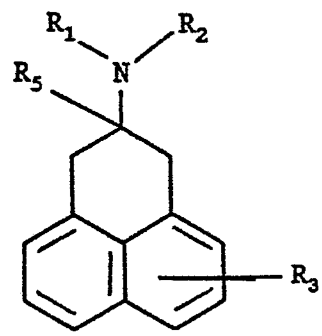

(I)

where

$R_1$ and $R_2$ are independently selected from the group consisting of hydrogen, $C_1$ to $C_4$-alkyl, $C_2$ to $C_4$-alkenyl, $C_3$ to $C_6$-cycloalkyl, or

$R_1$ and $R_2$ are taken together with the nitrogen to which they are bonded to complete a nitrogen containing ring selected from the group consisting of 1-azetidinyl, 1-pyrrolidinyl, 1-piperidinyl and 4-morpholinyl;

$R_3$ is selected from the group consisting of

hydrogen,

$C_1$ to $C_4$-alkyl,

$C_1$ to $C_4$-alkyloxy,

-trifluoromethyl,

-fluorine, chlorine or bromine,

-hydroxy,

-S(O)m-$C_1$ to $C_4$-alkyl, - $C_1$ to $C_4$-alkyl,

-$C_1$ to 9-alkanoyloxy,

$C_1$ to $C_4$-alkyloxy-C(O)-O-(i.e., $C_1$ to $C_4$-alkyl-O-C(O)-O-);

2-($C_1$ to $C_4$-alkyl)-1,3-dioxolan-2-yl-,

hydroxy-$C_1$ to $C_4$-alkyl,

$C_1$ to $C_5$-alkanoyl,

$R_4C(O)O$-,

$R_4CH_2C(O)O$-,

$R_4CH_2O$-,

$R_4C(O)$-,

$R_4$ is unsubstituted phenyl, or phenyl substituted with 1 to 3 substituents selected from the group consisting of

$C_1$ to $C_4$-alkyl,

$C_1$ to $C_4$-alkyloxy,

$C_1$ to $C_4$-alkyl-S-,

fluorine, chlorine or bromine,

$R_5$ is hydrogen or methyl;

n is zero to 2;

m is zero to 2;

in its racemic or optically active form;

or a pharmaceutically acceptable acid addition salt thereof.

2. A method according to claim 1 wherein the 2-amino-2,3-dihydro-1H-phenalene compound is one wherein

$R_1$ and $R_2$ are each $C_1$ to $C_4$-alkyl;

$R_3$ is $C_1$ to $C_4$-alkyloxy or hydroxy

or a pharmaceutically acceptable acid addition salt thereof.

3. A method according to claim 2 wherein the 2-amino-2,3-dihydro-1H-phenalene compound is 2-(N,N-di-n-propylamino)-4-methoxy-2,3-dihydro-1H-phenalene, or a pharmaceutically acceptable salt thereof.

4. A method according to claim 2 wherein the compound is a stereoisomer mixture of (±)-2,3-dihydro-2-(N,N-di-n-propylamino)-1H-phenalen-5-ol, or a pharmaceutically acceptable salt thereof.

5. A method according to claim 4 wherein the compound is (+)-2,3-dihydro-2-(N,N-di-n-propylamino)-1H-phenalen-5-ol, or a pharmaceutically acceptable salt thereof.

6. A method according to claim 4 wherein the compound in the (-)-2,3-dihydro-2-(N,N-di-n-propylamino)-1H-phenalen-5-ol, or a pharmaceutically acceptable salt thereof.

7. A method according to claim 5 wherein the 2-amino-2,3-dihydro-1H-phenalene is (+)-2,3-dihydro-2-(N,N-di-n-propylamino)-1H-phenalen-5-ol hydrochloride.

8. A method according to claim 5 wherein the 2-amino-2,3-dihydro-1H-phenalene is (+)-2,3-dihydro-2-(N,N-di-n-propylamino)-1H-phenalen-5-ol hydrobromide.

9. A method according to Claim 1 wherein the 2-amino-2,3-dihydro-1H-phenalene is 2,3-dihydro-N-monomethyl-1H-phenalen-2-amine, or a pharmaceutically acceptable salt thereof.

10. A compound of the formula

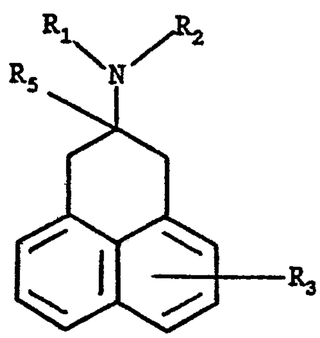

(I)

wherein $R_1$ and $R_2$ are independently selected from the group consisting of hydrogen, $C_1$ to $C_4$-alkyl, $C_2$ to $C_4$-alkenyl, $C_3$ to $C_6$-cycloalkyl, or

$R_1$ and $R_2$ are taken together with the nitrogen to which they are bonded to complete a 1-azetidinyl, 1-pyrrolidinyl, 1-piperidinyl or 4-morpholinyl ring;

$R_3$ is selected from the group consisting of

hydrogen,

$C_1$ to $C_4$-alkyl,

$C_1$ to $C_4$-alkyloxy,

trifluoromethyl,

fluorine, chlorine, bromine,

hydroxy,

$-S(O)_m-C_1$ to $C_4$-alkyl,

$C_1$ to $C_9$-alkanoyloxy,

$C_1$ to $C_4$-alkyloxy-C(O)-,

$C_1$ to $C_4$-alkyloxy-C(O)-O- (i.e., $C_1$ to $C_4$-alkyl-C(O)-),

2-($C_1$ to $C_4$-alkyl)-1,3-dioxolan-2-yl-,

hydroxy-$C_1$ to $C_4$-alkyl,

$C_1$ to $C_5$-alkanoyl,

$R_4C(O)O$-,

$R_4CH_2C(O)O$-,

$R_4CH_2O$-,

$R_4C(O)$-,

$R_4$ is unsubstituted phenyl or phenyl substituted with 1 to 3 substituents selected from the group consisting of

(a) $C_1$ to $C_4$-alkyl,

(b) $C_1$ to $C_4$-alkyloxy,

(c) $C_1$ to $C_4$-alkylthio,

(d) fluorine, chlorine or bromine,

$R_5$ is hydrogen or methyl;

n is zero to 2;

m is zero to 2;

in its racemic or optically active form;
or acid addition salt thereof, except

4-methyl-2-monoethylamino-2,3-dihydro-1H-phenalene

2,3-dihydro-N-methyl-1H-phenalen-2-amine,

2,3-dihydro-N-dimethyl-1H-phenalen-2-amine,

1-[2,3-dihydro-2-(dimethylamino)-1H-phenalen-6-yl]ethanone,

or an acid addition salt thereof.

11. A compound according to claim 9 wherein

$R_1$ and $R_2$ are each $C_1$ to $C_4$-alkyl, and

$R_3$ is hydroxy in the 5-position, or a pharmaceutically acceptable salt thereof.

12. A compound according to claim 10 which is 2-(N,N-di-n-propylamino)-2,3-dihydro-1H-phenalen-5-ol or a pharmaceutically acceptable salt thereof.

13. A compound according to claim 11 which is (+)-2-(N,N-di-n-propylamino)-2,3-dihydro-1H-phenalen-5-ol, or a pharmaceutically acceptable salt thereof.

14. A compound according to claim 11 which is (-)-2-(di-n-propyl-

amino)-2,3-dihydro-1H-phenalen-5-ol, or a pharmaceutically acceptable salt thereof.

15. A compound according to claim 9 wherein $R_1$ and $R_2$ are each $C_1$ to $C_4$-alkyl, and

$R_3$ is $C_1$ to $C_4$-alkyloxy in the 5-position or a pharmaceutically acceptable salt thereof.

16. A compound according to claim 14 which is N-di-n-propyl-5-methoxy-2,3-dihydro-1H-phenalen-2-amine, or a pharmaceutically acceptable salt thereof.

17. A pharmaceutical composition, useful in dosage unit forms comprising a compound of the formula

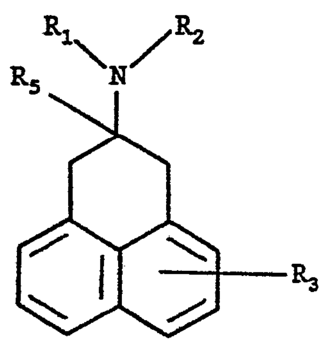

(I)

where

$R_1$ and $R_2$ are independently selected from the group consisting of hydrogen, $C_1$ to $C_4$-alkyl, $C_2$ to $C_4$-alkenyl, $C_3$ to $C_6$-cycloalkyl, or

$R_1$ and $R_2$ are taken together with the nitrogen to which they are bonded to complete a nitrogen containing ring selected from the group consisting of 1-azetidinyl, 1-pyrrolidinyl, 1-piperidinyl and 4-morpholinyl;

$R_3$ is selected from the group consisting of

hydrogen,

$C_1$ to $C_4$-alkyl,

$C_1$ to $C_4$-alkyloxy,

-trifluoromethyl,

-fluorine, chlorine or bromine,

-hydroxy,

-S(O)m-$C_1$ to $C_4$-alkyl, - $C_1$ to $C_4$-alkyl,

$-C_1$ to 9-alkanoyloxy,

$C_1$ to $C_4$-alkyloxy-C(O)-O-(i.e., $C_1$ to $C_4$-alkyl-O-C(O)-O-);

2-($C_1$ to $C_4$-alkyl)-1,3-dioxolan-2-yl-,

hydroxy-$C_1$ to $C_4$-alkyl,

$C_1$ to $C_5$-alkanoyl,

$R_4C(O)O-$,

$R_4CH_2C(O)O-$,

$R_4CH_2O-$,

$R_4C(O)-$,

$R_4$ is unsubstituted phenyl, or phenyl substituted with 1 to 3 substituents selected from the group consisting of

$C_1$ to $C_4$-alkyl,

$C_1$ to $C_4$-alkyloxy,

$C_1$ to $C_4$-alkyl-S-,

fluorine, chlorine or bromine,

$R_5$ is hydrogen or methyl;

n is zero to 2;

m is zero to 2;

in its racemic or optically active form;

or a pharmaceutically acceptable acid addition salt thereof, as an antipsychotic drug component thereof in association with a pharmaceutically acceptable diluent therefor.

18. A pharmaceutical composition according to Claim 17 wherein the anti-psychotic drug component compound is 2-(N,N-dipropylamino)-1H-phenalen-5-ol, or a pharmaceutically acceptable salt thereof.

19. A pharmaceutical composition according to Claim 17 wherein the anti-psychotic drug compound is 2-(N,N-di-n-propylamino)-4-methoxy-2,3-dihydro-1H-phenalene, or a pharmaceutically acceptable salt thereof.

20. A pharmaceutical composition according to Claim 17 wherein the anti-psychotic drug compound is 2,3-dihydro-N-monomethyl-1H-phenalene-2-amine, or a pharmaceutically acceptable salt thereof.

0234113

European Patent Office

# PARTIAL EUROPEAN SEARCH REPORT

which under Rule 45 of the European Patent Convention shall be considered, for the purposes of subsequent proceedings, as the European search report

Application number

EP 86 30 9767

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | CHIMIE THERAPEUTIQUE, vol. VI, no. 3, May-June 1971, pages 196-202; Paris, FR<br>R. VIOLLAND et al.: "VIII. Psychotropes potentiels. Synthèse et activité pharmacologique d'amino-2 tétralines apparentées à divers psychotomimétiques."<br>* Pages 198-200 *<br>--- | 10,17 | C 07 C 87/458<br>C 07 C 91/28<br>C 07 C 97/10<br>C 07 C 93/14<br>C 07 D 295/06<br>C 07 C 93/26<br>C 07 D 205/04<br>C 07 D 319/06<br>A 61 K 31/135<br>A 61 K 31/215<br>A 61 K 31/395 |
| X,D | JOURNAL OF THE CHEMICAL SOCIETY, Serie C, 1971, pages 1607-1609; London, GB<br>C. EVANS et al.: "Reactions of 2,3-dihydro-4-methylphenalen-1-one"<br>* Page 1607, compound VI; page 1609 *<br>--- | 10 | |
| A | EP-A-0 041 293 (AKZO NV)<br>* Claims; pages 8,9 *<br>-------------- | 10,17 | **TECHNICAL FIELDS SEARCHED (Int. Cl.4)**<br>C 07 D 295/00<br>C 07 C 87/00<br>C 07 C 91/00<br>C 07 C 93/00<br>C 07 C 97/00<br>C 07 D 205/00<br>C 07 D 319/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 10-20

Claims searched incompletely:

Claims not searched: 1-9

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 22-04-1987 | HENRY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document